# EUROPEAN PATENT APPLICATION

(11) **EP 4 778 388 A1**
(43) Date of publication of application: **22.07.2026**
(21) Application number: 24865581.3
(22) Date of filing: 13.09.2024
(51) Int. Cl.: A01H 5/02, A01H 5/04, A01H 5/10, A01H 6/14, C12N 15/29, C12Q 1/68

(54) **SUNFLOWER PLANT**

(30) Priority: 16.09.2023 JP 2023173251
(71) Applicant: Takii & Co., Ltd., Umekoji-dori, Shimogyo-ku Kyoto-shi Kyoto 600-8686 (JP)
(72) Inventor: AOIKE Hitomi, Kyoto-shi Kyoto 600-8686 (JP); SENDO Takahiro, Kyoto-shi Kyoto 600-8686 (JP); IKEGUCHI Hideaki, Kyoto-shi Kyoto 600-8686 (JP)
(74) Representative: J A Kemp LLP
(86) International application number: PCT/JP2024/032996
(87) International publication number: WO 2025/058082

(57) **Abstract**

The present disclosure provides a new sunflower line. A sunflower plant in which the peduncle is upright in the present disclosure comprises an upright locus of the peduncle on chromosome 4.

## Description

### TECHNICAL FIELD

The present disclosure relates to a sunflower plant.

### BACKGROUND ART

A sunflower plant *(Helianthus annuus* L.) belongs to Asteraceae and is an annual or perennial herbaceous plant originating from southwestern North America. This species is not only widely cultivated throughout the world for oil production, but many varieties are also sold for ornamental use.

### Citation List

### Non-Patent Documents

[Non-Patent Document 1] Vandenbrink, Joshua P et al. "Turning heads: the biology of solar tracking in sunflower". Plant science: an international journal of experimental plant biology vol. 224 (2014): 20-6. doi:10.1016/j.plantsci.2014.04.006

### SUMMARY OF INVENTION

### Technical Problem

In a sunflower plant, the direction of a peduncle is oriented toward the east due to genetic traits (Non-Patent Document 1). For this reason, in commercial production sites, when bundling a plurality of cut flowers to meet shipping standards at the time of shipment, it is necessary to align the direction of the flowers and tie them, which requires labor and time. Also, in the case of packing sunflower plants in boxes, it is necessary to align the direction of the flowers for the same reason.

In the field of flower arrangement, particularly in situations where use in an upward direction is desired, it takes a lot of effort to correct the bending of the flower neck with wire. For this reason, a sunflower plant in which the direction of the peduncle is upright is required.

Accordingly, an object is to provide a new sunflower plant.

### Solution to Problem

In order to achieve the object, a sunflower plant of the present disclosure comprises a mutant of the *Ha412HOChr04g0188691* gene, and the peduncle is upright.

A sunflower plant of the present disclosure in which the peduncle is upright comprises an upright locus of the peduncle on chromosome 4.

A method for producing a sunflower plant of the present disclosure (hereinafter referred to as a "producing method") is a method for producing a sunflower plant showing an upright trait of the peduncle, the method comprising the following steps (a) and (b):
(a) a step of crossing the sunflower plant of the present disclosure with another sunflower plant;
(b) a step of selecting a sunflower plant showing the upright trait of the peduncle from the sunflower plant or a progeny line thereof obtained by the step (a).

### Advantageous Effects of Invention

According to the present disclosure, a new sunflower plant can be provided.

### BRIEF DESCRIPTION OF THE DRAWINGS

[FIG. 1] FIG. 1 is a schematic view showing relative located positions of SNPs (single nucleotide polymorphisms) and the like on chromosome 4.
[FIG. 2] FIG. 2 is a schematic view showing an inclination angle of a peduncle of a sunflower plant.
[FIG. 3] FIG. 3 is a schematic view showing an example of a growth stage of a flower of a sunflower plant.
[FIG. 4] FIG. 4 is a schematic view showing an example of a direction of a flower head of a sunflower plant.
[FIG. 5] FIG. 5 is a photograph comparing inclination angles of the peduncle of each sunflower plant in Example 1.
[FIG. 6] FIG. 6 is a diagram comparing inclination angles of the peduncle of each sunflower plant in Example 1.
[FIG. 7] FIG. 7 is a graph showing G value representing a degree of contribution of each SNP on chromosome 4 to a trait, shown by QTL-seq analysis in Example 1.
[FIG. 8] FIG. 8 is a graph showing a distribution of inclination angles of the peduncle for each genotype of an upright trait SNP marker of the peduncle in Example 1.
[FIG. 9] FIG. 9 is a graph showing a distribution of inclination angles of the peduncle for each genotype of an upright trait Indel marker of the peduncle in Example 2.
[FIG. 10] FIG. 10 is a graph showing an expression level of the *Ha412HOChr04g0188691* gene in a stem in Example 2.

### DESCRIPTION OF EMBODIMENTS

### <Definitions>

In the present disclosure, "sunflower plant" means a plant belonging to Asteraceae, Asteroideae, genus *Helianthus,* species *Helianthus annuus.* The sunflower plant may be, for example, a hybrid with a related species or a wild species. Examples of the related species include *Helianthus argophyllus* (Silverleaf Sunflower), *Helianthus debilis* (Cucumberleaf Sunflower), *Helianthus giganteus* (Giant Sunflower), and the like.

In the present specification, "sunflower plant for cultivation", "sunflower variety for cultivation", or "sunflower for cultivation" means a sunflower plant or its variety, breeding line, or cultivar that is cultivated by humans and is excellent agronomically. The "sunflower plant for cultivation", "sunflower variety for cultivation", or "sunflower for cultivation" may be a pure line, or a hybrid thereof, or a hybrid with other species such as *Helianthus argophyllus* species, *Helianthus debilis* species, or other related species such as *Helianthus giganteus* species.

In the present specification, "plant body" or "plant" means a plant individual showing the entire plant.

In the present specification, "part of plant body" or "plant part" means a part of a plant individual.

In the present specification, "plant population" means a cluster in which two or more plants or plant parts are gathered as one group. The plant population can also be, for example, a collection of two or more plants or plant parts that are distinguishably separated from other plants or plant parts.

In the present specification, "crossing" means a crossing of two parent lines. The crossing can also be called, for example, mating.

In the present specification, "loss of function" means, for example, a state in which a function originally possessed by the gene is (significantly) reduced or lost. That is, in the present specification, "loss-of-function mutation" may be used to mean either a mutation in which a function originally possessed by the gene is (significantly) attenuated or a mutation in which complete loss of function occurs. In addition, the "mutation in which complete loss of function occurs" can also be called, for example, a null mutation or an amorph.

In the present specification, "isolated" means a state of being identified and separated, and/or a state of being recovered from components in a natural state.

Sequence information of a protein or a nucleic acid (for example, DNA or RNA) encoding thereof described in the present specification is available from Protein Data Bank, UniProt, Ensembl, GenBank, or the like. In addition, a nucleic acid sequence of RNA can also be obtained from a nucleic acid sequence of corresponding DNA by using sequence conversion software or the like, as appropriate.

Hereinafter, the present disclosure will be described with examples, but the present disclosure is not limited to the following examples and the like, and can be implemented with arbitrary modifications. In addition, each description in the present disclosure can be applied to each other unless otherwise specified. In the present specification, when the expression "~" is used, it is used in a sense including numerical values or physical values before and after it. In the present specification, when a plurality of upper limit values and/or a plurality of lower limit values are illustrated in a numerical range, any illustrated upper limit value and lower limit value can be combined as the upper limit value and lower limit value of the numerical range. In addition, in the present specification, the expression "A and/or B" includes "only A", "only B", and "both A and B."

### <Sunflower plant in which the peduncle is upright>

In a certain aspect, the present disclosure provides a sunflower plant in which the peduncle is upright. The sunflower plant of the present disclosure in which the peduncle is upright comprises, for example, an upright locus of the peduncle on chromosome 4. In addition, in the sunflower plant of the present disclosure in which the peduncle is upright, for example, the peduncle angle or average angle is 0 to 45°. According to the present disclosure, a sunflower plant in which the peduncle is upright can be provided.

In the sunflower plant of the present disclosure, the upright trait of the peduncle may be conferred, for example, by the presence of an upright locus of the peduncle on chromosome 4. Hereinafter, the upright locus of the peduncle on the fourth chromosome is also referred to as an upright marker of the peduncle.

The sunflower plant is known to have chromosomes 1 to 17. Each chromosome in the sunflower plant can be determined, for example, by comparing genome base sequence information of a target sunflower plant with genome base sequence information of HA412-HO, based on genome base sequence information of HA412-HO *(Helianthus annuus).* The comparison can be performed, for example, with default parameters using analysis software such as BLAST, FASTA, or the like. Genome base sequence information of HA412-HO (Ha412HOv2.0) is available, for example, from the following website 1 of the sunflower plant genome database. Genome base sequence information of another sunflower plant (HanXRQr2.0-SUNRISE) is available, for example, from the following website 2 of the sunflower plant genome database.
website 1: https://sunflowergenome.org/assembly-data/
website 2: https://www.heliagene.org/HanXRQr2.0-SUNRISE/

When the sunflower plant of the present disclosure is conferred with the upright trait of the peduncle by the presence of an upright locus of the peduncle on chromosome 4, the sunflower plant of the present disclosure comprises the upright locus of the peduncle on chromosome 4. However, the sunflower plant of the present disclosure is not limited thereto, and for example, instead of chromosome 4, it may have the upright locus of the peduncle on chromosome 4 on any chromosome other than chromosome 4. That is, the sunflower plant having the upright locus of the peduncle may have the upright locus of the peduncle on chromosome 4 on any of chromosome 1, chromosome 2, chromosome 3, chromosome 5, chromosome 6, chromosome 7, chromosome 8, chromosome 9, chromosome 10, chromosome 11, chromosome 12, chromosome 13, chromosome 14, chromosome 15, chromosome 16, and chromosome 17.

The upright locus of the peduncle can be defined using a molecular marker indicating a locus on a chromosome, i.e., a position, that is, it can be identified using a molecular marker linked to a target locus. As a technique for defining a locus such as QTL using the molecular marker, a technique well known in the technical field of the present application can be utilized.

The molecular marker used for defining the upright locus of the peduncle is not particularly limited. Examples of the molecular marker include an SNP marker, an Indel (insertion/deletion) marker, an AFLP (amplified fragment length polymorphism) marker, an RFLP (restriction fragment length polymorphism) marker, a microsatellite marker, a SCAR (sequence-characterized amplified region) marker, or a CAPS (cleaved amplified polymorphic sequence) marker. In the present disclosure, one type of the molecular marker may be used, or two or more types may be used in combination. When an SNP marker is used as the molecular marker, the SNP marker may be, for example, one SNP as the SNP marker, or a combination of two or more SNPs as the SNP marker, i.e., an SNP marker set.

The upright locus of the peduncle is linked to some or all of the molecular markers described below. For this reason, in the present disclosure, the upright locus of the peduncle can be defined, for example, by using the molecular marker. As a specific example, the upright locus of the peduncle may be defined (hereinafter also referred to as "specified"), for example, (1) by the molecular marker, (2) by a base sequence comprising the molecular marker, (3) by a region between sites of two molecular markers, i.e., by a located position, or may be defined by a combination thereof. In the case of defining by the combination, for example, the following combinations can be exemplified as the combination.
the combination of the (1) and the (2);
the combination of the (1) and the (3);
the combination of the (2) and the (3); and
the combination of the (1), the (2), and the (3).

### (1) Identification by molecular marker

The upright locus of the peduncle may be defined, for example, by the molecular marker as shown in (1). The molecular marker is not particularly limited, and examples thereof include Ha_chr04_172545117, Ha_chr04_175698294, Ha_chr04_181000331, Indel marker of Ha412HOChr04g0188691, Ha_chr04_200021302, Ha_chr04_204904126, and Ha_chr04_208654314. For SNP analysis, for example, reference can be referred to the following Reference Documents 1 to 4. In addition, Ha_chr04_172545117, Ha_chr04_175698294, Ha_chr04_181000331, Indel marker of the *Ha412HOChr04g0188691* gene, Ha_chr04_200021302, Ha_chr04_204904126, and Ha_chr04_208654314 are molecular markers newly identified by the present inventors, and a person skilled in the technical field can identify the located position of the molecular marker based on a base sequence comprising these molecular markers, as described below.
Reference Document 1: Erik W. Ohlson et al. "Identification and Mapping of Late Blight Resistance Quantitative Trait Loci in Tomato Accession PI163245", The Plant Genome, vol. 11, no. 3, 180007.
Reference Document 2: Hamilton JP, Sim SC, Stoffel K, Van Deynze A, Buell CR, et al. (2012) " Single Nucleotide Polymorphism Discovery in Cultivated Tomato via Sequencing by Synthesis. ", The Plant Genome 5.
Reference Document 3: Sim S-C, Durstewitz G, Plieske J, Wieseke R, Ganal MW, et al. (2012) "Development of a Large SNP Genotyping Array and Generation of High-Density. ", Genetic Maps in Tomato. PLoS ONE 7(7).
Reference Document 4: Blanca J, Canizares J, Cordero L, Pascual L, Diez MJ, et al. (2012) "Variation Revealed by SNP Genotyping and Morphology Provides Insight into the Origin of the Tomato"., PLoS ONE 7(10).

When the molecular marker is an SNP marker and/or an Indel marker, analysis of the molecular marker can be performed, for example, by specifically detecting a base sequence comprising the SNP and/or Indel. The length of the base sequence comprising the SNP and/or Indel is, for example, 15 to 20,000 bases, 30 to 10,000 bases, 50 to 5,000 bases, 70 to 2,000 bases, 70 to 1,500 bases, 70 to 1,000 bases, 70 to 500 bases, or 70 to 200 bases.

Detection of the base sequence comprising the SNP and/or Indel may be, for example, direct detection or indirect detection. The direct detection can be performed, for example, by detecting with a probe specific to a nucleic acid (polynucleotide) comprising a base sequence comprising the SNP and/or Indel on a chromosome of the sunflower plant. The indirect detection can be performed, for example, by amplifying a nucleic acid comprising the SNP and/or Indel on a chromosome of the sunflower plant and detecting the obtained amplified fragment. In analysis of the SNP and/or Indel, for example, prior to detection of the base sequence comprising the SNP and/or Indel, the chromosome or the nucleic acid may be cleaved with a restriction enzyme or the like.

Examples of the probe include a polynucleotide capable of specifically hybridizing to a nucleic acid comprising a base sequence comprising the SNP and/or Indel. The probe can be designed, for example, as a polynucleotide having a base sequence of a partial region or a whole region of at least 10 bases, 15 bases, 20 bases, 25 bases, 30 bases, 35 bases, 40 bases, 45 bases, 50 bases, or more in length in the base sequence comprising the SNP and/or Indel or a base sequence complementary thereto.

Detection with the probe can be performed, for example, on a chromosome or DNA extracted from the sunflower plant by a method such as a Southern blotting method or a FISH (fluorescence in situ hybridization) method.

Amplification of the nucleic acid may be performed, for example, using primers designed to specifically amplify a nucleic acid fragment. When the primers are used, amplification of the nucleic acid can be performed, for example, by a method such as a PCR (Polymerase Chain Reaction) method, an RCA (Rolling Circle Amplification) method, a CPT (Cycling Probe Technology) method, an ICAN (Isothermal and Chimeric Primer-Initiated Amplification of Nucleic Acids) method, a LAMP (Loop-Mediated Isothermal Amplification of DNA) method, an SDA (Strand Displacement Amplification) method, a NASBA (Nucleic Acid Sequence-Based Amplification) method, and a TMA (Transcription Mediated Amplification) method.

Detection of the amplified fragment may be performed by detecting the presence or absence of the amplified fragment, or may be performed using the probe. Specifically, detection of the amplified fragment includes, for example: a method of subjecting a solution after an amplification reaction to agarose electrophoresis, binding a fluorescent intercalator such as Ethidium Bromide and SYBR Green, and observing specific fluorescence; a method of adding a fluorescent intercalator to a solution of a nucleic acid amplification reaction and performing fluorescence detection after the amplification reaction; a method of performing a nucleic acid amplification reaction using fluorescence-labeled primers and performing fluorescence detection after the amplification reaction; and the like.

The Ha_chr04_172545117 (hereinafter also referred to as "SNP (a)") is a polymorphism in which the underlined base in brackets in the base sequence of SEQ ID NO: 1 (101st base of SEQ ID NO: 1) is A. That is, SNP (a) indicates the sunflower plant shows, for example, an upright trait of the peduncle when the SNP (a) is A, and does not show, for example, the upright trait of the peduncle when SNP (a) is a base other than A (e.g., T). In addition, the base sequence of SEQ ID NO: 1 can be obtained, for example, from a sunflower plant deposited under FERM BP-22482, as described below. SNP (a) can also be identified, for example, on the basis of known information in a database of the website. Specifically, the SNP (a) means a polymorphism of a base corresponding to the 172545117th base on chromosome 4 in the genome base sequence information (Ha412HOv2.0).

SEQ ID NO: 1: 5'-AGTATAAACACGAATCGGAACCGAAAGTCTTGTAAATCGACTTTT
TCGTAGACTATCGATTCGGATCCGTGCATGCATGTTTGAGATCTGTATTGGAGCC[A]AT
TTTTGACCATTTTTATTTTAGTATAACTTTCTGGAATTTTTGATACTTGAGTCTATGCTTA
GCCGATTCATTTGCATGTTTCCGATTTATGCTTAAAG-3'

The Ha_chr04_175698294 (hereinafter also referred to as "SNP (b)") is a polymorphism in which the underlined base in brackets in the base sequence of SEQ ID NO: 2 (202nd to 213th of SEQ ID NO: 2) is TAAAAAAAAAAA. That is, SNP (b) indicates that the sunflower plant shows, for example, an upright trait of the peduncle when the SNP (b) is TAAAAAAAAAAA, and it does not show, for example, the upright trait of the peduncle when SNP (b) are bases other than TAAAAAAAAAAA (e.g., TAAAAAAAAAAAA). In addition, the base sequence of SEQ ID NO: 2 can be obtained, for example, from a sunflower plant deposited under FERM BP-22482, as described below. The SNP (b) can also be identified, for example, on the basis of known information on a database of the website. Specifically, the SNP (b) means a polymorphism of bases corresponding to the 175698294th base on chromosome 4 in the genome base sequence information (Ha412HOv2.0).

SEQ ID NO: 2: 5'-CTACGACGCCCACTTGAATATGTTTTATTATATTCTTCGCAAAACCT
ATCGACGAACCCATTCATTTTTCGCCACTTAGATGACACCGAGTTGATATCTCGATACG
GGCCTTGGTCCATTAGCTCAAGGAACTTCGCCAAAACCGCATTCCAAAACCCCTCAC
CCATTTGATTATTACCTACAAAATATGTTACAAAAAA[TAAAAAAAAAAA]CTAATACTA
CAAAATATAAACTTAAAAAAAAAGTTATTGTTACGAACCTTTTATCGGGTGTGTAGAC
ATGCCTATGTAAGCGTTCGCTAAGGCTTCTTCATCGAGTTGCGTCTACGGTTTCGCCTT
TGGTTTGGACGGTTGCTCACCCACCACTTGCTTGCCTTTTCTTCGTTTGCCTTTTTGCG
GTT-3'

The Ha_chr04_181000331 (hereinafter also referred to as "SNP (c)") is a polymorphism in which the underlined base in brackets in the base sequence of SEQ ID NO: 3 (101st base of SEQ ID NO: 3) is T. That is, SNP(c) indicates that the sunflower plant shows, for example, an upright trait of the peduncle when SNP (c) is T, and does not show, for example, the upright trait of the peduncle when SNP(c) is a base other than T (e.g., C). In addition, the base sequence of SEQ ID NO: 3 can be obtained, for example, from a sunflower plant deposited under FERM BP-22482, as described below. The SNP (c) can also be identified, for example, on the basis of known information on a database of the above-described website. Specifically, the SNP (c) means a polymorphism of a base corresponding to the 181000331st base on chromosome 4 in the genome base sequence information (Ha412HOv2.0).

SEQ ID NO: 3: 5'-TTTTTGACTAATTAATTAGTCATAAAGACTAGTATCCTCCACACTA
ACTTATTTTTGCATACACACATCAAAAGTTATCTTACAAATTTCGAAATTCACC[T]TAC
ACCTTAAAATTTATCCTACACAACTCGTAATGTATCTTACACAACTCGTAATTTATCATA
CACTTTAAATTATTTTATTTCATTTTTTTGAAAAAAA-3'

The Ha_chr04_200021302 (hereinafter also referred to as "SNP (d)") is a polymorphism in which the underlined base in brackets in the base sequence of SEQ ID NO: 4 (101st base of SEQ ID NO: 4) is C. That is, SNP (d) indicates that the sunflower plant shows, for example, an upright trait of the peduncle when SNP (d) is C, and does not show, for example, the upright trait of the peduncle when SNP (d) is a base other than C (e.g., A). In addition, the base sequence of SEQ ID NO: 4 can be obtained, for example, from a sunflower plant deposited under FERM BP-22482, as described below. The SNP (d) can also be identified, for example, on the basis of known information on a database of the above-mentioned website. Specifically, the SNP (d) means a polymorphism of a base corresponding to the 200021302nd base on chromosome 4 in the genome base sequence information (Ha412HOv2.0).

SEQ ID NO: 4: 5'-CGTTTATATTACAGATTATGCATAAATCGCAAAAATAGCTTTCTGTT
GACTTTTTAAGTACGCGTTTGACTCGACATTTGAACTAGTTAGAGTGGTGATC[C]TAG
GGAACCCTTTCAGAGGTTTATTACCCACATAAATACCAACACATAACCACTTTTGATTC
GACAAGTGACTGAGCCATTAAGGATTTATCTTGAAGTC-3'

The Ha_chr04_204904126 (hereinafter also referred to as "SNP (e)") is a polymorphism in which the underlined base in brackets in the base sequence of SEQ ID NO: 5 (101st base of SEQ ID NO: 5) is G. That is, SNP (e) indicates that the sunflower plant shows, for example, an upright trait of the peduncle when SNP (e) is G, and does not show, for example, the upright trait of the peduncle when SNP (e) is a nucleotide other than G (e.g., A). In addition, the base sequence of SEQ ID NO: 5 can be obtained, for example, from a sunflower plant deposited under FERM BP-22482, as described below. The SNP (e) can also be identified, for example, on the basis of known information on a database of the above-mentioned website. Specifically, the SNP (e) means a polymorphism of a base corresponding to the 204904126th base on chromosome 4 in the genome base sequence information (Ha412HOv2.0).

SEQ ID NO: 5: 5'-GACTAATTTCATGCCTGCCTTCAAAATGGMAAAAGAAACTAATTG
GAAAWTTTTGTGTTTATATYTTGTAATAATATTTGTGCAAAAAGGTTCATGCCTT[G]TA
ATCTATCTCTTATACTAAAGCAAAATAATGTTGACTATTTGACTATGATGTGGCAGTGCT
ACWTATTCAGAGAATTGKGAATTRGGCGGCATTTGTTT-3'

The Ha _chr04_208654314 (hereinafter also referred to as "SNP (f)") is a polymorphism in which the underlined base in brackets in the base sequence of SEQ ID NO: 6 (101st base of SEQ ID NO: 6) is A. That is, SNP (f) indicates that the sunflower plant shows, for example, an upright trait of the peduncle when SNP (f) is A, and does not show, for example, the upright trait of the peduncle when SNP(f) is a base other than A (e.g., G). In addition, the base sequence of SEQ ID NO: 6 can be obtained, for example, from a sunflower plant deposited under FERM BP-22482, as described below. The SNP (f) can also be identified, for example, on the basis of known information on a database of the above-mentioned website. Specifically, the SNP (f) means a polymorphism of a base corresponding to the 208654314th base on chromosome 4 in the genome base sequence information (Ha412HOv2.0).

SEQ ID NO: 6: 5'-CTCCATGGCGTGTAACAATTTCTCTCATGTAAATTTCAGCCAACTT
GCTCGTGTTATCTTTCTTTCAGATAGGTAAGAAGTGCGCAGATTTCGTTAATCG[A]TCC
ACTATTACCCAAATAGTGTCATGGCCTCTTGGTGTTCTAGGAAACTTTGTAATAAAATC
CATGGAGATTTGTTCCCATTTCCACTTGGGAATTTCTG-3'

The Indel marker of the *Ha412HOChr04g0188691* gene (hereinafter also referred to as "Indel (ga)") shows the base sequence of the following SEQ ID NO: 13. The base sequence of SEQ ID NO: 13 can be obtained, for example, from a sunflower plant deposited under FERM BP-22482, as described below. The Indel (ga) can also be identified, for example, on the basis of known information on a database of the above-mentioned website. Specifically, the Indel (ga) means a polymorphism of nucleotides inserted between the 198408790th nucleotide and the 198408791st nucleotide on chromosome 4 in the genome base sequence information (Ha412HOv2.0).

SEQ ID NO: 13: 5'-CAGTGGCGGACCCAAGAATTTTTCCATGGGGGTGCGAAACATTT
TTAAAAATTTTAGGCCCCTAGGTATATAAGTAAAAAAATCGGTTCGTATCGGGTCGGTT
CGGGTCGGGTCATGTAAAACAAAAGAAATTGCGCCATAACGTCCCTACTCATGGTTTC
TATCACAAACAAATTGATGCATAAGTTTATCGCTCCATAAAATAATGTTTCAATTTTAAT
TTTCAACCCTAGAAATCGTGTGTAATCAGCCTAAAAATTATCTAAACAACATAATCACC
CTAAAAATCATCTAAACAACCATAAACATCGTCAAAACACATGAAATTTCAAACCTAT
TTAAGAATTTTGTTACCCTTTGGTGTCGGACGGCGGTGGTTCGCGGCTGCTGATGACG
TGCTGTTGTGGTCGATGATCGCTTGTAGGAGACGACCCAAGAGAGGGAGGGCGGGAT
GAATTCTAAGGGACTATTGGGCTTATTTTAATTATTATACTTTGAACTTAGGTTGAGTTT
GGTTAATGGGCTAGTAATTAGTGGGCTAATTATGTTTAATGAAATAGTGGGTTAAGGTA
TTGGGTATTTGGGTTAAGTTGGGTAGTATAAGTTTATATAATTTAGATAGTTTTTTTAATT
AGAGTTTACTAAAAAATTTAAAATATAAATCGAAAACATTTTTTACCTAAGGTGTGTG
GACGAAAAATTCCAGGGGTGCAGATGAAAAAATTCAAGGGGTGCGGACGAAAAAAT

CCAAAGGGTGCAGACGAGATTTTCAACGGAAAATAGCACTAAATTTTTTTTTCCCGG
GGGTGCGCCCGCCCACCTTGGGCTATATGTATGTCCGCCCTTGTAAGTATG-3'

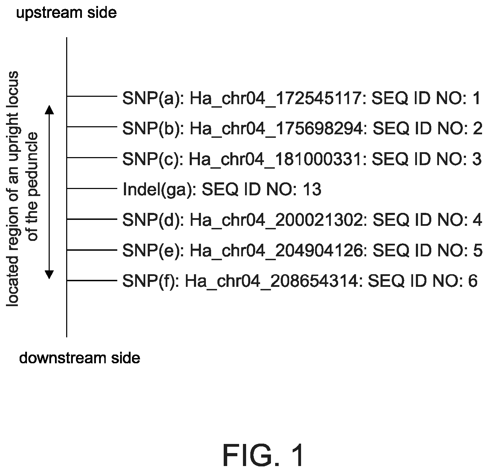

The located position of the molecular marker on the chromosome is not particularly limited. As shown in FIG. 1, for example, on chromosome 4 of the sunflower plant, Ha_chr04_172545117, Ha_chr04_175698294, Ha_chr04_181000331, Indel marker of the *Ha412HOChr04g0188691* gene, Ha_chr04_200021302, Ha_chr04_204904126, and Ha_chr04_208654314 are located in this order from an upstream side (Ha_chr04_172545117) to a downstream side (Ha_chr04_208654314).

The number of molecular markers present in the upright locus of the peduncle is not particularly limited. For example, the upright locus may include any one of the molecular markers, or two or more, i.e., two, three, four, five, six, or all seven of the markers. The relevance between these seven types of polymorphisms (SNP marker and/or Indel marker) and the upright trait of the peduncle has not been reported heretofore, and they are novel polymorphisms discovered for the first time by the present inventors as being involved in the upright trait of the peduncle.

The combination of the molecular markers is not particularly limited, and for example, the following combinations can be exemplified as:
the combination of SNP (a) and SNP (b);
the combination of SNP (a) and SNP (c);
the combination of SNP (a) and SNP (d);
the combination of SNP (a) and SNP (e);
the combination of SNP (a) and SNP (f);
the combination of SNP (a) and Indel (ga);
the combination of SNP (b) and SNP (c);
the combination of SNP (b) and SNP (d);
the combination of SNP (b) and SNP (e);
the combination of SNP (b) and SNP (f);
the combination of SNP (b) and Indel (ga);
the combination of SNP (c) and SNP (d);
the combination of SNP (c) and SNP (e);
the combination of SNP (c) and SNP (f);
the combination of SNP (c) and Indel (ga);
the combination of SNP (d) and SNP (e);
the combination of SNP (d) and SNP (f);
the combination of SNP (d) and Indel (ga);
the combination of SNP (e) and SNP (f);
the combination of SNP (e) and Indel (ga);
the combination of SNP (f) and Indel (ga);
the combination of SNP (a), SNP (b), and SNP (c);
the combination of SNP (a), SNP (b), and SNP (d);
the combination of SNP (a), SNP (b), and SNP (e);
the combination of SNP (a), SNP (b), and SNP (f);
the combination of SNP (a), SNP (b), and Indel (ga);
the combination of SNP (a), SNP (c), and SNP (d);
the combination of SNP (a), SNP (c), and SNP (e);
the combination of SNP (a), SNP (c), and SNP (f);
the combination of SNP (a), SNP (c), and Indel (ga);
the combination of SNP (a), SNP (d), and SNP (e);
the combination of SNP (a), SNP (d), and SNP (f);
the combination of SNP (a), SNP (d), and Indel (ga);
the combination of SNP (a), SNP (e), and SNP (f);
the combination of SNP (a), SNP (e), and Indel (ga);
the combination of SNP (a), SNP (f), and Indel (ga);
the combination of SNP (b), SNP (c), and SNP (d);
the combination of SNP (b), SNP (c), and SNP (e);
the combination of SNP (b), SNP (c), and SNP (f);
the combination of SNP (b), SNP (c), and Indel (ga);
the combination of SNP (b), SNP (d), and SNP (e);
the combination of SNP (b), SNP (d), and SNP (f);
the combination of SNP (b), SNP (d), and Indel (ga);
the combination of SNP (b), SNP (e), and SNP (f);
the combination of SNP (b), SNP (e), and Indel (ga);
the combination of SNP (b), SNP (f), and Indel (ga);
the combination of SNP (c), SNP (d), and SNP (e);
the combination of SNP (c), SNP (d), and SNP (f);
the combination of SNP (c), SNP (d), and Indel (ga);
the combination of SNP (c), SNP (e), and SNP (f);
the combination of SNP (c), SNP (e), and Indel (ga);
the combination of SNP (c), SNP (f), and Indel (ga);
the combination of SNP (d), SNP (e), and SNP (f);
the combination of SNP (d), SNP (e), and Indel (ga);
the combination of SNP (d), SNP (f), and Indel (ga);
the combination of SNP (e), SNP (f), and Indel (ga);
the combination of SNP (a), SNP (b), SNP (c), and SNP (d);
the combination of SNP (a), SNP (b), SNP (c), and SNP (e);
the combination of SNP (a), SNP (b), SNP (c), and SNP (f);
the combination of SNP (a), SNP (b), SNP (c), and Indel (ga);
the combination of SNP (a), SNP (b), SNP (d), and SNP (e);
the combination of SNP (a), SNP (b), SNP (d), and SNP (f);
the combination of SNP (a), SNP (b), SNP (d), and Indel (ga);
the combination of SNP (a), SNP (b), SNP (e), and SNP (f);
the combination of SNP (a), SNP (b), SNP (e), and Indel (ga);
the combination of SNP (a), SNP (b), SNP (f), and Indel (ga);
the combination of SNP (a), SNP (c), SNP (d), and SNP (e);
the combination of SNP (a), SNP (c), SNP (d), and SNP (f);
the combination of SNP (a), SNP (c), SNP (d), and Indel (ga);
the combination of SNP (a), SNP (c), SNP (e), and SNP (f);
the combination of SNP (a), SNP (c), SNP (e), and Indel (ga);
the combination of SNP (a), SNP (c), SNP (f), and Indel (ga);
the combination of SNP (a), SNP (d), SNP (e), and SNP (f);
the combination of SNP (a), SNP (d), SNP (e), and Indel (ga);
the combination of SNP (a), SNP (d), SNP (f), and Indel (ga);
the combination of SNP (a), SNP (e), SNP (f), and Indel (ga);
the combination of SNP (b), SNP (c), SNP (d), and SNP (e);
the combination of SNP (b), SNP (c), SNP (d), and SNP (f);
the combination of SNP (b), SNP (c), SNP (d), and Indel (ga);
the combination of SNP (b), SNP (c), SNP (e), and SNP (f);
the combination of SNP (b), SNP (c), SNP (e), and Indel (ga);
the combination of SNP (b), SNP (c), SNP (f), and Indel (ga);
the combination of SNP (b), SNP (d), SNP (e), and SNP (f);
the combination of SNP (b), SNP (d), SNP (e), and Indel (ga);
the combination of SNP (b), SNP (d), SNP (f), and Indel (ga);
the combination of SNP (b), SNP (e), SNP (f), and Indel (ga);
the combination of SNP (c), SNP (d), SNP (e), and SNP (f);
the combination of SNP (c), SNP (d), SNP (e), and Indel (ga);
the combination of SNP (c), SNP (d), SNP (f), and Indel (ga);
the combination of SNP (c), SNP (e), SNP (f), and Indel (ga);
the combination of SNP (d), SNP (e), SNP (f), and Indel (ga);
the combination of SNP (b), SNP (c), SNP (d), SNP (e), and Indel (ga); and
the combination of SNP (a), SNP (b), SNP (c), SNP (d), SNP (e), SNP (f), and Indel (ga);
of these combinations, for example, the following combinations are preferable because they show a higher correlation with the upright trait of the peduncle:
   the combination of SNP (b) and SNP (e);
   the combination of SNP (c) and SNP (d);
   the combination of SNP (c) and Indel (ga);
   the combination of SNP (d) and Indel (ga);
   the combination of SNP (b), SNP (c), and SNP (d);
   the combination of SNP (c), SNP (d), and SNP (e);
   the combination of SNP (c), SNP (d), and Indel (ga);
   the combination of SNP (b), SNP (c), and SNP (d), and SNP (e); and
   the combination of SNP (b), SNP (c), and SNP (d), SNP (e), and Indel (ga).

### (2) Identification by base sequence comprising molecular marker

The upright locus of the peduncle may be defined, for example, by a base sequence comprising the molecular marker, as shown in (2). The upright locus of the peduncle may, for example, comprise of the base sequence, or may comprise the base sequence.

The base sequence comprising the molecular marker is not particularly limited, and examples thereof include polynucleotides (a), (b), (c), (d), (e), (f), (g), and (ga) as described below. The polynucleotides (a), (b), (c), (d), (e), (f), and (ga) respectively correspond to base sequences comprising the molecular markers SNP (a), SNP (b), SNP (c), SNP (d), SNP (e), SNP (f), and Indel (ga).

The polynucleotide (a) is a base sequence comprising the SNP (a), i.e., Ha_chr04_172545117, and is, for example, a polynucleotide (a1), (a2), or (a3) below. The polynucleotides (a2) and (a3) are respectively polynucleotides having functions equivalent to those of the polynucleotide (a1) regarding the upright trait of the peduncle at the upright locus of the peduncle. The equivalent functions mean, for example, that a sunflower plant having the upright locus of the peduncle identified by the polynucleotide (a2) or (a3) shows the upright trait of the peduncle.
(a) a polynucleotide (a1), (a2), or (a3) below:
   (a1) a polynucleotide comprising of SEQ ID NO: 1;
   (a2) a polynucleotide that comprises of a base sequence in which one or several bases are deleted, substituted, inserted and/or added in the base sequence of the (a1) with the 101st base (A) of the (a1) being conserved, and has a function equivalent to that of the polynucleotide (a1) regarding the upright trait of the peduncle at the upright locus of the peduncle;
   (a3) a polynucleotide that comprises of a base sequence having an identity of at least 80% to the base sequence of the (a1) with the 101st base (A) of the (a1) being conserved, and has a function equivalent to that of the polynucleotide (a1) regarding the upright trait of the peduncle at the upright locus of the peduncle.

In the polynucleotide (a1), the underlined base (A) in brackets in SEQ ID NO: 1 is a base corresponding to the polymorphism of the SNP (a). The polynucleotide (a1) can be obtained, for example, from a sunflower plant deposited under Accession No. FERM BP-22482 as described below.

In the polynucleotide (a2), the one or several means, for example, 1 to 40, 1 to 35, 1 to 30, 1 to 25, 1 to 20, 1 to 18, 1 to 12, 1 to 8, 1 to 4, 1 to 3, 1, or 2.
In the present disclosure, the numerical range regarding the number of bases or the like discloses all of the positive integers falling within that range, for example. That is, for example, the description "one to five" is intended to disclose all of "one, two, three, four, and five" (the same applies hereinafter).

In the polynucleotide (a3), the identity means, for example, at least 80%, at least 85%, at least 89%, at least 90%, at least 95%, at least 96%, at least 97%, at least 98%, or at least 99%. The identity can be determined by aligning two base sequences (the same applies hereinafter). Specifically, the identity can be calculated, for example, using analysis software such as BLAST, FASTA, or the like with default parameters (the same applies hereinafter).

The polynucleotide (b) is a base sequence comprising the SNP (b), i.e., Ha_chr04_175698294, and is, for example, a polynucleotide (b1), (b2), or (b3) below. The polynucleotides (b2) and (b3) are respectively polynucleotides having functions equivalent to those of the polynucleotide (b1) regarding the upright trait of the peduncle at the upright locus of the peduncle. The equivalent functions mean, for example, that a sunflower plant having the upright locus of the peduncle identified by the polynucleotide (b2) or (b3) shows the upright trait of the peduncle.
(b) a polynucleotide (b1), (b2), or (b3) below:
(b1) a polynucleotide comprising of SEQ ID NO: 2;
(b2) a polynucleotide that comprises of a base sequence in which one or several bases are deleted, substituted, inserted and/or added in the base sequence of the (b1) with the 202nd to 213th bases (TAAAAAAAAAAA) of the (b1) being conserved, and has a function equivalent to that of the polynucleotide (b1) regarding the upright trait of the peduncle at the upright locus of the peduncle;
(b3) a polynucleotide that comprises of a base sequence having an identity of at least 80% to the base sequence of the (b1) with the 202nd to 213th bases (A) of the (b1) being conserved, and has a function equivalent to that of the polynucleotide (b1) regarding the upright trait of the peduncle at the upright locus of the peduncle.

In the polynucleotide (b1), the underlined bases (TAAAAAAAAAAA) in brackets in SEQ ID NO: 2 are bases corresponding to the polymorphism of the SNP (b). The polynucleotide (b1) can be obtained, for example, from a sunflower plant deposited under the Accession No. FERM BP-22482 as described below.

In the polynucleotide (b2), the one or several means, for example, 1 to 80, 1 to 70, 1 to 60, 1 to 50, 1 to 40, 1 to 38, 1 to 36, 1 to 30, 1 to 24, 1 to 20, 1 to 18, 1 to 16, 1 to 12, 1 to 10, 1 to 8, 1 to 6, 1 to 4, 1 to 3, 1, or 2.

In the polynucleotide (b3), the identity means, for example, at least 80%, at least 85%, at least 89%, at least 90%, at least 95%, at least 96%, at least 97%, at least 98%, or at least 99%.

The polynucleotide (c) is a base sequence comprising the SNP (c), i.e., Ha_chr04_181000331, and is, for example, a polynucleotide (c1), (c2), or (c3) below. The polynucleotides (c2) and (c3) are respectively polynucleotides having functions equivalent to those of the polynucleotide (c1) regarding the upright trait of the peduncle at the upright locus of the peduncle. The equivalent functions mean, for example, that a sunflower plant having the upright locus of the peduncle identified by the polynucleotide (c2) or (c3) shows the upright trait of the peduncle.
(c) a polynucleotide (c1), (c2), or (c3) below:
(c1) a polynucleotide comprising of SEQ ID NO: 3;
(c2) a polynucleotide that comprises of a base sequence in which one or several bases are deleted, substituted, inserted and/or added in the base sequence of the (c1) with the 101st base (T) of the (c1) being conserved, and has a function equivalent to that of the polynucleotide (c1) regarding the upright trait of the peduncle at the upright locus of the peduncle;
(c3) a polynucleotide that comprises of a base sequence having an identity of at least 80% to the base sequence of the (c1) with the 101st base (T) of the (c1) being conserved, and has a function equivalent to that of the polynucleotide (c1) regarding the upright trait of the peduncle at the upright locus of the peduncle.

In the polynucleotide (c1), the underlined base (T) in brackets in SEQ ID NO: 3 is a base corresponding to the polymorphism of SNP (c). The polynucleotide (c1) can be obtained, for example, from a sunflower plant deposited under the Accession No. FERM BP-22482 as described below.

In the polynucleotide (c2), the one or several means, for example, 1 to 40, 1 to 35, 1 to 30, 1 to 25, 1 to 20, 1 to 18, 1 to 12, 1 to 10, 1 to 8, 1 to 4, 1 to 3, 1, or 2.

In the polynucleotide (c3), the identity means, for example, at least 80%, at least 85%, at least 89%, at least 90%, at least 95%, at least 96%, at least 97%, at least 98%, or at least 99%.

The polynucleotide (d) is a base sequence comprising the SNP (d), i.e., Ha_chr04_200021302, and is, for example, a polynucleotide (d1), (d2), or (d3) below. The polynucleotides (d2) and (d3) are respectively polynucleotides having functions equivalent to those of the polynucleotide (d1) regarding the upright trait of the peduncle at the upright locus of the peduncle. The equivalent functions mean, for example, that a sunflower plant having the upright locus of the peduncle identified by the polynucleotide (d2) or (d3) shows the upright trait of the peduncle.
(d) a polynucleotide (d1), (d2), or (d3) below:
(d1) a polynucleotide comprising of SEQ ID NO: 4;
(d2) a polynucleotide that comprises of a base sequence in which one or several bases are deleted, substituted, inserted and/or added in the base sequence of the (d1) with the 101st base (C) of the (d1) being conserved, and has a function equivalent to that of the polynucleotide (d1) regarding the upright trait of the peduncle at the upright locus of the peduncle;
(d3) a polynucleotide that comprises of a base sequence having an identity of at least 80% to the base sequence of the (d1) with the 101st base (C) of the (d1) being conserved, and has a function equivalent to that of the polynucleotide (d1) regarding the upright trait of the peduncle at the upright locus of the peduncle.

In the polynucleotide (d1), the underlined base (C) in brackets in SEQ ID NO: 4 is a base corresponding to the polymorphism of the SNP (d). The polynucleotide (d1) can be obtained, for example, from a sunflower plant deposited under Accession No. FERM BP-22482 as described below.

In the polynucleotide (d2), the one or several means, for example, 1 to 40, 1 to 35, 1 to 30, 1 to 25, 1 to 20, 1 to 18, 1 to 12, 1 to 10, 1 to 8, 1 to 4, 1 to 3, 1, or 2.

In the polynucleotide (d3), the identity means, for example, at least 80%, at least 85%, at least 89%, at least 90%, at least 95%, at least 96%, at least 97%, at least 98%, or at least 99%.

The polynucleotide (e) is a base sequence comprising the SNP (e), i.e., Ha_chr04_204904126, and is, for example, a polynucleotide (e1), (e2), or (e3) below. The polynucleotides (e2) and (e3) are respectively polynucleotides having functions equivalent to those of the polynucleotide (e1) regarding the upright trait of the peduncle at the upright locus of the peduncle. The equivalent functions mean, for example, that a sunflower plant having the upright locus of the peduncle identified by the polynucleotide (e2) or (e3) shows the upright trait of the peduncle.
(e) a polynucleotide (e1), (e2), or (e3) below:
(e1) a polynucleotide comprising of SEQ ID NO: 5;
(e2) a polynucleotide that comprises of a base sequence in which one or several bases are deleted, substituted, inserted and/or added in the base sequence of the (e1) with the 101st base (G) of the (e1) being conserved, and has a function equivalent to that of the polynucleotide (e1) regarding the upright trait of the peduncle at the upright locus of the peduncle;
(e3) a polynucleotide that comprises of a base sequence having an identity of at least 80% to the base sequence of the (e1) with the 101st base (G) of the (e1) being conserved, and has a function equivalent to that of the polynucleotide (e1) regarding the upright trait of the peduncle at the upright locus of the peduncle.

In the polynucleotide (e1), the underlined base (G) in brackets in SEQ ID NO: 5 is a base corresponding to the polymorphism of the SNP (e). The polynucleotide (e1) can be obtained, for example, from a sunflower plant deposited under the Accession No. FERM BP-22482 as described below.

In the polynucleotide (e2), the one or several means, for example, 1 to 40, 1 to 35, 1 to 30, 1 to 25, 1 to 20, 1 to 18, 1 to 12, 1 to 10, 1 to 8, 1 to 4, 1 to 3, 1, or 2.

In the polynucleotide (e3), the identity means, for example, at least 80%, at least 85%, at least 89%, at least 90%, at least 95%, at least 96%, at least 97%, at least 98%, or at least 99%.

The polynucleotide (f) is a base sequence comprising the SNP (f), i.e., Ha_chr04_208654314, and is, for example, a polynucleotide (f1), (f2), or (f3) below. The polynucleotides (f2) and (f3) are respectively polynucleotides having functions equivalent to those of the polynucleotide (f1) regarding the upright trait of the peduncle at the upright locus of the peduncle. The equivalent functions mean, for example, that a sunflower plant having the upright locus of the peduncle identified by the polynucleotide (f2) or (f3) shows the upright trait of the peduncle.
(f) a polynucleotide (f1), (f2), or (f3) below:
(fl) a polynucleotide comprising of SEQ ID NO: 6;
(f2) a polynucleotide that comprises of a base sequence in which one or several bases are deleted, substituted, inserted and/or added in the base sequence of the (f1) with the 101st base (A) of the (f1) being conserved, and has a function equivalent to that of the polynucleotide (f1) regarding the upright trait of the peduncle at the upright locus of the peduncle;
(f3) a polynucleotide that comprises of a base sequence having an identity of at least 80% to the base sequence of the (fl) with the 101st base (A) of the (f1) being conserved, and has a function equivalent to that of the polynucleotide (f1) regarding the upright trait of the peduncle at the upright locus of the peduncle.

In the polynucleotide (f1), the underlined base (A) in brackets in SEQ ID NO: 6 is a base corresponding to the polymorphism of the SNP (f). The polynucleotide (f1) can be obtained, for example, from a sunflower plant deposited under the Accession No. FERM BP-22482 as described below.

In the polynucleotide (f2), the one or several means, for example, 1 to 40, 1 to 35, 1 to 30, 1 to 25, 1 to 20, 1 to 18, 1 to 12, 1 to 10, 1 to 8, 1 to 4, 1 to 3, 1, or 2 bases.

In the polynucleotide (f3), the identity means, for example, at least 80%, at least 85%, at least 89%, at least 90%, at least 95%, at least 96%, at least 97%, at least 98%, or at least 99%.

The polynucleotide (g) is a base sequence comprising a part of the base sequence of the Indel marker of the *Ha412HOChr04g0188691* gene, i.e., the Indel (ga), and is, for example, a polynucleotide (gl), (g2), or (g3) below. The polynucleotides (g2) and (g3) are respectively polynucleotides having functions equivalent to those of the polynucleotide (g1) regarding the upright trait of the peduncle at the upright locus of the peduncle. The equivalent functions mean, for example, that a sunflower plant having the upright locus of the peduncle identified by the polynucleotide (g2) or (g3) shows the upright trait of the peduncle.
(g) a polynucleotide (g1), (g2), or (g3) below:
(g1) a polynucleotide having a polynucleotide of 1 to 855 bases between a polynucleotide comprising of the base sequence of SEQ ID NO: 14 and a polynucleotide comprising of the base sequence of SEQ ID NO: 15;
(g2) a polynucleotide that comprises of a base sequence in which one or several bases are deleted, substituted, inserted and/or added in the base sequence of the (g1) with the base sequence of the polynucleotide of 1 to 855 bases in the (g1) being conserved;
(g3) a polynucleotide that comprises of a base sequence having an identity of 80% or more to the base sequence of the (g1) with the base sequence of the polynucleotide of 1 to 855 bases in the (g1) being conserved.

SEQ ID NO: 14: 5'-AAAGCTTGTAAGTATG-3'
SEQ ID NO: 15: 5'-GGTTCTTTCATGATAA-3'

The polynucleotide (g1) can be obtained, for example, from a sunflower plant deposited under Accession No. FERM BP-22482 as described below.

In the polynucleotide (g1), the length of the polynucleotide existing between the polynucleotide comprising of the base sequence of SEQ ID NO: 14 and the polynucleotide comprising of the base sequence of SEQ ID NO: 15 (polynucleotide between the base sequences) is, for example, 1 to 855 bases, 50 to 855 bases, 100 to 855 bases, 150 to 855 bases, 200 to 855 bases, 250 to 855 bases, 300 to 855 bases, 350 to 855 bases, 400 to 855 bases, 450 to 855 bases, 500 to 855 bases, 550 to 855 bases, 600 to 855 bases, 650 to 855 bases, 700 to 855 bases, 750 to 855 bases, 800 to 855 bases, 820 to 855 bases, 830 to 855 bases, 840 to 855 bases, 850 to 855 bases, 853 to 855 bases, 854 or 855 bases.

The base sequence of the polynucleotide between the base sequences is, for example, an arbitrary base sequence, and specific examples thereof include the polynucleotide (ga). When the polynucleotide between the base sequences exists between the polynucleotide comprising of the base sequence of SEQ ID NO: 14 and the polynucleotide comprising of the base sequence of SEQ ID NO: 15, the base sequence of the polynucleotide between the base sequences is, for example, a polynucleotide (ga) below, where a (ga1) is a polynucleotide comprising of the base sequence of SEQ ID NO: 13.

In the polynucleotide (g2), the one or several means, for example, 1 to 6, 1 to 5, 1 to 4, 1 to 3, 1, or 2.

In the polynucleotide (g3), the identity means, for example, at least 80%, at least 85%, at least 89%, at least 90%, at least 95%, at least 96%, at least 97%, at least 98%, or at least 99%.

The polynucleotide (ga) is a base sequence of the Indel marker of the *Ha412HOChr04g0188691* gene, i.e., the Indel (ga), and is, for example, a polynucleotide (ga1), (ga2), or (ga3) below. The polynucleotides (ga2) and (ga3) are respectively polynucleotides having functions equivalent to those of the polynucleotide (gal) regarding the upright trait of the peduncle at the upright locus of the peduncle. The equivalent functions mean, for example, that a sunflower plant having the upright locus of the peduncle identified by the polynucleotide (ga2) or (ga3) shows the upright trait of the peduncle.
(ga) a polynucleotide (ga1), (ga2), or (ga3) below:
(ga1) a polynucleotide comprising of the base sequence of SEQ ID NO: 13;
(ga2) a polynucleotide that comprises of a base sequence in which one or several bases are deleted, substituted, inserted and/or added in the base sequence of the (ga1), and has a function equivalent to that of the polynucleotide (ga1) regarding the upright trait of the peduncle at the upright locus of the peduncle;
(ga3) a polynucleotide that comprises of a base sequence in which the identity is at least 80% to the base sequence of the (ga1), and which has a function equivalent to that of the polynucleotide (ga1) regarding the upright trait of the peduncle at the upright locus of the peduncle.

SEQ ID NO: 13: 5'-CAGTGGCGGACCCAAGAATTTTTCCATGGGGGTGCGAA
ACATTTTTAAAAATTTTAGGCCCCTAGGTATATAAGTAAAAAAATCGGTTCGTATCGGG
TCGGTTCGGGTCGGGTCATGTAAAACAAAAGAAATTGCGCCATAACGTCCCTACTCAT
GGTTTCTATCACAAACAAATTGATGCATAAGTTTATCGCTCCATAAAATAATGTTTCAA
TTTTAATTTTCAACCCTAGAAATCGTGTGTAATCAGCCTAAAAATTATCTAAACAACAT
AATCACCCTAAAAATCATCTAAACAACCATAAACATCGTCAAAACACATGAAATTTCA
AACCTATTTAAGAATTTTGTTACCCTTTGGTGTCGGACGGCGGTGGTTCGCGGCTGCT
GATGACGTGCTGTTGTGGTCGATGATCGCTTGTAGGAGACGACCCAAGAGAGGGAGG
GCGGGATGAATTCTAAGGGACTATTGGGCTTATTTTAATTATTATACTTTGAACTTAGGT
TGAGTTTGGTTAATGGGCTAGTAATTAGTGGGCTAATTATGTTTAATGAAATAGTGGGT
TAAGGTATTGGGTATTTGGGTTAAGTTGGGTAGTATAAGTTTATATAATTTAGATAGTTT
TTTTAATTAGAGTTTACTAAAAAATTTAAAATATAAATCGAAAACATTTTTTACCTAAG
GTGTGTGGACGAAAAATTCCAGGGGTGCAGATGAAAAAATTCAAGGGGTGCGGACG
AAAAAATCCAAAGGGTGCAGACGAGATTTTCAACGGAAAATAGCACTAAATTTTTTT
TTCCCGGGGGTGCGCCCGCCCACCTTGGGCTATATGTATGTCCGCCCTTGTAAGTATG-
3'

The polynucleotide (ga1) can be obtained, for example, from a sunflower plant deposited under Accession No. FERM BP-22482 as described below.

In the polynucleotide (ga2), the one or several means, for example, 1 to 171, 1 to 128, 1 to 85, 1 to 42, 1 to 34, 1 to 25, 1 to 17, 1 to 12, 1 to 10, 1 to 8, 1 to 4, 1 to 3, 1, or 2 bases.

In the polynucleotide (ga3), the identity means, for example, at least 80%, at least 85%, at least 89%, at least 90%, at least 95%, at least 96%, at least 97%, at least 98%, or at least 99%.

The number of base sequences comprising the SNP marker present in the upright locus of the peduncle, i.e., the number of base sequences comprising the SNP marker linked to the upright locus of the peduncle, is not particularly limited. For example, the upright locus may include any one, or two or more, i.e., two, three, four, five, six, seven, or all eight of the polynucleotides (a), (b), (c), (d), (e), (f), (g), and (ga).

The combination of the base sequences comprising the SNP marker is not particularly limited, and for example, the following combinations can be exemplified:
the combination of polynucleotides (a) and (b);
the combination of polynucleotides (a) and (c);
the combination of polynucleotides (a) and (d);
the combination of polynucleotides (a) and (e);
the combination of polynucleotides (a) and (f);
the combination of polynucleotides (a) and (g);
the combination of polynucleotides (a) and (ga);
the combination of polynucleotides (b) and (c);
the combination of polynucleotides (b) and (d);
the combination of polynucleotides (b) and (e);
the combination of polynucleotides (b) and (f);
the combination of polynucleotides (b) and (g);
the combination of polynucleotides (b) and (ga);
the combination of polynucleotides (c) and (d);
the combination of polynucleotides (c) and (e);
the combination of polynucleotides (c) and (f);
the combination of polynucleotides (c) and (g);
the combination of polynucleotides (c) and (ga);
the combination of polynucleotides (d) and (e);
the combination of polynucleotides (d) and (f);
the combination of polynucleotides (d) and (g);
the combination of polynucleotides (d) and (ga);
the combination of polynucleotides (e) and (f);
the combination of polynucleotides (e) and (g);
the combination of polynucleotides (e) and (ga);
the combination of polynucleotides (f) and (g);
the combination of polynucleotides (f) and (ga);
the combination of polynucleotides (g) and (ga);
the combination of polynucleotides (a), (b), and (c);
the combination of polynucleotides (a), (b), and (d);
the combination of polynucleotides (a), (b), and (e);
the combination of polynucleotides (a), (b), and (f);
the combination of polynucleotides (a), (b), and (g);
the combination of polynucleotides (a), (b), and (ga);
the combination of polynucleotides (a), (c), and (d);
the combination of polynucleotides (a), (c), and (e);
the combination of polynucleotides (a), (c), and (f);
the combination of polynucleotides (a), (c), and (g);
the combination of polynucleotides (a), (c), and (ga);
the combination of polynucleotides (a), (d), and (e);
the combination of polynucleotides (a), (d), and (f);
the combination of polynucleotides (a), (d), and (g);
the combination of polynucleotides (a), (d), and (ga);
the combination of polynucleotides (a), (e), and (f);
the combination of polynucleotides (a), (e), and (g);
the combination of polynucleotides (a), (e), and (ga);
the combination of polynucleotides (a), (f), and (g);
the combination of polynucleotides (a), (f), and (ga);
the combination of polynucleotides (a), (g), and (ga);
the combination of polynucleotides (b), (c), and (d);
the combination of polynucleotides (b), (c), and (e);
the combination of polynucleotides (b), (c), and (f);
the combination of polynucleotides (b), (c), and (g);
the combination of polynucleotides (b), (c), and (ga);
the combination of polynucleotides (b), (d), and (e);
the combination of polynucleotides (b), (d), and (f);
the combination of polynucleotides (b), (d), and (g);
the combination of polynucleotides (b), (d), and (ga);
the combination of polynucleotides (b), (e), and (f);
the combination of polynucleotides (b), (e), and (g);
the combination of polynucleotides (b), (e), and (ga);
the combination of polynucleotides (b), (f), and (g);
the combination of polynucleotides (b), (f), and (ga);
the combination of polynucleotides (b), (g), and (ga);
the combination of polynucleotides (c), (d), and (e);
the combination of polynucleotides (c), (d), and (f);
the combination of polynucleotides (c), (d), and (g);
the combination of polynucleotides (c), (d), and (ga);
the combination of polynucleotides (c), (e), and (f);
the combination of polynucleotides (c), (e), and (g);
the combination of polynucleotides (c), (e), and (ga);
the combination of polynucleotides (c), (f), and (g);
the combination of polynucleotides (c), (f), and (ga);
the combination of polynucleotides (c), (g), and (ga);
the combination of polynucleotides (d), (e), and (f);
the combination of polynucleotides (d), (e), and (g);
the combination of polynucleotides (d), (e), and (ga);
the combination of polynucleotides (d), (f), and (g);
the combination of polynucleotides (d), (f), and (ga);
the combination of polynucleotides (d), (g), and (ga);
the combination of polynucleotides (e), (f), and (g);
the combination of polynucleotides (e), (f), and (ga);
the combination of polynucleotides (e), (g), and (ga);
the combination of polynucleotides (f), (g), and (ga);
the combination of polynucleotides (a), (b), (c), and (g);
the combination of polynucleotides (a), (b), (c), and (ga);
the combination of polynucleotides (b), (c), (d), and (e);
the combination of polynucleotides (b), (c), (d), and (g);
the combination of polynucleotides (b), (c), (d), and (ga);
the combination of polynucleotides (b), (c), (e), and (g);
the combination of polynucleotides (b), (c), (e), and (ga);
the combination of polynucleotides (b), (c), (g), and (ga);
the combination of polynucleotides (b), (d), (e), and (g);
the combination of polynucleotides (b), (d), (e), and (ga);
the combination of polynucleotides (b), (d), (g), and (ga);
the combination of polynucleotides (b), (e), (g), and (ga);
the combination of polynucleotides (c), (d), (e), and (g);
the combination of polynucleotides (c), (d), and (e), and (ga);
the combination of polynucleotides (c), (d), (g), and (ga);
the combination of polynucleotides (c), (e), (g), and (ga);
the combination of polynucleotides (d), (e), (f), and (g);
the combination of polynucleotides (d), (e), (f), and (ga);
the combination of polynucleotides (d), (e), (g), and (ga);
the combination of polynucleotides (b), (c), (d), (e), and (g);
the combination of polynucleotides (b), (c), (d), (e), and (ga);
the combination of polynucleotides (a), (b), (c), (d), (e), (f), (g), and (ga);
of these combinations, for example, the following combinations are preferable because they show the correlation with the upright trait of the peduncle is higher:
   the combination of polynucleotides (b) and (e);
   the combination of polynucleotides (c) and (d);
   the combination of polynucleotides (c) and (g);
   the combination of polynucleotides (c) and (ga);
   the combination of polynucleotides (d) and (g);
   the combination of polynucleotides (d) and (ga);
   the combination of polynucleotides (g) and (ga);
   the combination of polynucleotides (b), (c), and (d);
   the combination of polynucleotides (c), (d), and (e);
   the combination of polynucleotides (c), (d), and (g);
   the combination of polynucleotides (c), (d), and (ga);
   the combination of polynucleotides (b), (c), (d), and (e);
   the combination of polynucleotides (b), (c), (d), (e), and (g);
   the combination of polynucleotides (b), (c), (d), (e), and (ga).

### (3) Identification by region between sites of two molecular markers

The upright locus of the peduncle is linked to molecular markers, as described above. Accordingly, the upright locus of the peduncle may be defined, as shown in the (3), for example, by a base sequence of a region between the sites of the two molecular markers, or by a region specified by the two molecular markers (also referred to as a chromosomal location or a mapping region). The base sequence of the region between the sites of the two molecular markers is not particularly limited, and examples thereof include a base sequence of a region between the sites of two molecular markers selected from the group comprising of Ha_chr04_172545117, Ha_chr04_1f75698294, Ha_chr04_181000331, an Indel marker of the *Ha412HOChr04g0188691* gene, Ha_chr04_200021302, Ha_chr04_204904126, and Ha_chr04_208654314 in the chromosome. Regarding the base sequence of the region between the sites of the two molecular markers, reference may be made to, for example, the base sequence of a region between the sites of the corresponding two molecular markers in a sunflower plant deposited under the Accession No. FERM BP-22482 as described below (hereinafter, also referred to as a "deposited line"). When the base sequence of the deposited line is referred to, the base sequence of the region between the sites of the two molecular markers, for example, completely or partially matches the base sequence of the deposited line. In the latter case, the base sequence of the region between the sites of the two molecular markers only needs to be such that a sunflower plant having the upright locus of the peduncle identified by the base sequence of the region between the sites of the two molecular markers exhibits an upright trait. The partial match can be defined, for example, by identity to the base sequence of the deposited line. The identity is, for example, at least 50%, at least 55%, at least 60%, at least 65%, at least 70%, at least 75%, at least 80%, at least 85%, at least 89%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9%.

When the upright locus of the peduncle is identified in the (3), it can also be that the upright locus of the peduncle is located in a region between the sites of the two molecular markers, for example. In this case, the sunflower plant is conferred with the upright trait, for example, by the presence of the upright locus of the peduncle. When the upright locus of the peduncle is identified by the region in which it is located, the upright locus of the peduncle may be further identified in combination with the molecular marker or a base sequence comprising the molecular marker. Further, when the upright locus of the peduncle is identified in the (3), it can also be that the upright locus of the peduncle is comprised or exists in a genomic region between the sites of the two molecular markers.

The two molecular markers include, for example, a combination of Ha_chr04_175698294 and the Ha_chr04_204904126, a combination of molecular markers of the Ha_chr04_181000331 and the Ha_chr04_200021302, a combination of an SNP marker of the Ha_chr04_181000331 and an Indel marker of the *Ha412HOChr04g0188691* gene, and a combination of an Indel marker of the *Ha412HOChr04g0188691* gene and an SNP marker of the Ha_chr04_200021302.

The region, as described above, can be identified by an upstream side end and a downstream side end, for example, using the sites of the two molecular markers. The region is not limited as long as, for example, it extends between the sites of the two molecular markers, and for example, may or may not comprise both or one of the sites of the two molecular markers. Further, when the region comprises a site of the molecular marker, the upstream side end and the downstream side end of the region corresponds to the sites of the molecular marker, but the base of the upstream side end and the base of the downstream side end may be, for example, the above underlined base in the base sequences, or may be a base other than the underlined base.

When the upright locus of the peduncle is defined by a region between the sites of the two molecular markers, it is preferable that the upright locus of the peduncle further has the molecular marker located in the region. Specifically, it is preferable that the upright locus of the peduncle has, in the region, for example, a molecular marker selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314.

The molecular marker located in the region may be, for example, one or both of the sites of the two molecular markers specifying the region on the chromosome, or may be a molecular marker located between the sites of the two molecular markers specifying the region. The former is also referred to as a terminal molecular marker of the region, and the latter is also referred to as an internal molecular marker of the region. The molecular marker located in the region may be, for example, both the terminal molecular marker of the region and the internal molecular marker of the region.

The internal molecular marker of the region includes, for example, an SNP marker located between the site of the molecular marker on the upstream side and the site of the molecular marker on the downstream side specifying the region, and can be appropriately determined, for example, based on a located position of the molecular marker shown in FIG. 1. The number of the molecular markers between the sites of the two molecular markers are not limited as long as, for example, it includes at least one, and as a specific example, includes all molecular markers located between sites of the molecular markers specifying the region.

The internal molecular marker of the region includes, for example, a molecular marker located between the site of the molecular marker on the upstream side and the site of the SNP marker on the downstream side specifying the region, and can be designed by a common method in the technical field of the present application. The internal molecular marker of the region can be designed, for example, by referring to Reference Document 5 below. As a specific example, first, a base sequence between two molecular markers selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 of the deposited line is set as a reference sequence, base sequence information obtained by sequencing or the like is mapped, and polymorphisms such as SNPs and/or Indels are detected. Then, the molecular marker can be set, for example, by creating a marker based on the obtained polymorphisms.
Reference Document 5: L. L. Qi et al., "Development and dissection of diagnostic SNP markers for the downy mildew resistance genes PlArg and P18 and maker assisted gene pyramiding in sunflower (Helianthus annuus L.)", Molecular Genetics and Genomics volume 292, pages 551-563 (2017)

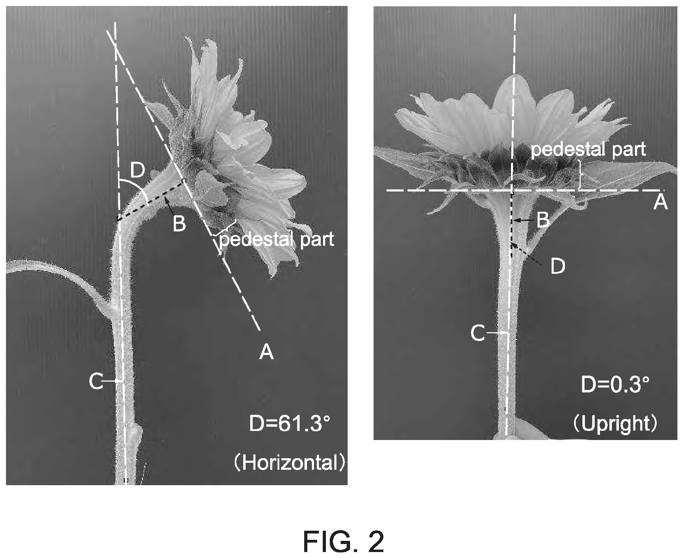

The sunflower plant of the present disclosure shows that a direction of a peduncle is upright (hereinafter, also referred to as an "upright trait"). The direction of the peduncle can be measured by a cultivation test of a target sunflower plant. The cultivation test of the upright trait can be performed under the following cultivation test conditions. The direction of the peduncle can be measured, as shown in FIG. 2, as an angle (peduncle inclination angle: D) at which a line (B) in a direction orthogonal to a bottom surface direction (A) of a pedestal part of a flower and a line (C) in an axial direction of a stem intersect in a peduncle of the target sunflower plant. Measurement of the peduncle inclination angle can be performed under the following measurement conditions. Then, when an average value of the peduncle inclination angle (D) of the target sunflower plant is at most 45°, at most 40°, at most 35°, at most 30°, at most 25°, at most 20°, at most 15°, or at most 10°, the direction of the peduncle of the target sunflower plant can be evaluated as upright. The average value is, for example, an average value of measured values of peduncle inclination angles of at least 20 individuals of target sunflower plants.

### (Cultivation test conditions)

Cultivation start timing (sowing timing): April to May in general regions of Japan (timing satisfying germination temperature: 20 to 25°C and growth temperature: 15 to 30°C)
Cultivation environment: Unheated greenhouse
Plant spacing: 20 cm × 20 cm
Number of rows: 4 rows
Sowing: Direct sowing
Irrigation: From 2 weeks after germination, irrigate only when wilting is observed in leaves

### (Measurement conditions)

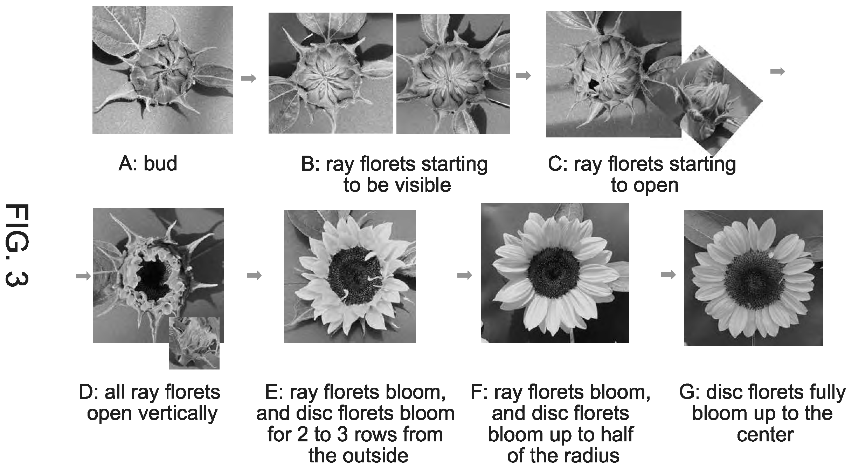

Measurement timing: Stages E to F shown in FIG. 3
Measurement target: Flowers in a central part of a ridge (individuals excluding 3 rows at both ends of each ridge)

In the sunflower plant of the present disclosure, for example, the peduncle angle or average angle is at most 45°, at most 40°, at most 35°, at most 30°, at most 25°, at most 20°, at most 15°, at most 10°, or at most 5°. The average angle is, for example, an average value of measured values of peduncle inclination angles of at least 20 individuals of target sunflower plants.

Examples of the sunflower plant of the present disclosure include a sunflower plant deposited under the Accession No. FERM BP-22482 (deposited line) or a progeny line thereof. Information of deposit is shown below.
Type of deposit: International deposit
Name of depository institution: National Institute of Technology and Evaluation; NITE-IPOD
Address: 2-5-8-120, Kazusakamatari, Kisarazu-shi, Chiba 292-0818, Japan
Accession No.: FERM BP-22482
Indication for identification: Takii34
Date of receipt: August 4, 2023

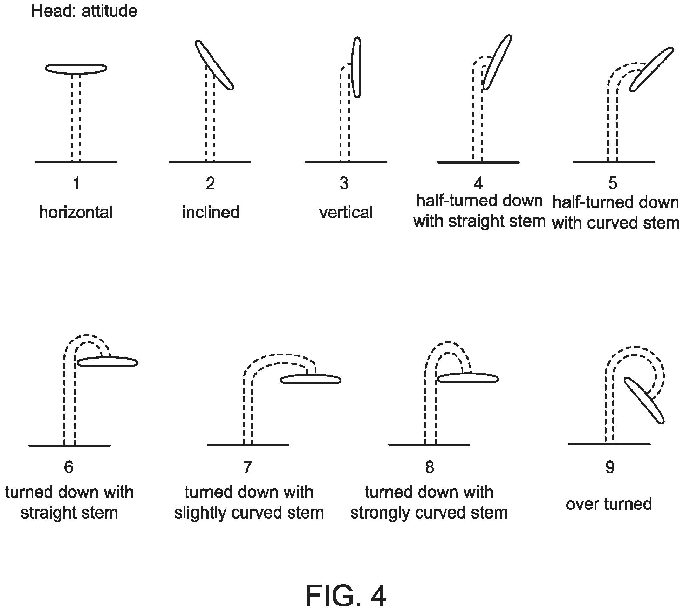

When the sunflower plant of the present disclosure is the deposited line or a progeny line thereof, the sunflower plant of the present disclosure shows morphological and physiological characteristics shown in Table 1 below, for example. In Table 1 below, the morphological and physiological characteristics are based on a trial cultivation in Japan in 2021. In Table 1 below, the morphological and physiological characteristics are evaluated based on the test guidelines for Sunflower *(Helianthus* L.) (issued in March 2007) issued by the Ministry of Agriculture, Forestry and Fisheries of Japan (MAFF). Further, the morphological and physiological characteristics in Table 1 below are evaluated based on the criteria described below. Note that for the morphological and physiological characteristics, FIG. 4 can be referred to.

**[Table 1]**

| Characteristics Number | Characteristics | Characteristic | Deposit line |
|---|---|---|---|
| 20 | Ray floret: color | 1. yellowish white | 5. orange |
| | | 2. light yellow | |
| | | 3. medium yellow | |
| | | 4. orange yellow | |
| | | 5. orange | |
| | | 6. purple | |
| | | 7. reddish brown | |
| | | 8. multicolored | |
| 21 | Disk flower: color | 1. yellow | 3. purple |
| | | 2. orange | |
| | | 3. purple | |
| 24 | Disk flower: production of | 1. absent | 9. present |
| | pollen | 9. present | |
| 33 | Head: attitude | 1. horizontal | 1. horizontal |
| | | 2. inclined | |
| | | 3. vertical | |
| | | 4. half-turned down with straight stem | |
| | | 5. half-turned down with curved stem | |
| | | 6. turned down with straight stem | |
| | | 7. turned down with slightly curved stem | |
| | | 8. turned down with strongly curved stem | |
| | | 9. over turned | |

### (Characteristics Number 20)

The "Ray floret: color" means a color of ray florets that have bloomed. The "Ray floret: color" can be evaluated by visual observation of sunflower plants at F3.2. The "Ray floret: color" can be evaluated based on Class 1 (yellowish white), Class 2 (light yellow), Class 3 (medium yellow), Class 4 (orange yellow), Class 5 (orange), Class 6 (purple), Class 7 (reddish brown), and Class 8 (multicolored). The sunflower at F3.2 means a timing when disk florets in the outermost 3 rows undergo anthesis and become visible, and stigmas expand.

### (Characteristics Number 21)

The "Disk flower: color" means a color of disk florets that have bloomed. The "Disk flower: color" can be evaluated by visual observation of sunflower plants at F3.2. The "Disk flower: color" can be evaluated based on Class 1 (yellow), Class 2 (orange), and Class 3 (purple).

### (Characteristics Number 24)

The "Disk flower: production of pollen" means the presence or absence of pollen of disk florets. The "Disk flower: production of pollen" can be evaluated by visual observation of sunflower plants at F3.2. The "Disk flower: production of pollen" can be evaluated based on Class 1 (absent) and Class 9 (present).

### (Characteristics Number 33)

The "Head: attitude" means a direction of a flower head. In the case of an ornamental variety, the "Head: attitude", can be evaluated by visual observation of sunflower plants at F3.2. The "Head: attitude" can be evaluated, with reference to FIG. 4, based on Class 1 (horizontal), Class 2 (inclined, standard variety: Taiyo), Class 3 (vertical), Class 4 (half-turned down with straight stem), Class 5 (half-turned down with curved stem), Class 6 (turned down with straight stem), Class 7 (turned down with slightly curved stem), Class 8 (turned down with strongly curved stem), and Class 9 (over turned).

In the present disclosure, a plant having "essentially all physiological and morphological characteristics of the deposited line" means a plant having major characteristics of the deposited line when grown in the same environment. The major characteristics include the upright trait, and the characteristics of the Characteristics Numbers 20, 21, 24, and 33. The major characteristics preferably include the upright trait. The plant having "essentially all physiological and morphological characteristics of the deposited line" may be, for example, a plant having the same traits as the deposited line except for at most 5, at most 4, at most 3, at most 2, or one trait, that is, at most 5, at most 4, at most 3, at most 2, or one characteristic(s) may be different from the deposited line. The "characteristic different from the deposited line" may be a major characteristic(s) of the deposited line, or may be a characteristic(s) other than the major characteristic(s) of the deposited line, but the characteristic(s) other than the major traits of the deposited line is preferable. The "characteristic different from the deposited line" can be implemented, for example, by the introduction of characteristic(s) and/or the introduction of genes as described below. In the plant having the "essentially all physiological and morphological characteristics of the deposited line", the upright trait and all characteristics of the Characteristic Numbers 20, 21, 24, and 33 may be the same as the deposited line.

The upright locus of the peduncle is, for example, an upright locus of the peduncle on chromosome 4 of the sunflower plant deposited under the Accession No. FERM BP-22482 above-described.

The sunflower plant of the present disclosure can also be produced, for example, by introducing the upright locus of the peduncle into a sunflower plant. A method for introducing the upright locus of the peduncle into the sunflower plant is not particularly limited, and examples thereof include crossing with the sunflower plant of the present disclosure, tissue culture such as embryo culture, or conventionally known genetic engineering methods. Examples of the genetic engineering methods include methods using recombinant DNA technology, genome editing technology, and the like. Examples of the upright locus of the peduncle to be introduced include the above upright locus of the peduncle. When the introduction is performed by crossing with the sunflower plant of the present disclosure, it is preferable that the sunflower plant of the present disclosure used for crossing comprises, for example, the upright locus of the peduncle in a homozygous form.

Regarding the sunflower plant of the present disclosure, characteristics other than the upright trait, for example, morphological and ecological characteristics, etc., are not particularly limited.

### <Sunflower plant comprising mutant of the Ha412HOChr04g0188691 gene >

In another aspect, the present disclosure provides a sunflower plant in which the peduncle is upright. The sunflower plant of the present disclosure comprises a mutant of the *Ha412HOChr04g0188691* gene (a mutant gene of *Ha412HOChr04g0188691* gene), and the peduncle is upright.

In the present disclosure, the *Ha412HOChr04g0188691* gene includes, for example, a polynucleotide comprising of a base sequence registered as accession number XM_022179978.2 in Genbank as mRNA (SEQ ID NO: 16), a polypeptide comprising of an amino acid sequence registered as accession number XP_022035670.1 as a protein (SEQ ID NO: 19), and the like. The *Ha412HOChr04g0188691* gene is located, for example, on chromosome 4.

As a specific example, the *Ha412HOChr04g0188691* gene of the sunflower plant (wild-type *Ha412HOChr04g0188691* gene) can be exemplified by the polynucleotide of the following (P), or a genomic region encoding thereof.

(P) Any of the polynucleotides of the following (P1) to (P7):
(P1) a polynucleotide comprising of any of the base sequences of SEQ ID NOs: 16 to 18;
(P2) a polynucleotide comprising of a base sequence in which one or several bases are deleted, substituted, inserted and/or added in the base sequence of the (P1), and encoding a protein that controls the upright trait of the peduncle;
(P3) a polynucleotide comprising of a base sequence having at least 80% identity to the base sequence of the (P1), and encoding a protein that controls the upright trait of the peduncle;
(P4) a polynucleotide comprising of a base sequence complementary to a polynucleotide that hybridizes under stringent conditions to the polynucleotide comprising of the base sequence of the (P1), and encoding a protein that controls the upright trait of the peduncle;
(P5) a polynucleotide encoding a protein comprising of any of the amino acid sequences of SEQ ID NOs: 19 to 21;
(P6) a polynucleotide encoding a protein comprising of an amino acid sequence in which one or several amino acids are deleted, substituted, inserted and/or added in the amino acid sequence of the (P5), and that controls the upright trait of the peduncle;
(P7) a polynucleotide encoding a protein comprising of an amino acid sequence having at least 80% identity to the amino acid sequence of the (P5), and that controls the upright trait of the peduncle.

Base sequence of the *Ha412HOChr04g0188691* gene of the sunflower plant (corresponding to Chr04g0181821 in HanXRQr2 database) (SEQ ID NO: 16)

5'-ATGAAGATCTTCAACTGGGTTCATCGTCGATTTCATCACAAATATGAAGTTAGCCAA

GATGTAAAGAAAGCCGAATTTACTGCGAATGGGAAGGATAATCTATCACTTCTTGAAA
ATGAAGGAATGGATGGTGTGTTTGATGGTTGGAAAGAAGGCATACTTGCTATTGGGAC
ATTTGGGTTTGATCCTTCGTTATTGAAAGATTTCGATCATGAAGACGACACATACT
TATGTGAATACACGCGTAAAGAATTATCTATTGATCATGATGAGGATAATGGTGGAGAC
CAAGAAGAAGAAGAAGAAGAAGTTGAGTTTCCATTGGTGTTCAAAGCTTGTAA
GTATGGATTCTTTCATGATCAAAAAGATGATCACACGCAATGTGATGACGTTTGTAAA
CCTAATGATCATGAAGACTGTGTGGATGATCATACTGACCAGATGAAAAAGGGAAAG
AAGGCTGGAGAAAGAACCACTCTTGCTGATCTGTTTTTGGCTGATTCTGATAAGAACT
TGTTGAACAAGAAGTTGACTGATGAAGATGATCAAGAAGTCAAAGTCCACTACATAC
CTGAATCCAACATAAAACATACTTCATATGATGAAAGGGTTGCTTTGATCTCTAAAAA
GAAGATAACGAAAGATGTTGCTGCTCGCCCGATCAAGAAAATTAACCGAATGGTGAG
GAAAATGTTAAAGAAGAAGATCCATCCCGACGTCGAAAATCAAAAGGATAAATGCAG
GTTTGAAGCCATGCGCGTACATGGATGA-3’

Base sequence of the *Ha412HOChr04g0188691* gene of the sunflower plant (isolated from horizontal sunflower plant) (SEQ ID NO: 17)

5’-ATGAAGATCTTCAACTGGGTTCATCGTCGATTTCATCACAAATATGAAGTTAGCCAA
GATGTGAAGAAAGCCGAATTTACTGCGAATGGGAAGGACAATCTATCACTTCTTGAA
AATGAAGGAATGGATGGTGTGTTTGATGGTTGGAAAGAAGGCATACTTGCTATTGGG
ACATTTGGGTTTGATCCTTCGTTATTGAAAGATTTCGATCATGAAGACGACACAT
ACTTATGTGAATACACGCGTAAAGAATTATCTATTGATCATGATGAGGATAATGGTGGA
GACCAAGAAGAAGAAGAAGAAGAAGTTGAGTTTCCATTGGTGTTCAAAGCTTG
TAAGTATGGGTTCTTTCATGATAAAAAAGATGATCACACGCAATGTGATGACGTTTGT
AAACCTAATGATCATGAAGACTGTGTGGATGATCATACTGACCAGATGAAAAAGGGA
AAGAAGGCTGGAGAAAGAACCACTCTTGCTGATCTGTTTTTGGCTGATTCTGATAAG
AACTTGTTGAACAAGAAGTTGACTGATGAAGATGATCAAGAAGTCAAAGTCCACTAC
ATACCTGAATCCAACATAAAACATACTTCATGTGATGAAAGGGTTGCTTTGATCTCTAA
AAAGAAGATAACGAAAGATGTTGCTGCTCGCCCGATCAAGAAAATTAACCGAATGGT
GAGGAAAATGTTGAAGAAGAAGATCCATCCCGACGTCGAAAATCAAAAGGATAAAT
GCAGGTTTGAAGCCATGCGCGTACATGGATGA-3’

Base sequence of the *Ha412HOChr04g0188691* gene of the sunflower plant (corresponding to Chr04g0188691 in Ha412HOv2.0 database) (SEQ ID NO: 18)

5’-ATGAAGATCTTCAACTGGGTTCACCGTCGATTTCATCACAAATATGAAGTTAGCCA
AGATGTGAAGAAAGCCGAATTTACCGTGAATGGGAAGGACAATCTATCACTTCTTGA
AAATGAAGGAATGGATGGTGTGTTTGATGGTTGGAAAGAAGGCATACTTGCTATTGG

GACATTTGGGTTTGATCCTTCGTTATTGAAAGATTTCGATCATGAAGACGACGACACA
TACTTATGTGAATACACGCGTAAAGAATTATCTATTGATCATGATGAGGATAATGGTGG
AGACCAAGAAGAAGAAGAAGAAGAAGTTGAGTTTCCATTGGTGTTCAAAGCTT
GTAAGTATGGGTTCTTTCATGATAAAAAAGATGATCACACGCAATGTGATGACGTTTG
TAAACCTAATGATCATGAAGACTGTGTGGATGATCATACTGACCAGATGAAAAAGGG
AAAGAAGGCTGGAGAAAGAACCACTCTTGCTGATCTGTTTTTGGCTGATTCTGATAA
GAACTTGTTGAACAAGAAGTTGACTGATGAAGATGATCAAGAAGTCAAAGTCCACTA
CATACCTGAATCCAACATAAAACATACTTCATATGATGAAAGGGTTGCTTTGATCTCTA
AAAAGAAGATAACGAAAGATGTTGCTGCTCGCCCGATCAAGAAAATTAACCGAATGG
TGAGGAAAATGTTAAAGAAGAAGATCCATCCCGACGTCGAAAATCAAAAGGATAAAT
GCAGGTTTGAAGCCATGCGCGTACATGGATGA-3’

In the (P1), the base sequence of SEQ ID NO: 16 is a base sequence encoding the amino acid sequence of SEQ ID NO: 19 of the (P5). In the (P1), the base sequence of SEQ ID NO: 17 is a base sequence encoding the amino acid sequence of SEQ ID NO: 20 of the (P5). In the (P1), the base sequence of SEQ ID NO: 18 is a base sequence encoding the amino acid sequence of SEQ ID NO: 21 of the (P5). The base sequences of SEQ ID NOs: 16 to 18 can be obtained, for example, from the sunflower plant *(Helianthus annuus).*

In the (P2), "one or several" only needs to be within a range in which a protein encoded by the polynucleotide of the (P2) can control the upright trait of the peduncle. The control of the upright trait of the peduncle means, for example, a function that can inhibit, suppress, or decrease expression of the upright trait of the peduncle (the same applies hereinafter). The "one or several" of the (P2) is, in the base sequence of the (P1), for example, 1 to 155, 1 to 116, 1 to 77, 1 to 38, 1 to 31, 1 to 23, 1 to 15, 1 to 7, 1 to 5, 1 to 3, 1 or 2, or 1 base.

In the (P3), "identity" only needs to be within a range in which a protein encoded by the polynucleotide of the (P3) can control the upright trait of the peduncle. The identity of the (P3) is, relative to the base sequence of the (P1), for example, at least 80%, at least 85%, at least 90%, at least 95%, at least 96%, at least 97%, at least 98%, or at least 99%.

In the (P4), "stringent conditions" may be, for example, any of low stringent conditions, medium stringent conditions, and high stringent conditions. "Low stringent conditions" are, for example, conditions of 5 × SSC, 5 × Denhardt's solution, 0.5% SDS, 50% formamide, and 32°C. "Medium stringent conditions" are, for example, conditions of 5 × SSC, 5 × Denhardt's solution, 0.5% SDS, 50% formamide, and 42°C. "High stringent conditions" are, for example, conditions of 5 × SSC, 5 × Denhardt's solution, 0.5% SDS, 50% formamide, and 50°C. The degree of stringency can be set by those skilled in the art, for example, by appropriately selecting conditions such as temperature, salt concentration, probe concentration and length, ionic strength, and time. For "stringent conditions", for example, the conditions described in "Molecular Cloning: A Laboratory Manual 2nd Ed." edited by Sambrook et al. [Cold Spring Harbor Laboratory Press (1989)] and the like can also be adopted.

The polynucleotide of the (P5) only needs to be within a range in which a protein encoded by the polynucleotide of the (P5) can control the upright trait of the peduncle. The base sequence of the polynucleotide of the (P5) can be designed, for example, by replacing with corresponding codons based on any of the amino acid sequences of SEQ ID NOs: 19 to 21.

Amino acid sequence of the Ha412HOChr04g0188691 protein (corresponding to Chr04g0181821 in HanXRQr2 database) (SEQ ID NO: 19)

MKIFNWVHRRFHHKYEVSQDVKKAEFTANGKDNLSLLENEGMDGVFDGWKEGILAIG
TFGFDPSLLKDFDHEDDDTYLCEYTRKELSIDHDEDNGGDQEEEEEEEVEFPLVFKACKY
GFFHDQKDDHTQCDDVCKPNDHEDCVDDHTDQMKKGKKAGERTTLADLFLADSDKN
LLNKKLTDEDDQEVKVHYIPESNIKHTSYDERVALISKKKITKDVAARPIKKINRMVRKM
LKKKIHPDVENQKDKCRFEAMRVHG

Amino acid sequence of the Ha412HOChr04g0188691 protein (isolated from the horizontal sunflower plant) (SEQ ID NO: 20)

MKIFNWVHRRFHHKYEVSQDVKKAEFTANGKDNLSLLENEGMDGVFDGWKEGILAIG
TFGFDPSLLKDFDHEDDDTYLCEYTRKELSIDHDEDNGGDQEEEEEEEVEFPLVFKACKY
GFFHDKKDDHTQCDDVCKPNDHEDCVDDHTDQMKKGKKAGERTTLADLFLADSDKN
LLNKKLTDEDDQEVKVHYIPESNIKHTSCDERVALISKKKITKDVAARPIKKINRMVRKM
LKKKIHPDVENQKDKCRFEAMRVHG

Amino acid sequence of the Ha412HOChr04g0188691 protein (corresponding to Chr04g0188691 in Ha412HOv2.0 database) (SEQ ID NO: 21)

MKIFNWVHRRFHHKYEVSQDVKKAEFTVNGKDNLSLLENEGMDGVFDGWKEGILAIG
TFGFDPSLLKDFDHEDDDTYLCEYTRKELSIDHDEDNGGDQEEEEEEEVEFPLVFKACKY
GFFHDKKDDHTQCDDVCKPNDHEDCVDDHTDQMKKGKKAGERTTLADLFLADSDKN
LLNKKLTDEDDQEVKVHYIPESNIKHTSYDERVALISKKKITKDVAARPIKKINRMVRKM
LKKKIHPDVENQKDKCRFEAMRVHG

In the (P6), "one or several" regarding the amino acid sequence only needs to be within a range in which a protein encoded by the polynucleotide of the (P1) can control the upright trait of the peduncle. The "one or several" of the (P6) is, in any of the amino acid sequences of SEQ ID NOs: 19 to 21, for example, 1 to 51, 1 to 38, 1 to 25, 1 to 12, 1 to 10, 1 to 7, 1 to 5, 1 to 3, 1 or 2, or 1 amino acid.

In the (P7), "identity" regarding the amino acid sequence only needs to be within a range in which a protein encoded by the polynucleotide of the (P7) can control the upright trait of the peduncle. The identity of the (P7) is, relative to any of the amino acid sequences of SEQ ID NOs: 19 to 21, for example, at least 80%, at least 85%, at least 90%, at least 95%, at least 96%, at least 97%, at least 98%, or at least 99%.

The *Ha412HOChr04g0188691* gene is estimated to control the direction of the peduncle. More specifically, the *Ha412HOChr04g0188691* gene, a wild-type gene, for example, makes the direction of the peduncle a direction that is not upright, i.e., horizontal, and it is estimated that when a mutation that reduces or loses the function of the wild-type gene is introduced, the direction of the peduncle is made upright. For this reason, the "control of the upright trait of the peduncle" can also be called, for example, "suppression activity of the upright position of the peduncle". Further, a wild-type *Ha412HOChr04g0188691* gene can also be called, for example, an upright suppression gene of the peduncle.

In the present disclosure, "control of the upright trait of the peduncle" may be evaluated directly based on, for example, the direction of the peduncle of a target sunflower plant (test sunflower plant), or may be evaluated indirectly based on the expression of the *Ha412HOChr04g0188691* gene, or a protein encoded by the *Ha412HOChr04g0188691* gene, in a plant sample of the test sunflower plant. The plant sample of the test sunflower plant is not particularly limited, and may be, for example, either the entire sunflower plant of the test sunflower plant or a plant part of the sunflower plant, and is preferably a stem of the sunflower plant. The type of plant sample to be used may be, for example, one type or two or more types.

When the evaluation is performed based on the direction of the peduncle of the test sunflower plant, the control of the upright trait of the peduncle can be implemented according to the measurement method of the peduncle of a sunflower described above. Specifically, a test sunflower plant cultivated under the cultivation test conditions is measured using the measurement conditions, and when the average value of the peduncle inclination angle (D) of the test sunflower plant is at least more than 45°, at least 50°, at least 55°, at least 60°, at least 65°, at least 70°, at least 75°, at least 80°, at least 85°, or at least 90°, the test sunflower plant can be evaluated as being capable of, for example, controlling the upright trait of the peduncle. On the other hand, when the average value of the peduncle inclination angle (D) of the test sunflower plant is at most 45°, at most 40°, at most 35°, at most 30°, at most 25°, at most 20°, at most 15°, or at most 10°, the test sunflower plant can be evaluated as being not capable to, for example, control the upright trait of the peduncle. The average value is, for example, an average value of measured values of peduncle inclination angles of at least 20 individuals of target sunflower plants.

When the evaluation is performed based on the expression of the *Ha412HOChr04g0188691* gene, the expression of the *Ha412HOChr04g0188691* gene can be implemented by, for example, detecting expression of mRNA of the *Ha412HOChr04g0188691* gene. Extraction of mRNA from a plant sample of the sunflower plant can be implemented by a common method. Detection of expression of mRNA of the *Ha412HOChr04g0188691* gene includes, for example, detection such as semi-quantitative PCR, quantitative PCR, Northern blotting, digital PCR, and RNA sequence analysis (RNAseq). Primers and/or probes used for detection of expression of the mRNA can be designed by, for example, a general technique in the present technical field. Then, when the expression level of *Ha412HOChr04g0188691* gene in the plant sample of the test sunflower plant is the same as (no significant difference) the expression level of the *Ha412HOChr04g0188691* gene in a sunflower plant having the wild-type *Ha412HOChr04g0188691* gene in a homozygous form, when the expression level of the *Ha412HOChr04g0188691* gene in the plant sample of the test sunflower plant is (significantly) higher than the expression level of the *Ha412HOChr04g0188691* gene in a sunflower plant having the wild-type *Ha412HOChr04g0188691* gene in a homozygous form, and/or, when the expression level of the *Ha412HOChr04g0188691* gene in the plant sample of the test sunflower plant is (significantly) higher than the expression level of the *Ha412HOChr04g0188691* gene in a plant sample of a sunflower plant having a loss of function mutant of the *Ha412HOChr04g0188691* gene in a homozygous form or a heterozygous form, the test sunflower plant can be evaluated as being capable of, for example, controlling the upright trait of the peduncle. On the other hand, when the expression level of the *Ha412HOChr04g0188691* gene in the plant sample of the test sunflower plant is (significantly) lower than the expression level of the *Ha412HOChr04g0188691* gene in a plant sample of a sunflower plant having the wild-type *Ha412HOChr04g0188691* gene in a homozygous form, when the expression level of the *Ha412HOChr04g0188691* gene in the plant sample of the test sunflower plant is the same as (no significant difference) the expression level of the *Ha412HOChr04g0188691* gene in a plant sample of a sunflower plant having a loss of function mutant of the *Ha412HOChr04g0188691* gene in a homozygous form or a heterozygous form, and/or, when the expression level of *Ha412HOChr04g0188691* gene in the plant sample of the test sunflower plant is (significantly) lower than the expression level of the *Ha412HOChr04g0188691* gene in a plant sample of a sunflower plant having a loss of function mutant of the *Ha412HOChr04g0188691* gene in a homozygous form or a heterozygous form, the test sunflower plant can be evaluated as being not capable to, for example, control the upright trait of the peduncle.

When evaluation is performed based on expression of the protein encoded by the *Ha412HOChr04g0188691* gene, expression of the protein encoded by the *Ha412HOChr04g0188691* gene can be detected by, for example, a method using a spectrophotometer such as an ultraviolet absorption method or a bicinchoninic acid method, ELISA, Western blotting, or the like. Preparation of an extract comprising protein from the sunflower plant can be implemented by a general technique in the present technical field such as ultrasonic disruption or physical disruption using a homogenizer. And, when the expression level of the protein encoded by the *Ha412HOChr04g0188691* gene in the plant sample of the test sunflower plant is the same as (no significant difference) the expression level of the protein encoded by the *Ha412HOChr04g0188691* gene in a sunflower plant having the wild-type *Ha412HOChr04g0188691* gene in a homozygous form, when the expression level of the protein encoded by the *Ha412HOChr04g0188691* gene in the plant sample of the test sunflower plant is (significantly) higher than the expression level of the protein encoded by the *Ha412HOChr04g0188691* gene in a sunflower plant having the wild-type *Ha412HOChr04g0188691* gene in a homozygous form, and/or, when it is (significantly) higher than the expression level of the protein encoded by the *Ha412HOChr04g0188691* gene in a plant sample of a sunflower plant having a loss of function mutant of the *Ha412HOChr04g0188691* gene in a homozygous form or a heterozygous form, the test sunflower plant can be evaluated as being capable of, for example, controlling the upright trait of the peduncle. On the other hand, when the expression level of the protein encoded by the *Ha412HOChr04g0188691* gene in the plant sample of the sunflower plant is (significantly) lower than the expression level of the protein encoded by the *Ha412HOChr04g0188691* gene in the plant sample of a sunflower plant having the wild-type *Ha412HOChr04g0188691* gene in a homozygous form, when it is the same as (no significant difference) the expression level of the protein encoded by the *Ha412HOChr04g0188691* gene in a plant sample of a sunflower plant having a loss of function mutant of the protein encoded by the *Ha412HOChr04g0188691* gene in a homozygous form or a heterozygous form, and/or, when it is (significantly) lower than the expression level of the protein encoded by the *Ha412HOChr04g0188691* gene in a plant sample of a sunflower plant having a loss of function mutant of the *Ha412HOChr04g0188691* gene in a homozygous form or a heterozygous form, the test sunflower plant can be evaluated as being not capable to, for example, control the upright trait of the peduncle.

Further, when the evaluation is performed based on expression of the protein encoded by the *Ha412HOChr04g0188691* gene, when the molecular weight of the protein encoded by *Ha412HOChr04g0188691* gene in the plant sample of the test sunflower plant is the same as the molecular weight of the protein encoded by the *Ha412HOChr04g0188691* gene in a sunflower plant having the wild-type *Ha412HOChr04g0188691* gene in a homozygous form, the test sunflower plant can be evaluated as being capable of, for example, controlling the upright trait of the peduncle. On the other hand, when the molecular weight of the protein encoded by the *Ha412HOChr04g0188691* gene in the plant sample of the sunflower plant is larger than or smaller than the molecular weight of the protein encoded by the *Ha412HOChr04g0188691* gene in the plant sample of a sunflower plant having the wild-type *Ha412HOChr04g0188691* gene in a homozygous form, the test sunflower plant can be evaluated as being not capable to, for example, control the upright trait of the peduncle.

In the present disclosure, the *Ha412HOChr04g0188691* gene may exist in the form of RNA (for example, mRNA) or in the form of DNA (cDNA or genomic DNA). DNA may be double-stranded or single-stranded. In the present disclosure, the gene may comprise an additional sequence, such as a sequence of an untranslated region (UTR).

The mutation of the *Ha412HOChr04g0188691* gene may mean a state in which the function of the *Ha412HOChr04g0188691* gene is (significantly) reduced or lost to such an extent that a sunflower plant having a mutant of the *Ha412HOChr04g0188691* gene has an upright peduncle compared to a sunflower plant having a wild-type *Ha412HOChr04g0188691* gene (hereinafter, also called a "wild-type sunflower plant"). Specifically, the mutation of the *Ha412HOChr04g0188691* gene may mean, for example, a state in which the expression level of mRNA of the *Ha412HOChr04g0188691* gene or a protein encoded by the gene is (significantly) decreased, or a state in which mRNA of the *Ha412HOChr04g0188691* gene or a protein encoded by the gene is not expressed at all, or a state in which the expression level of mRNA of the functional *Ha412HOChr04g0188691* gene or a protein encoded by the *Ha412HOChr04g0188691* gene is decreased, or a state in which mRNA of functional *Ha412HOChr04g0188691* gene or a protein encoded by the *Ha412HOChr04g0188691* gene is not expressed at all. For this reason, in the present disclosure, the loss of function of the *Ha412HOChr04g0188691* gene may be implemented by introducing a loss-of-function mutation into the *Ha412HOChr04g0188691* gene, or may be implemented by introducing a polynucleotide that suppresses expression of the *Ha412HOChr04g0188691* gene. The "suppress expression of gene" may be suppression of the transcription of a gene, or may be suppression of translation into a protein.

In the present disclosure, the sunflower plant comprising the mutation of the *Ha412HOChr04g0188691* gene can also be called, for example, a sunflower plant having a mutant of the *Ha412HOChr04g0188691* gene. The sunflower plant comprising the mutation of the *Ha412HOChr04g0188691* gene may have, for example, a mutant of the *Ha412HOChr04g0188691* gene in a heterozygous form, or may have the *Ha412HOChr04g0188691* gene in a homozygous form. The sunflower plant comprising the mutation of the *Ha412HOChr04g0188691* gene may further have genes modified, altered, introduced, and/or lost in function other than the *Ha412HOChr04g0188691* gene.

The mutation of the *Ha412HOChr04g0188691* gene can be caused by, for example, introducing a mutation such as a loss-of-function mutation into the *Ha412HOChr04g0188691* gene. The type of the mutation is not particularly limited, and includes, for example, a point mutation, a missense mutation, a nonsense mutation, a frameshift mutation, a splicing mutation, base deletion over a wide range (large deletion), and the like. The mutation may, for example, cause deletion of a part of the *Ha412HOChr04g0188691* gene, or may cause deletion of the entirety thereof. The frameshift mutation is a mutation that occurs when base deletion or insertion occurs and a triplet reading frame (codon) is shifted. The frameshift mutation has a very large influence on gene function compared to base pair substitution mutations. This is because when the frameshift mutation occurs, in the gene, genetic codes after the location where the frameshift mutation was introduced are significantly shifted, which not only changes amino acids but also shifts stop codons and the like. A mutant of the *Ha412HOChr04g0188691* gene can also be called, for example, a loss of function mutant of the *Ha412HOChr04g0188691* gene if loss of function is caused by the mutation.

A mutant of the *Ha412HOChr04g0188691* gene is, for example, a gene in which a mutation, such as insertion, deletion, and/or substitution of one or several bases (hereinafter, also called "at least one base"), is introduced relative to a base sequence of a wild-type *Ha412HOChr04g0188691* gene. For at least one base, for example, the description of the number of bases in the description of the (P2) can be applied. The frameshift mutation occurs, for example, by insertion or deletion of 3m+1 bases or 3m+2 bases (m is an integer of 0 or more).

In the present disclosure, a mutation to the *Ha412HOChr04g0188691* gene can be caused by, for example, introducing a mutation into a target gene in the genome of a target sunflower plant by a common method. The method for introducing the mutation can be implemented by, for example, homologous recombination; genome editing technology using ZFN, TALEN, CRISPR-CAS9, CRISPR-CPF1, or the like; and the like. The method for introducing the mutation may be implemented by, for example, a mutation introduction method such as a site-directed mutagenesis method. Further, the method for introducing the mutation may be implemented by, for example, a random mutagenesis method. The random mutagenesis method includes, for example, radiation irradiation treatment with α rays, β rays, γ rays, X-rays, or the like; chemical substance treatment with a mutagen such as ethyl methanesulfonate (EMS) or ethinyl nitrosourea (ENU); heavy ion beam treatment; and the like. The introduction of a mutation using the genome editing technology can be implemented specifically by, for example, introducing a protein and a nucleic acid constituting the genome editing technology, or a vector encoding thereof. The protein includes, for example, a CRISPR (Clustered Regularly Interspaced Short Palindromic Repeats) enzyme, and specific examples thereof include Cas1, Cas1B, Cas2, Cas3, Cas4, Cas5, Cas6, Cas7, Cas8, Cas9, Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, and the like. The nucleic acid includes, for example, crRNA and tracrRNA, or a single-stranded nucleic acid in which these are linked via a linker. In this case, in the nucleic acid, for example, a base sequence that anneals to a target sequence in crRNA is designed to be a base sequence complementary to a base sequence encoding the *Ha412HOChr04g0188691* gene. As the nucleic acid, one type may be used alone, or two or more types may be used in combination. When the genome editing technology is used, for example, by using two or more types of nucleic acids, a large deletion of a base sequence between target sequences can be induced. The method for introducing the mutation may be implemented by, for example, a mutation introduction method such as a site-directed mutagenesis method.

The location of the mutation of the *Ha412HOChr04g0188691* gene is not particularly limited, and can be set in an arbitrary region related to each gene. As a specific example, the location of the mutation in the *Ha412HOChr04g0188691* gene includes, for example, an expression control region such as a promoter region of the *Ha412HOChr04g0188691* gene, an exon region including a coding region encoding a protein encoded by the *Ha412HOChr04g0188691* gene, a non-coding region not encoding a protein encoded by the *Ha412HOChr04g0188691* gene (for example, an intron region, an enhancer region, etc.), and the like. The exon region includes, for example, a first exon region, a second exon region, a third exon region, or a fourth exon region.

As a specific example, the location of the mutation in the *Ha412HOChr04g0188691* gene is, for example, in the base sequence of SEQ ID NO: 22, the 300th to 400th, preferably the 340th to 360th, and more preferably the 353rd base. The mutation introduced at these mutation locations is preferably a nonsense mutation, a frameshift mutation, or a splicing mutation.

Base sequence of insertion sequence at mutation location of the *Ha412HOChr04g0188691* gene of sunflower plant (SEQ ID NO: 13)

5’-
CAGTGGCGGACCCAAGAATTTTTCCATGGGGGTGCGAAACATTTTTAAAAATTTTAGG
CCCCTAGGTATATAAGTAAAAAAATCGGTTCGTATCGGGTCGGTTCGGGTCGGGTCAT
GTAAAACAAAAGAAATTGCGCCATAACGTCCCTACTCATGGTTTCTATCACAAACAAA

TTGATGCATAAGTTTATCGCTCCATAAAATAATGTTTCAATTTTAATTTTCAACCCTAGA
AATCGTGTGTAATCAGCCTAAAAATTATCTAAACAACATAATCACCCTAAAAATCATCT
AAACAACCATAAACATCGTCAAAACACATGAAATTTCAAACCTATTTAAGAATTTTGT
TACCCTTTGGTGTCGGACGGCGGTGGTTCGCGGCTGCTGATGACGTGCTGTTGTGGTC
GATGATCGCTTGTAGGAGACGACCCAAGAGAGGGAGGGCGGGATGAATTCTAAGGG
ΛCTΛTTGGGCTTΛTTTΛΛTTΛTTΛTΛCTTTGΛΛCTTΛGGTTGΛGTTTGGTTΛΛTGGGCT
AGTAATTAGTGGGCTAATTATGTTTAATGAAATAGTGGGTTAAGGTATTGGGTATTTGG
GTTAAGTTGGGTAGTATAAGTTTATATAATTTAGATAGTTTTTTTAATTAGAGTTTACTA
AAAAATTTAAAATATAAATCGAAAACATTTTTTACCTAAGGTGTGTGGACGAAAAATT
CCAGGGGTGCAGATGAAAAAATTCAAGGGGTGCGGACGAAAAAATCCAAAGGGTGC
AGACGAGATTTTCAACGGAAAATAGCACTAAATTTTTTTTTCCCGGGGGTGCGCCCGC
CCACCTTGGGCTATATGTATGTCCGCCCTTGTAAGTATG-3’

As the mutant of the *Ha412HOChr04g0188691* gene of the sunflower plant, for example, the polynucleotide of (M) below, or a genomic region encoding these, can be exemplified.

(M) any of polynucleotides (M1) to (M7) below:
(M1) a polynucleotide comprising of the base sequence of SEQ ID NO: 22;
(M2) a polynucleotide comprising of a base sequence in which one or several bases are deleted, substituted, inserted and/or added in the base sequence of the (M1);
(M3) a polynucleotide comprising of a base sequence having an identity of at least 80% to the base sequence of the (M1);
(M4) a polynucleotide comprising of a base sequence complementary to a polynucleotide that hybridizes under stringent conditions to the polynucleotide comprising of the base sequence of the (M1);
(M5) a polynucleotide encoding a protein comprising of the amino acid sequence of SEQ ID NO: 23;
(M6) a polynucleotide comprising of an amino acid sequence in which one or several amino acids are deleted, substituted, inserted and/or added in the amino acid sequence of the (M5);
(M7) a polynucleotide encoding a protein comprising of an amino acid sequence having an identity of at least 80% to the amino acid sequence of the (M5).

Base sequence of the *Ha412HOChr04g0188691* gene of a sunflower plant show in g an upright trait (SEQ ID NO: 22)

5'-ATGAAGATCTTCAACTGGGTTCATCGTCGATTTCATCACAAATATGAAGTTAGCCAA
GATGTGAAGAAAGCCGAATTTACTGCGAATGGGAAGGACAATCTATCACTTCTTGAA
AATGAAGGAATGGATGGTGTGTTTGATGGTTGGAAAGAAGGCATACTTGCTATTGGG

ACATTTGGGTTTGATCCTTCGTTATTGAAAGATTTCGATCATGAAGACGACGACACAT
ACTTATGTGAATACACGCGTAAAGAATTATCTATTGATCATGATGAGGATAATGGTGGA
GACCAAGAAGAAGAAGAAGAAGAAGTTGAGTTTCCATTGGTGTTCAAAGCTTG
TAAGTATGCAGTGGCGGACCCAAGAATTTTTCCATGGGGGTGCGAAACATTTTTAAAA
ATTTTAGGCCCCTAGGTATATAAGTAAAAAAATCGGTTCGTATCGGGTCGGTTCGGGTC
GGGTCATGTAAAACAAAAGAAATTGCGCCATAACGTCCCTACTCATGGTTTCTATCAC
AAACAAATTGATGCATAAGTTTATCGCTCCATAAAATAATGTTTCAATTTTAATTTTCAA
CCCTAGAAATCGTGTGTAATCAGCCTAAAAATTATCTAAACAACATAATCACCCTAAA
AATCATCTAAACAACCATAAACATCGTCAAAACACATGAAATTTCAAACCTATTTAAG
AATTTTGTTACCCTTTGGTGTCGGACGGCGGTGGTTCGCGGCTGCTGATGACGTGCTG
TTGTGGTCGATGATCGCTTGTAGGAGACGACCCAAGAGAGGGAGGGCGGGATGAATT
CTAAGGGACTATTGGGCTTATTTTAATTATTATACTTTGAACTTAGGTTGAGTTTGGTTA
ATGGGCTAGTAATTAGTGGGCTAATTATGTTTAATGAAATAGTGGGTTAAGGTATTGGG
TATTTGGGTTAAGTTGGGTAGTATAAGTTTATATAATTTAGATAGTTTTTTTAATTAGAG
TTTACTAAAAAATTTAAAATATAAATCGAAAACATTTTTTACCTAAGGTGTGTGGACGA
AAAATTCCAGGGGTGCAGATGAAAAAATTCAAGGGGTGCGGACGAAAAAATCCAAA
GGGTGCAGACGAGATTTTCAACGGAAAATAGCACTAAATTTTTTTTTCCCGGGGGTGC
GCCCGCCCACCTTGGGCTATATGTATGTCCGCCCTTGTAAGTATGGGTTCTTTCATGAT
AAAAAAGATGATCACACGCAATGTGATGACGTTTGTAAACCTAATGATCATGAAGACT
GTGTGGATGATCATACTGACCAGATGAAAAAGGGAAAGAAGGCTGGAGAAAGAACC
ACTCTTGCTGATCTGTTTTTGGCTGATTCTGATAAGAACTTGTTGAACAAGAAGTTGA
CTGATGAAGATGATCAAGAAGTCAAAGTCCACTACATACCTGAATCCAACATAAAAC
ATACTTCATGTGATGAAAGGGTTGCTTTGATCTCTAAAAAGAAGATAACGAAAGATGT
TGCTGCTCGCCCGATCAAGAAAATTAACCGAATGGTGAGGAAAATGTTGAAGAAGAA
GATCCATCCCGACGTCGAAAATCAAAAGGATAAATGCAGGTTTGAAGCCATGCGCGT
ACATGGATGA-3'

The polynucleotides of the (M1) to (M7) are preferably polynucleotides encoding a protein that expresses the upright trait.

In the polynucleotides of the (M1) to (M7), "expressing an upright trait" means, for example, that the control of the upright trait is significantly suppressed as compared to the protein encoded by the polynucleotide of the (P1) to (P7), and preferably means t hat the control of the upright trait is completely lost.

The "one or several" of the (M2) is, in the base sequence of the SEQ ID NO: 22, for example, 1 to 326, 1 to 244, 1 to 163, 1 to 81, 1 to 65, 1 to 48, 1 to 32, 1 to 16, 1 to 10, 1 to 8, 1 to 5, 1 to 3, 1 or 2, or 1 base.

The identity of the (M3) is, relative to the base sequence of the SEQ ID NO: 2 2, for example, at least 80%, at least 85%, at least 90%, at least 95%, at least 96%, at least 97%, at least 98%, or at least 99%.

In the (M4), the "hybridizing polynucleotide" is, for example, a polynucleotide completely or partially complementary to the polynucleotide comprising of the base sequence of the SEQ ID NO: 22. The hybridization can be detected by, for example, various hybridization assays. In the (M4), for the hybridizing and stringent conditions, the description in the (P4) can be applied.

The base sequence of the polynucleotide of the (M5) can be designed, for example, by replacing with corresponding codons based on the amino acid sequence of SEQ ID NO: 23.

Amino acid sequence of the Ha412HOChr04g0188691 protein of a sunflower plant showing an upright trait (SEQ ID NO: 23)

MKIFNWVHRRFHHKYEVSQDVKKAEFTANGKDNLSLLENEGMDGVFDGWKEGILAIG
TFGFDPSLLKDFDHEDDDTYLCEYTRKELSIDHDEDNGGDQEEEEEEEVEFPLVFKACKY
AVADPRIFPWGCETFLKILGP

The "one or several" of the (M6) is, for example, in the amino acid sequence of the SEQ ID NO: 23, for example, 1 to 27, 1 to 20, 1 to 13, 1 to 10, 1 to 6, 1 to 5, 1 to 3, 1 or 2, or 1 amino acid.

The identity of the (M7) is, relative to the amino acid sequence of the SEQ ID NO: 23, for example, at least 80%, at least 85%, at least 90%, at least 95%, at least 96%, at least 97%, at least 98%, or at least 99%.

In the present disclosure, when the function of the *Ha412HOChr04g0188691* gene is lost by suppressing expression of the *Ha412HOChr04g0188691* gene, the loss of function of the *Ha412HOChr04g0188691* gene can be implemented by, for example, introducing a polynucleotide that suppresses expression of the *Ha412HOChr04g0188691* gene into a target sunflower plant. The method for introducing the polynucleotide is not particularly limited and can be implemented by, for example, a method such as RNA interference, antisense RNA, or genome editing technology. An expression cassette, such as an expression vector comprising the polynucleotide, can be introduced into a target sunflower plant by, for example, microinjection, a polyethylene glycol method, an electroporation method (electroporation method), an *Agrobacterium* method, a particle gun method, or the like. The target sunflower plant may be, for example, any of a plant cell, a callus, a plant tissue, and a plant individual.

As described above, the sunflower plant of the present disclosure is obtained by the occurrence of a mutation in a wild-type *Ha412HOChr04g0188691* gene. For this reason, the sunflower plant of the present disclosure can also be called, for example, a "mutant of a sunflower plant showing an upright trait." Further, the sunflower plant of the present disclosure is a progeny line of a "mutant of a sunflower plant showing an upright trait", and may be a sunflower plant having a mutation in the *Ha412HOChr04g0188691* gene. The sunflower plant of the present disclosure can also be called, for example, a mutant of a sunflower plant having a genetic mutation introduced into the base sequence of the *Ha412HOChr04g0188691* gene by the above-described method for introducing a mutation. The sunflower plant of the present disclosure can also be called, for example, a sunflower plant showing an upright trait, excluding sunflower plants obtained only by means of an essentially biological process.

For the method for producing the sunflower plant of the present disclosure, the description of the method for producing described below can be applied.

### <Plant population of sunflower plants>

In another aspect, the present disclosure provides a plant population of sunflower plants in which the peduncle is upright. The plant population of sunflower plants of the present disclosure comprises sunflower plants in which the peduncle is upright, and in the plant population, a ratio of individuals in which the peduncle angle is 0 to 45° is at least 50%. For the plant population of sunflower plants of the present disclosure, the description of the sunflower plant of the present disclosure in which the peduncle is upright can be applied.

The population of sunflower plants of the present disclosure is comprised of, for example, a part or all of the plants from the sunflower plant of the present disclosure. The population of sunflower plants of the present disclosure may be, for example, comprised of two or more sunflower plant individuals, or may be comprised of two or more plant parts of sunflower plants (for example, flowers and stems, seeds, etc.).

The number of plants constituting the plant population is at least two, and the upper limit is not particularly limited. The number of plants constituting the plant population is, for example, 10 to 200 individuals, 20 to 150 individuals, or 20 to 100 individuals.

In the plant population of sunflower plants of the present disclosure, for example, when a ratio of individuals in which the peduncle angle is 0 to 45° is at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, or at least 99%, the plant population of sunflower plants can be evaluated as being a plant population of sunflower plants showing the upright trait. The plant population of sunflower plants is, for example, 10 to 200, 20 to 150, or 50 to 100 sunflower plants.

### <Part of sunflower plant in which the peduncle is upright>

In another aspect, the present disclosure provides a part of a sunflower plant in which the peduncle is upright. The present disclosure is a part of the sunflower plant in which the peduncle is upright of the present disclosure and/or the sunflower plant comprising a mutant of the *Ha412HOChr04g0188691* gene.

The plant part includes, for example, a plant cell, a plant protoplast, a plant cell culture or a tissue culture capable of regenerating a plant body, a plant callus, plant clumps, a plant cell isolated from a plant or a plant part, a leaf, pollen, an embryo, a cotyledon, a hypocotyl, a root, a root tip (root end), an anther, a pistil, a flower, an ovary, an ovule, a seed, a fruit, a stem, a seedling, and the like. The part of the plant individual includes, for example, an organ, a tissue, a cell, or a vegetative propagule, and any of these may be used. The organ includes, for example, a petal, a corolla, a flower, a leaf, a seed, a fruit, a stem, a root, and the like. The tissue is, for example, a part of the organ. As a specific example, the part of the plant individual includes a microspore, a flower, a flower bud, a pistil, an anther, pollen, an ovary, an embryo, an ovule, a hypocotyl, an embryo sac, an egg cell, a cutting, a root, a root tip, a trunk, a stem, a leaf, a petiole, a leaf pith, a cotyledon, a cell, a meristematic cell, a protoplast, a seed, and the like. The pollen may be mature pollen or immature pollen. The part of the plant individual may be derived from, for example, any growth stage of a plant, and examples thereof include those derived from before rooting, after rooting, a seedling, a cutting, a mature individual, and the like. The part of the plant individual may be, for example, one type of organ, tissue and/or cell, or may be two or more types of organs, tissues and/or cells.

The plant part of the present disclosure can be produced, for example, by obtaining a target site in the sunflower plant of the present disclosure.

### <Method for producing sunflower plant or plant population of sunflower plants>

In another aspect, the present disclosure provides a method for producing a sunflower plant or a plant population of sunflower plants showing an upright trait. The method for producing a sunflower plant or a plant population of sunflower plants of the present disclosure comprises following steps (a) and (b). The method for producing the present disclosure may comprise only following step (a).
(a) a step of crossing the sunflower plant in which the peduncle is upright of the present disclosure with another sunflower plant;
(b) a step of selecting a sunflower plant showing the upright trait from the sunflower plant or a progeny line thereof obtained by the step (a).

According to the method for the producing of the present disclosure, by crossing the sunflower plant of the present disclosure with another sunflower plant, a sunflower plant or a plant population of sunflower plants showing the upright trait can be obtained from the obtained progeny line. According to the method for the producing of the present disclosure, a variety of sunflower plants showing the upright trait can be bred using an arbitrary sunflower plant. Thus, the method for the producing of the present disclosure can also be called, for example, a breeding method or a growing method.

In the step (a), the sunflower plant showing the upright trait used as a first parent only needs to be the sunflower plant of the present disclosure. The sunflower plant of the present disclosure is preferably, for example, the sunflower plant deposited under the above-described Accession No. FERM BP-22482 or a progeny line thereof. In step (a), the sunflower plant in which the peduncle is upright and used as the first parent can also be obtained, for example, by a screening method of the present disclosure described below. Thus, the sunflower plant showing the upright trait may be prepared, for example, by selecting from a test sunflower plant prior to step (a), for example, by step (x) below.
(x) a step of selecting the sunflower plant showing the upright trait of the present disclosure from a test sunflower plant

In the step (x), the selection of the sunflower plant showing the upright trait can be implemented by, for example, directly and/or indirectly evaluating the upright trait of a test sunflower plant and selecting a sunflower plant showing the upright trait. For the direct evaluation, the description of the cultivation test of the upright trait above-described can be applied.

When evaluation is implemented by the indirect evaluation, in the step (x), for example, a sunflower plant showing the upright trait may be selected using the presence or absence of a mutant of the *Ha412HOChr04g0188691* gene as an indicator. For the description of the mutant of the *Ha412HOChr04g0188691* gene, for example, the description of the sunflower plant of the present disclosure can be applied. Specifically, in the step (x), the selection of the sunflower plant showing the upright trait can be said to be the selection of a sunflower plant having the mutant of the *Ha412HOChr04g0188691* gene. The mutation of the *Ha412HOChr04g0188691* gene may be implemented, for example, by decoding a base sequence of the *Ha412HOChr04g0188691* gene of the test sunflower plant and comparing the decoded base sequence with a base sequence of the wild-type *Ha412HOChr04g0188691* gene. And, for example, when a mutation is introduced into the base sequence of *Ha412HOChr04g0188691* gene of the test sunflower plant relative to the base sequence of the wild-type *Ha412HOChr04g0188691* gene, the test sunflower plant is selected as a sunflower plant showing the upright trait of the present disclosure. The comparison of base sequences can be implemented, for example, by known base sequence analysis software. Further, when the mutation of the *Ha412HOChr04g0188691* gene is caused by introducing a mutation such as insertion, deletion, and/or substitution of at least one base relative to the base sequence of the wild-type *Ha412HOChr04g0188691* gene, the evaluation may be implemented using, for example, a primer set, a probe, or a combination thereof capable of detecting at least one mutation. The primer set and probe can be designed, for example, using a known design method based on the type of the mutation. Further, the mutation of the *Ha412HOChr04g0188691* gene may be implemented, for example, by judging based on a function of mRNA of the *Ha412HOChr04g0188691* gene or a protein encoded by the *Ha412HOChr04g0188691* gene of the test sunflower plant. Furthermore, the mutation of the *Ha412HOChr04g0188691* gene may be implemented, for example, by judging based on the presence or absence of expression of a protein encoded by the *Ha412HOChr04g0188691* gene in the test sunflower plant, an expression level of the protein encoded by the *Ha412HOChr04g0188691* gene in the test sunflower plant, or a molecular weight of the protein encoded by the *Ha412HOChr04g0188691* gene in the test sunflower plant.

When judging is performed based on the molecular weight of the protein encoded by the *Ha412HOChr04g0188691* gene in the test sunflower plant, the method for the producing of the present disclosure comprises, for example, a measurement step of measuring a molecular weight of a protein encoded by the *Ha412HOChr04g0188691* gene in a plant sample of the test sunflower plant, and by comparing the molecular weight of the protein encoded by the *Ha412HOChr04g0188691* gene in the plant sample of the test sunflower plant with a molecular weight of a protein encoded by a wild-type *Ha412HOChr04g0188691* gene, a test sunflower plant in which the *Ha412HOChr04g0188691* gene is mutated and can be selected. The molecular weight of the protein encoded by the *Ha412HOChr04g0188691* gene measured in the measurement step can be determined, for example, by a known method for quantifying a molecular weight of a protein such as Western blotting.

Further, when evaluation is implemented by the indirect evaluation, in the step (x), for example, a sunflower plant showing the upright trait may be selected using the presence or absence of the upright locus of the peduncle on the fourth chromosome as an indicator. Specifically, in the step (x), the selection of the sunflower plant showing the upright trait can be said to be selection of a sunflower plant having the upright locus of the peduncle. Thus, the step (x) can be performed, for example, by following steps (x1) and (x2).
(x1) a detection step of detecting presence or absence of the upright locus of the peduncle on a chromosome of the test sunflower plant
(x2) a selection step of selecting the test sunflower plant as a sunflower plant showing the upright trait, based on the presence of the upright locus of the peduncle

The selection in the step (x) is, as described above, for example, the selection of a sunflower plant having the upright locus of the peduncle on the fourth chromosome, and specifically, by investigating the upright locus of the peduncle for the test sunflower plant, a sunflower plant showing the upright trait can be selected.

The detection of the upright locus of the peduncle in the step (x1) can be implemented, for example, as explained for the sunflower plant of the present disclosure, by using (1) the molecular marker, (2) a base sequence comprising the molecular marker, (3) a region between the sites of the two molecular markers, and combinations thereof, which specify the upright locus of the peduncle. Specifically, in the step (x1), for example, a molecular marker such as an SNP and/or an Indel associated with the upright locus of the peduncle on a chromosome of the test sunflower plant is detected, and the presence or absence of the upright locus of the peduncle is detected from the obtained detection result.

In the step (x2), for example, when the upright locus of the peduncle exists in one chromosome in a pair of chromosomes, the test sunflower plant may be selected as the sunflower plant showing the upright trait, or when the upright locus of the peduncle exists in both chromosomes in a pair of chromosomes, the test sunflower plant may be selected as the sunflower plant showing the upright trait, but the latter is preferred.

### (1) Identification by molecular marker

The selection in the step (x) is, for example, the selection of a sunflower plant showing the upright trait having an upright locus of the peduncle identified by at least one molecular marker selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314. The selected molecular marker is not particularly limited, and, for example, the description of "(1) Identification by molecular marker" in the sunflower plant of the present disclosure can be applied.

### (2) Identification by base sequence comprising molecular marker

The selection in the step (x) is, for example, the selection of a sunflower plant showing the upright trait having an upright locus of the peduncle identified by at least one polynucleotide selected from the group comprising of the (a), (b), (c), (d), (e), (f), (g), and (ga). For the polynucleotides (a), (b), (c), (d), (e), (f), (g), and (ga), the description of "(2) Identification by base sequence comprising molecular marker" in the sunflower plant of the present disclosure can be applied, for example.

### (3) Identification by region between the sites of two molecular markers

The selection in the step (x) is, for example, the selection of a sunflower plant showing the upright trait having an upright locus of the peduncle comprising a base sequence of a region between the sites of two molecular markers selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 on the fourth chromosome. For the base sequence of the region between sites of the two molecular markers, the description of "(3) Identification by region between sites of two molecular markers" in the sunflower plant of the present disclosure can be applied, for example.

As a specific example, the selection in the step (x) includes, for example, the selection of a sunflower plant showing the upright trait having an upright locus of the peduncle located between sites of two molecular markers selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 in the chromosome.

When the detection is performed by the presence or absence of a base sequence of a region between sites of the two molecular markers, in the step (x1), for example, a base sequence of a genome (genomic DNA) of the test sunflower plant is decoded. Next, in the step (x1), the detection can be performed by comparing the obtained base sequence with a base sequence of a genome (genomic DNA) of the deposited line to confirm whether or not the base sequences match. The decoding of the base sequence can be implemented using, for example, a sample comprising a genome (genomic DNA) of a test sunflower plant, a sequencing reagent, and a sequencer. Further, the comparison of base sequences can be implemented by, for example, a base sequence analysis software (for example, the above-described BLAST or the like). In the obtained base sequence, the region in which the base sequences are compared is a region between the sites of the two molecular markers. As a specific example, the decoding in the step (x1) can be determined, for example, with reference to Reference Document 6 below, by using a sequencer and implementing scaffolding. Thereby, in the step (x1), a sunflower plant having a base sequence that matches the base sequence of the region between the sites of the two molecular markers in the deposited line, or that satisfies the identity above-described, can be identified (recognized or detected) as the test sunflower plant comprising the upright locus of the peduncle. In the step (x1), the detection may be implemented for the whole genome of the test sunflower plant, or may be implemented for a part of the genome. Further, the detection may be implemented for all chromosomes, or may be implemented for some chromosomes. Then, in the step (x2), for example, the test sunflower plant comprising the upright locus of the peduncle is selected as the sunflower plant of the present disclosure or a progeny line thereof, that is, the sunflower plant showing the upright trait.

When the upright locus of the peduncle in a genomic region between the sites of the two molecular markers is detected, in the step (x1), the upright locus of the peduncle may be detected by QTL analysis. According to the QTL analysis, a chromosomal region contributing to the upright position of the peduncle in the test sunflower plant can be identified. For a method of QTL analysis in the sunflower plant, for example, the Reference Document 1 above-described can be referred to. Then, in the step (x2), for example, a test sunflower plant comprising the upright locus of the peduncle in a region between the sites of two molecular markers in the chromosome is selected as the sunflower plant of the present disclosure or a progeny line thereof, that is, the sunflower plant showing the upright trait.

The chromosome in which the presence or absence of the upright locus of the peduncle is detected is preferably the fourth chromosome.

In the step (a), the sunflower plant used as the other parent is not particularly limited and may be, for example, a sunflower plant showing a known upright trait or not showing an upright trait, or may be the sunflower plant showing the upright trait of the present disclosure above-described.

In the step (a), the method for crossing the sunflower plant showing the upright trait and the other sunflower plant is not particularly limited, and a known method can be adopted.

In the step (b), a target for selecting a sunflower plant showing the upright trait may be, for example, the sunflower plant obtained in the step (a), or may be a progeny line obtained from the sunflower plant. Specifically, the target may be, for example, an F1 sunflower plant obtained by crossing in the step (a), or may be a progeny line thereof. The progeny line may be, for example, a self-pollinated progeny or a backcrossed progeny of an F1 sunflower plant obtained by crossing in the step (a), or may be a sunflower plant obtained by crossing the F1 sunflower plant with another sunflower plant.

In the step (b), selection of a sunflower plant showing the upright trait can be performed by, for example, directly or indirectly confirming the upright trait.

In the step (b), the direct confirmation can be performed by evaluating, for example, the direction of the peduncle of the obtained F1 sunflower plant or progeny line thereof by the above-described cultivation test of the upright trait. Specifically, for example, for the F1 sunflower plant or progeny line thereof, when a peduncle inclination angle of the sunflower plant is at most 45°, at most 40°, at most 35°, at most 30°, at most 25°, at most 20°, at most 15°, or at most 10°, the direction of the peduncle of the target sunflower plant can be evaluated as upright. In this case, for example, the F1 sunflower plant or progeny line thereof satisfying the prescribed criteria can be selected as a sunflower plant showing the upright trait.

Also, in the step (b), selection by the indirect confirmation can be performed by, for example, following steps (b1) and (b2).

(b1) a detection step of detecting the presence or absence of the upright locus of the peduncle on the fourth chromosome for the sunflower plant or progeny line thereof obtained in the step (a).

(b2) a selection step of selecting the sunflower plant or progeny line thereof obtained in the step (a) as a sunflower plant showing the upright trait, based on the presence of the upright locus of the peduncle.

Selection by the indirect evaluation of a sunflower plant showing the upright trait in the step (b) is, for example, similar to the method explained in the step (x), and can be performed by detecting the presence or absence of the upright locus of the peduncle.

In the method for the producing of the present disclosure, it is preferable to further grow the sunflower plant showing the upright trait selected in the step (b).

In this way, the sunflower plant or progeny line thereof in which the upright trait has been confirmed can be selected as a sunflower plant showing the upright trait.

The method for the producing of the present disclosure may further comprise a seed collection step of collecting seeds from the progeny line obtained by crossing.

The production method of the present disclosure comprises the step (a) and the step (b), but the present disclosure is not limited thereto and may comprise only the step (a). In this case, the sunflower plant of the present disclosure used in the step (a) preferably comprises the upright locus of the peduncle in a homozygous form.

### <Method for screening sunflower plant having upright trait>

In another aspect, the present disclosure provides a method for screening a sunflower plant showing an upright trait. The screening method of the present disclosure is a method for screening a sunflower plant showing an upright trait, the method comprising a selection step of selecting, from a test sunflower plant, a test sunflower plant in which the *Ha412HOChr04g0188691* gene is mutated, as a sunflower plant showing the upright trait. Further, the screening method of the present disclosure is a method for screening a sunflower plant showing an upright trait, the method comprising a selection step of selecting, from a test sunflower plant, a sunflower plant comprising an upright locus of the peduncle on chromosome 4, as a sunflower plant showing the upright trait. According to the screening method of the present disclosure, it is possible to screen a sunflower plant that has a possibility of showing the upright trait. Further, according to the screening method of the present disclosure, it is possible to screen a parent for producing a sunflower plant showing the upright trait by crossing.

For the selection of the sunflower plant showing the upright trait, for example, the description of the indirect selection (selection using indirect evaluation) in the step (x) or the step (x1) in the method for producing a sunflower plant showing an upright trait of the above-described present disclosure can be applied.

### <Method for detecting upright trait of peduncle of sunflower plant>

In another aspect, the present disclosure provides a method for detecting an upright trait in a sunflower plant. The detection method of the present disclosure is a method for detecting an upright trait of a peduncle of a sunflower plant, the method comprising a detection step of detecting whether the *Ha412HOChr04g0188691* gene is mutated in a test sunflower plant. Further, the detection method of the present disclosure is a method for detecting an upright trait of a peduncle of a sunflower plant, the method comprising a detection step of detecting an upright locus of the peduncle on chromosome 4 of a test sunflower plant. According to the detection method of the present disclosure, it is possible to detect whether a target sunflower plant shows the upright trait. The method for detecting an upright trait of a peduncle of a sunflower plant of the present disclosure can also be called, for example, a method for screening an upright trait in a sunflower plant.

For the detection of the sunflower plant showing the upright trait, for example, the description of the indirect selection in the step (x), i.e., the step (x1), in the method for producing a sunflower plant showing an upright trait of the above-described present disclosure can be applied.

### <Method for imparting upright trait to sunflower plant>

In another aspect, the present disclosure provides a method for imparting an upright trait to a sunflower plant. The method for imparting an upright trait to a sunflower plant of the present disclosure comprises an upright step of making a peduncle of a sunflower plant upright by changing a function of the *Ha412HOChr04g0188691* gene of the sunflower plant. Further, the method for imparting an upright trait to a sunflower plant of the present disclosure comprises an introduction step of introducing an upright locus of the peduncle on chromosome 4 into a sunflower plant. According to the imparting method of the present disclosure, it is possible to impart the upright trait to a sunflower plant by changing the function of the *Ha412HOChr04g0188691* gene and/or introducing the upright locus of the peduncle on chromosome 4.

The *Ha412HOChr04g0188691* gene of the sunflower plant is estimated to express the upright trait, for example, by inhibition of its gene function. Thus, the upright step may be a suppression step of suppressing gene expression of the *Ha412HOChr04g0188691* gene of the sunflower plant, or may be a mutation step of mutating the *Ha412HOChr04g0188691* gene of the sunflower plant.

The inhibition of the gene function may be implemented, for example, by applying genome editing technology to the *Ha412HOChr04g0188691* gene. Further, the inhibition of the gene function may be implemented, for example, by introducing an expression-suppressing nucleic acid molecule against mRNA encoded by the *Ha412HOChr04g0188691* gene. Examples of the expression-suppressing nucleic acid molecule include siRNA, antisense, and the like. The siRNA and antisense can be designed, for example, based on the *Ha412HOChr04g0188691* gene.

In the mutation step, the method for introducing a mutation into the *Ha412HOChr04g0188691* gene is not particularly limited. The introduction method can be implemented, for example, by introducing a mutation such as deletion, substitution, insertion and/or addition of at least one base to a base sequence of the wild-type *Ha412HOChr04g0188691* gene. In the introduction step, examples of the mutation include a partial deletion mutation and a complete deletion mutation of the *Ha412HOChr04g0188691* gene. For the position of the mutation and the type of the mutation of the *Ha412HOChr04g0188691* gene, the description of the position of the mutation, the type of the mutation, and the like in the sunflower plant comprising a mutant of the *Ha412HOChr04g0188691* gene of the above-described present disclosure can be applied.

In the introduction step, a method for introducing the upright locus of the peduncle on chromosome 4 is not particularly limited. Examples of the introduction method include crossing with a sunflower plant showing the upright trait, tissue culture such as embryo culture, and conventionally known genetic engineering techniques. Examples of the upright locus of the peduncle to be introduced include the above-described upright locus of the peduncle. In a case where introduction is implemented by crossing with the sunflower plant showing the upright trait, the sunflower plant showing the upright trait preferably comprises, for example, the upright locus of the peduncle in a homozygous form.

### <Upright trait marker of peduncle of sunflower plant>

In a certain aspect, the present disclosure provides a marker that can be used as an indicator of an upright trait in a sunflower plant. The marker of the present disclosure is an upright trait marker of a peduncle of a sunflower plant, and comprises an upright locus of the peduncle on chromosome 4. According to the marker of the present disclosure, it is possible to detect the presence or absence of an upright peduncle.

### <Nucleic acid>

In a certain aspect, the present disclosure provides a nucleic acid that can be used as an indicator of an upright trait in a sunflower plant. An isolated nucleic acid having a function capable of determining the upright trait of the peduncle of the sunflower plant comprises a contiguous base sequence of at least 15 bases in a base sequence selected from the group comprising of the following (a) to (g) and (ga) in a chromosome of a sunflower plant. The nucleic acid of the present disclosure can be suitably used for detection of the presence or absence of an upright peduncle of the sunflower plant.
(a) a base sequence of the following (a1), (a2), or (a3):
   (a1) the base sequence of SEQ ID NO: 1;
   (a2) a base sequence in which one or several bases are deleted, substituted, inserted and/or added in the base sequence of the (a1) with the 101st base (A) of the (a1) being conserved;
   (a3) a base sequence having an identity of at least 80% to the base sequence of the (a1) with the 101st base (A) of the (a1) being conserved;
(b) a base sequence of the following (b1), (b2), or (b3):
   (b1) the base sequence of SEQ ID NO: 2;
   (b2) a base sequence in which one or several bases are deleted, substituted, inserted and/or added in the base sequence of the (b1) with the 202nd to 213th bases (TAAAAAAAAAAA) of the (b1) being conserved;
   (b3) a base sequence having an identity of at least 80% to the base sequence of the (b1) with the 202nd to 213th bases (A) of the (b1) being conserved;
(c) a base sequence of the following (c1), (c2), or (c3):
   (c1) the base sequence of SEQ ID NO: 3;
   (c2) a base sequence in which one or several bases are deleted, substituted, inserted and/or added in the base sequence of the (c1) with the 101st base (T) of the (c1) being conserved;
   (c3) a base sequence having an identity of at least 80% to the base sequence of the (cl) with the 101st base (T) of the (c1) being conserved;
(d) a base sequence of the following (d1), (d2), or (d3):
   (d1) the base sequence of SEQ ID NO: 4;
   (d2) a base sequence in which one or several bases are deleted, substituted, inserted and/or added in the base sequence of the (d1) with the 101st base (C) of the (d1) being conserved;
   (d3) a base sequence having an identity of at least 80% to the base sequence of the (d1) with the 101st base (C) of the (d1) being conserved;
(e) a base sequence of the following (e1), (e2), or (e3):
   (e1) the base sequence of SEQ ID NO: 5;
   (e2) a base sequence in which one or several bases are deleted, substituted, inserted and/or added in the base sequence of the (e1) with the 101st base (G) of the (e1) being conserved;
   (e3) a base sequence having an identity of at least 80% to the base sequence of the (el) with the 101st base (G) of the (e1) being conserved;
(f) a base sequence of the following (f1), (f2), or (f3):
   (f1) the base sequence of SEQ ID NO: 6;
   (f2) a base sequence in which one or several bases are deleted, substituted, inserted and/or added in the base sequence of the (f1) with the 101st base (A) of the (f1) being conserved;
   (f3) a base sequence having an identity of at least 80% to the base sequence of the (f1) with the 101st base (A) of the (f1) being conserved;
(g) a base sequence of the following (g1), (g2), or (g3):
   (g1) a base sequence having a base sequence of 1 to 855 bases between the base sequence of SEQ ID NO: 14 and the base sequence of SEQ ID NO: 15;
   (g2) a base sequence in which one or several bases are deleted, substituted, inserted and/or added in the base sequence of the (g1) with the base sequence of the polynucleotide of 1 to 855 bases in the (g1) being conserved;
   (g3) a base sequence having an identity of 80% or more to the base sequence of the (g1) with the base sequence of the polynucleotide of 1 to 855 bases in the (g1) being conserved;
(ga) a base sequence of the following (gal), (ga2), or (ga3):
   (gal) the base sequence of SEQ ID NO: 13;
   (ga2) a base sequence in which 1 to 171 bases are deleted, substituted, inserted and/or added in the base sequence of the (gal);
   (ga3) a base sequence having an identity of at least 80% to the base sequence of the (gal).

For the description of the base sequences of the (a) to (g) and (ga), for example, the description of the polynucleotides of the (a) to (g) and (ga) can be applied respectively.

The nucleic acid is, for example, a nucleic acid having a contiguous base sequence of at least 15 bases, at least 30 bases, at least 50 bases, at least 100 bases, or at least 200 bases in a base sequence selected from the group comprising of the (a) to (g) and (ga).

The nucleic acid preferably comprises a part or all of the conserved base or base sequence in each polynucleotide.

Examples of the length of the nucleic acid include 15 to 10,000 bases, 30 to 10,000 bases, 50 to 5,000 bases, or 70 to 2,000 bases in length.

### <Harvested products and processed products of sunflower plant>

In another aspect, the present disclosure provides a harvested product and/or a processed product of a sunflower plant showing the upright trait. The harvested product is the entire plant or a part of a plant individual, and preferably comprises a flower, a flower and a stem (peduncle), or a seed.

In a case where the harvested product is a flower, the harvested product may comprise a stem directly below the flower in addition to the flower. The length of the harvested stem is, for example, about 40 to 100 cm, or about 60 to 80 cm. In a case where the harvested product is a flower and stem, the flower and stem may be a bundle of a plurality of the flowers and the stems.

The processed product includes any product obtained by processing the sunflower plant, the deposited line, or a progeny line. The processing is not particularly limited, and examples thereof include cutting, slicing, grinding, pureeing, drying, canning, bottling, washing, packaging, freezing and/or heat treatment, and the like. In the deposited line or the progeny line, the plant or part of the plant individual used for the processed product is, for example, a flower, a flower and a stem, or a seed. The processed product may be, for example, one in which the deposited line or the progeny line is washed and packaged. The processed product may be stored in a container of any size or shape, for example. Specific examples of the container include a bag, a box, a carton, and the like.

The present disclosure may provide a container comprising one or a plurality of sunflower plants. The container comprises the entire plant or a part of a plant individual, preferably a flower, or a flower and a stem.

The present disclosure may provide a method for producing a sunflower plant as a cut flower (a method for producing a cut flower). The method for producing a cut flower of the present disclosure comprises, for example, a step of collecting or harvesting the entire plant or a part of a plant individual of the sunflower plant showing the upright trait of the present disclosure, preferably a flower, or a flower and a stem of the sunflower plant showing the upright trait of the present disclosure.

### EXAMPLES

Hereinafter, the present disclosure will be explained in detail using examples, but the present disclosure is not limited to the aspects described in the examples.

### [Example 1]

Regarding a sunflower plant showing a novel upright trait of a peduncle, it was confirmed that it shows the upright trait. Further, the identification of a novel upright locus of the peduncle in the sunflower plant having the upright trait was performed.

### (1) Breeding of deposited line

In 2005, a sunflower line (manufactured by Takii & Co., Ltd.) having an upright trait was selected from various sunflower lines held by Takii & Co., Ltd. In 2006, a sunflower line having the upright trait selected was crossed with a plurality of sunflower lines (manufactured by Takii & Co., Ltd.), and it was confirmed that its progeny lines have uprightness of the peduncle. As the plurality of sunflower lines, the sunflower lines that do not have the upright trait and have various flower colors were used. After 2007, for each of the progeny lines obtained by crossing with the plurality of sunflower lines, mating and selection were repeatedly implemented. The selection was implemented based on the upright trait, earliness, and the like. Then, in 2012, the progeny lines were judged to be fixed lines in which each target trait was fixed, and the breeding was terminated.

In 2023, regarding the fixed lines, it was confirmed at the Headquarters Production Department of Takii & Co., Ltd. that there was no variation in characteristics after sowing. Thereby, it was confirmed that the fixed lines have uniformity and stability. Then, seeds obtained by population seed production of the fixed lines were deposited at NITE-IPOD under the Accession No. FERM BP-22482.

### (2) Traits of deposited line

Characteristics and traits of plant individuals of the deposited line were evaluated according to the examination guidelines for sunflower varieties issued by MAFF. Specifically, the seeds of the deposited line after passage of at least 3 months from seed collection were sown on April 28, 2019, at a test site (Konan City, Shiga Prefecture), and cultivated by unheated greenhouse rain-shelter cultivation. In the cultivation, 30 g/m² of fertilizer IB Kasei No. 1 (Zen-Noh 10-10-10) and 4 g/m² of trace element fertilizer F • T • E (manganese 19%, boron 9%, manufactured by TOMATEC) were mixed, ridges with a bottom surface of 80 cm were established in the north-south direction, and 4-row cultivation was performed with a plant spacing of 20 cm x 20 cm. In the sowing, seeds of the deposited line were sown by direct sowing to a depth of about 2 cm, and after the sowing, soil of the field was used for soil covering. After the sowing and soil covering, sufficient irrigation was performed. From 2 weeks after germination, irrigation was performed only when wilting was observed in leaves. In the cultivation, flower nets of 20 cm x 20 cm mesh were installed according to the plant height so that the sunflowers would not fall over. The installation was performed after the sowing and soil covering. Since disk florets began to bloom from June 20, 2019, the direction of the flower head of each deposited line was evaluated at the flowering stage F3.2 (stage F in FIG. 3). In order to eliminate differences in moisture conditions and sunlight conditions, the evaluation was performed only for sunflowers in the central part of the ridges without evaluating sunflowers in the 3 rows at the ends of the ridges. The evaluation was performed based on the evaluation criteria for Characteristic Numbers 20, 21, 24, and 33. The results are shown in Table 2 below. Note that for Head: attitude of Characteristic Number 33, FIG. 4 can be referred to. Sunflower plants for comparison were cultivated and evaluated by the same method as the cultivation method and evaluation method for the plant individuals of each deposited line.

**[Table 2]**

| Characteristics Number | Characteristics | Characteristic | Sunrich Orange | Vincent Orange | Deposit Line |
|---|---|---|---|---|---|
| 20 | Ray floret: color | 1. yellowish white | 5 | 5 | 5 |
| | | 2. light yellow | | | |
| | | 3. medium yellow | | | |
| | | 4. orange yellow | | | |
| | | 5. orange | | | |
| | | 6. purple | | | |
| | | 7. reddish brown | | | |
| | | 8. multicolored | | | |
| 21 | Disk flower: color | 1. yellow | 3 | 3 | 3 |
| | | 2. orange | | | |
| | | 3. purple | | | |
| 24 | Disk flower: production of | 1. absent | 1 | 1 | 9 |
| | pollen | 9. present | | | |
| 33 | Head: attitude | 1. horizontal | 3 | 2-3 | 1 |
| | | 2. inclined | | | |
| | | 3. vertical | | | |
| | | 4. half-turned down with straight stem | | | |
| | | 5. half-turned down | | | |
| | | with curved stem | | | |
| | | 6. turned down with straight stem | | | |
| | | 7. turned down with slightly curved stem | | | |
| | | 8. turned down with strongly curved stem | | | |
| | | 9. over turned | | | |

### (3) Peduncle inclination angle of deposited line

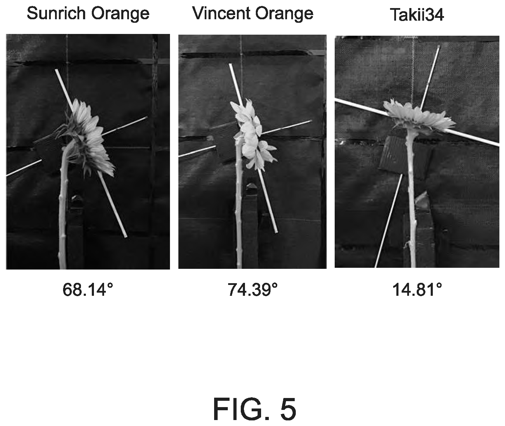

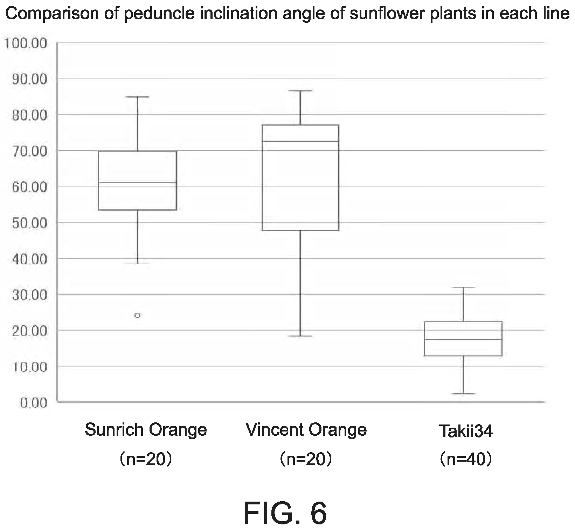

The peduncle inclination angle of the plant individuals of the deposited line was examined. Specifically, peduncle inclination angles (D) of plant individuals (40 individuals) of the deposited line and sunflower plants for comparison (20 individuals) obtained by the same cultivation method as in Example 1 (2) were measured. The measurement of the peduncle inclination angle was performed based on the cultivation test conditions and measurement conditions in the cultivation test of the upright trait described above. At the flowering stage F3.2, the plant individuals of the deposited line and the sunflower plants for comparison were photographed from directly beside each other, and the angle was measured using the Angle function of Image J. In order to eliminate differences in moisture conditions and sunlight conditions, the measurement was performed only for sunflowers in the central part of the ridges without evaluating sunflowers in the 3 rows at the ends of the ridges. These results are shown in Table 3 below, and FIG. 5 and FIG. 6.

FIG. 5 is a photograph comparing the peduncle inclination angles of each sunflower plant. As shown in FIG. 5, the peduncle inclination angles of Takii34, Sunrich Orange (Takii & Co., Ltd.), and Vincent Orange (Sakata Seed Corporation) were 14.81°, 68.14°, and 74.39°, respectively. From these results, in Takii34, the peduncle orientation angle was at most 45° and the peduncle orientation was upright, whereas in Sunrich Orange and Vincent Orange, the peduncle orientation angle exceeded 45° and the peduncle orientation was not upright.

**[Table 3]**

| | Sunrich Orange | Vincent Orange | Takii34 |
|---|---|---|---|
| Mean | 61.09° | 63.75° | 17.77° |
| Median | 61.10° | 72.49° | 17.50° |
| Number of individuals in the data | 20 | 20 | 40 |

FIG. 6 and Table 3 are a figure and a table comparing the peduncle inclination angles of each sunflower plant. In FIG. 6, the vertical axis indicates the peduncle inclination angle, and the horizontal axis indicates the line of the sunflower plant. As shown in FIG. 6 and Table 3, the peduncle inclination angle (D) of Takii34 showed a mean of about 17.77° and a median of about 17.50°, and it was found that the orientation of the peduncle is upright as compared with the sunflower plants for comparison (Sunrich Orange and Vincent Orange). Note that in a plant population comprising each sunflower plant, the ratio of individuals in which the peduncle angle is 0 to 45° was 15% for Sunrich Orange, 10% for Vincent Orange, and 100% for Takii34.

### (4) Identification of upright locus of peduncle

The deposited line (hereinafter, also called "Takii34") showing the upright trait and a sunflower plant "VL" (Takii & Co., Ltd.) showing a horizontal trait were crossed to obtain an F1 generation. Next, F2 generation seeds of 103 individuals were obtained by selfing the F1 generation.

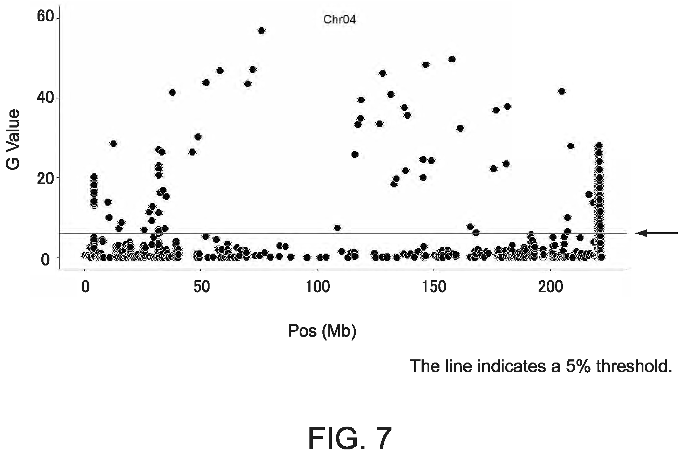

For the 103 individuals, the peduncle inclination angle was measured. Specifically, it was implemented by the same method as in Example 1 (3). For an F2 upright bulk (7 individuals) having a peduncle inclination angle of at most 15°, an F2 horizontal bulk (11 individuals) having a peduncle inclination angle of at least 75°, Takii34, and the F1 of VL, whole genome analysis was performed. Specifically, genomic DNA was extracted using PureGene DNA extraction kit (manufactured by Gentra). The extracted genomic DNA was subjected to whole genome sequencing using MGISEQ (BGI JAPAN). Using fastq files obtained by the whole genome sequencing, cleaning of reads by *Trimmomatic,* mapping to a reference sequence by BWA, and variant calling by bcftools were performed to obtain a vcf (polymorphism information) file. Next, for SNPs/InDels in the vcf file, filtering was performed based on the following criteria. After the filtering, for the vcf file, QTL-seq analysis was performed using the QTLseqr package (Reference Document 7) (Reference Document 8), and the G value was compared between both bulks. These results are shown in FIG. 7 and Table 4.
Reference Document 7: Mansfeld, Ben N, and Rebecca Grumet. "QTLseqr: An R Package for Bulk Segregant Analysis with Next-Generation Sequencing". The plant genome vol. 11,2 (2018): 10.3835/plantgenome2018.01.0006. doi:10.3835/plantgenome2018.01.0006
Reference Document 8: Takagi, Hiroki et al. "QTL-seq: rapid mapping of quantitative trait loci in rice by whole genome resequencing of DNA from two bulked populations". The Plant journal: for cell and molecular biology vol. 74,1 (2013): 174-83. doi:10.1111/tpj.12105

### (Filtering Criteria)

(1) The number of reads (depth) for each of the upright and horizontal bulks, both parent lines, and F1 individuals is at least 10.
(2) From the number of reads, both parent lines are estimated to be homozygous for REF (reference sequence type allele) or ALT (alternative type allele), and there is a polymorphism between both parent lines.
(3) Similarly, from the number of reads, F1 individuals are estimated to be heterozygous.

FIG. 7 is a graph showing G value representing a degree of contribution of each SNP on chromosome 4 to a trait, shown by QTL-seq analysis. In FIG. 7, the vertical axis indicates the G value, and the horizontal axis indicates the physical position (Mb) on chromosome 4. In FIG. 7, each dot indicates an SNP/InDel that showed a polymorphism between both parent lines. In FIG. 7, the horizontal line (arrow) in the graph indicates a 5% threshold (genomic false discovery rate of 0.05). As shown in FIG. 7, over the entire length of chromosome 4, many dots exceeded the 5% threshold, and the presence of an upright locus of the peduncle was confirmed.

**[Table 4]**

| Method | Chromosome | Start (bp) | G value |
|---|---|---|---|
| | | 37,851,026 | 41.37 |
| | | 52,243,009 | 43.86 |
| | | 58,181,846 | 46.84 |
| | | 70,153,412 | 43.53 |
| G | Chr4 | 72,137,195 | 47.13 |
| | | 75,930,135 | 56.87 |
| | | 128,036,911 | 46.13 |
| | | 146,636,140 | 48.38 |
| | | 157,743,960 | 49.71 |
| | | 204,904,126 | 41.76 |

The above-described Table 4 is a table showing top 10 G values on chromosome 4.

In order to obtain SNP information between Takii34 and VL, resequencing was performed for the Takii34 and VL. Based on the SNP information obtained by the resequencing and the upright locus region of the peduncle, 6 SNP markers were constructed. Table 5 below shows the correspondence between the constructed SNP markers and SNP (a), SNP (b), SNP (c), SNP (d), SNP (e), and SNP (f). In Table 5 below, the base [N₁/N₂] shown in parentheses indicates a single nucleotide polymorphism, where N₁ is the polymorphism (base) of VL, i.e., the polymorphism (base) of the horizontal trait, and N₂ is the polymorphism (base) of Takii34, i.e., the polymorphism (base) of the upright trait.

**[Table 5]**

| SNP | SNP marker | SNP sequence and its adjacent sequences (5'→3'), SNP[N1/N2]: N1=polymorphism of the horizontal trait of the peduncle, N2=polymorphism of the upright trait of the peduncle | SEQ ID NO: |
|---|---|---|---|
| SNP(a) | Ha_chr04_172545117 | AGTATAAACACGAATCGGAACCGAAAGTCTTGTAAATCGACTTTTTCGTAGACTATCG ATTCGGATCCGTGCATGCATGTTTGAGATCTGTATTGGAGCC[T/A]ATTTTGACCATTT TTATTTTAGTATAACTTTCTGGAATTTTTGATACTTGAGTCTATGCTTAGCCGATTCATTT GCATGTTTCCGATTTATGCTTAAAG | 7 |
| SNP(b) | Ha_chr04_175698294 | CTACGACGCCCACTTGAATATGTTTTATTATATTCTTCGCAAAACCTATCGACGAACCCA TTCATTTTTCGCCACTTAGATGACACCGAGTTGATATCTCGATACGGGCCTTGGTCCATT AGCTCAAGGAACTTCGCCAAAACCGCATTCCAAAACCCCTCACCCATTTGATTATTAC CTACAAAATATGTTACAAAAAA[TAAAAAAAAAAAA/TAAAAAAAAAAAA]CTAATACTA CAAAATATAAACTTAAAAAAAAAGTTATTGTTACGAACCTTTTATCGGGTGTGTAGACA TGCCTATGTAAGCGTTCGCTAAGGCTTCTTCATCGAGTTGCGTCTACGGTTTCGCCTTT GGTTTGGACGGTTGCTCACCCACCACTTGCTTGCCTTTTCTTCGTTTGCCTTTTTGCGG TT | 8 |
| SNP(c) | Ha_chr04_181000331 | TTTTTGACTAATTAATTAGTCATAAAGACTAGTATCCTCCACACTAACTTATTTTTGCAT ACACACATCAAAAGTTATCTTACAAATTTCGAAATTCAC[C/T]TACACCTTAAAATTTA TCCTACACAACTCGTAATGTATCTTACACAACTCGTAATTTATCATACACTTTAAATTATT TTATTTCATTTTTTTGAAAAAAA | 9 |
| SNP(d) | Ha_chr04_200021302 | CGTTTATATTACAGATTATGCATAAATCGCAAAAATAGCTTTCTGTTGACTTTTTAAGTA CGCGTTTGACTCGACATTTGAACTAGTTAGAGTGGTGATC[A/C]TAGGGAACCCTTTCA GAGGTTTATTACCCACATAAATACCAACACATAACCACTTTTGATTCGACAAGTGACTG | 10 |
| | | AGCCATTAAGGATTTATCTTGAAGTC | |
| SNP(e) | Ha_chr04_204904126 | GACTAATTTCATGCCTGCCTTCAAAATGGMAAAAGAAACTAATTGGAAAWTTTTGTGT TTATATYTTGTAATAATATTTGTGCAAAAAGGTTCATGCCTT[A/G]TAATCTATCTCTTAT ACTAAAGCAAAATAATGTTGACTATTTGACTATGATGTGGCAGTGCTACWTATTCAGAG AATTGKGAATTRGGCGGCATTTGTTT | 11 |
| SNP(f) | Ha_chr04_208654314 | CTCCATGGCGTGTAACAATTTCTCTCATGTAAATTTCAGCCAACTTGCTCGTGTTATCTT TCTTTCAGATAGGTAAGAAGTGCGCAGATTTCGTTAATC[G/A]TCCACTATTACCCAA ATAGTGTCATGGCCTCTTGGTGTTCTAGGAAACTTTGTAATAAAATCCATGGAGATTTG TTCCCATTTCCACTTGGGAATTTCTG | 12 |

Next, DNA was extracted from the F2 sunflower plants, and an SNP analysis was performed using the SNP markers. Further, F3 generation seeds (hereinafter, also called "103 lines") of 103 individuals were obtained by selfing the F2 generation plants. For each line, the upright trait was evaluated by the same method as in Example 1 (3). These results are shown in Table 6 below.

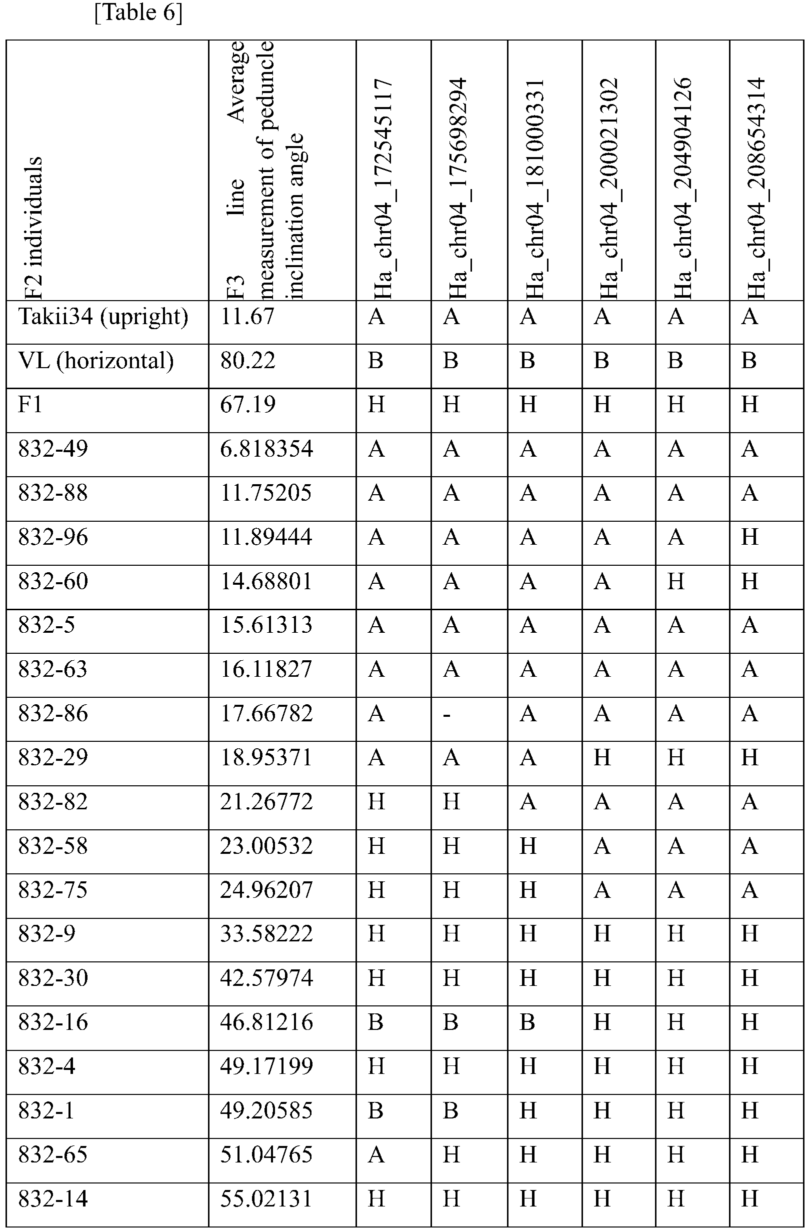

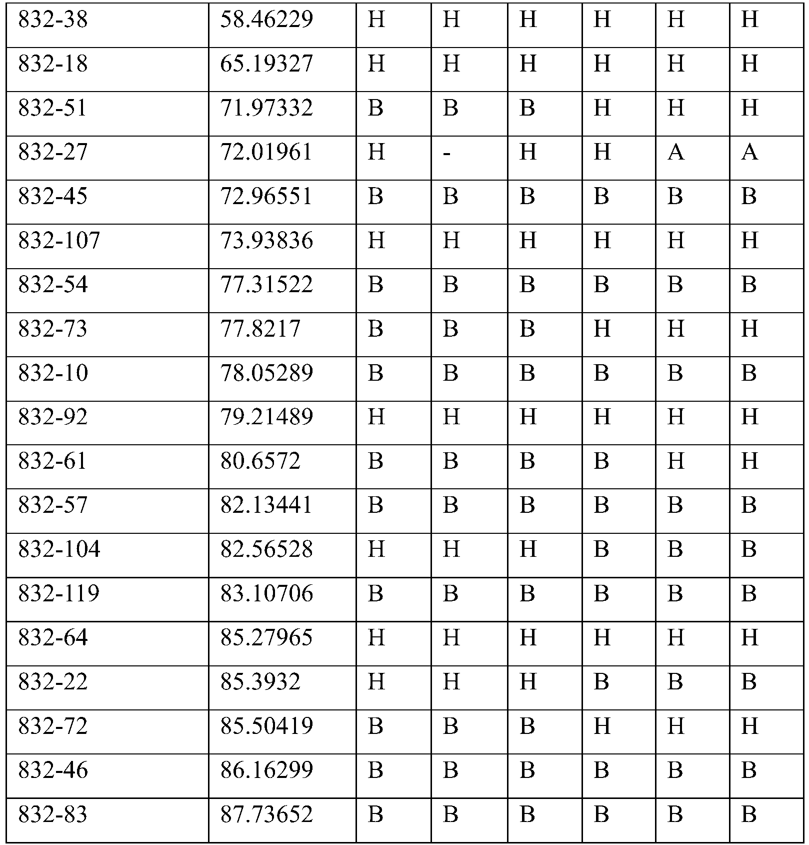

The above-described Table 6 is a table showing the mean value of F3 peduncle inclination and the results of SNP analysis of F2 plants. In Table 6, A indicates having the upright polymorphism in a homozygous form, B indicates having the horizontal (not upright) polymorphism in a homozygous form, and H indicates having each SNP in a heterozygous form. As shown in Table 6, in a case where the SNP marker of "Ha_chr04_181000331" or "Ha_chr04_200021302" was homozygous for upright peduncle property (A), the F3 generation showed the upright trait. From the above, it was found that the upright gene of the peduncle is located between 181,000,331 bp and 200,021,302 bp of chromosome 4. That is, it was found that this region is the located region of the upright locus of the peduncle. Furthermore, in a case where Ha_chr04_172545117 is A, i.e., in a case where the genotype from the upstream SNP is "AAAAAA", "AAAAAH", "AAAAHA", or "AAAHHH", the upright trait is shown, and in a case where Ha_chr04_208654314 is A, i.e., in a case of "AAAAAA", "HHAAAA", or "HHHAAA" from the upstream SNP, the upright trait is also shown, suggesting that the upright gene of the peduncle exists between 172,545,117 bp and 208,654,314 bp of chromosome 4 (roughly between SNP(a) - SNP(f)).

### (5) Analysis of distribution of peduncle inclination angles

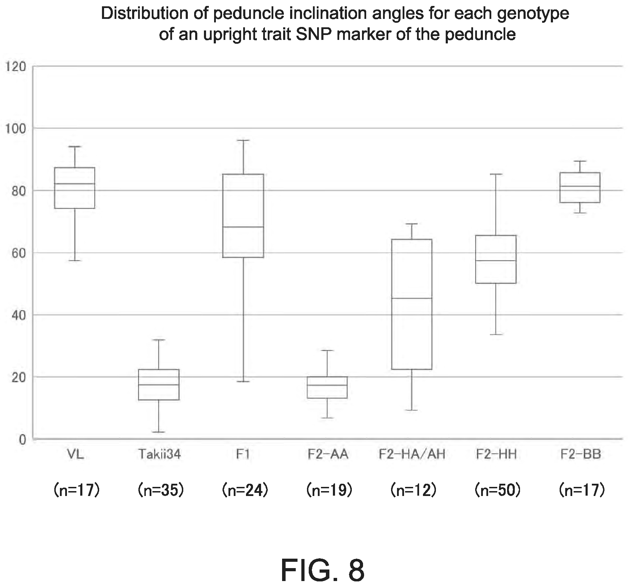

Next, in the F2, classification by the SNP markers "Ha_chr04_181000331" (SNP(c)) and "Ha_chr04_200021302" (SNP(d)) was performed into a group showing Takii34-type homozygous (hereinafter, A or A-type), a group showing VL-type homozygous (hereinafter, B or B-type) showing the horizontal trait, and a group showing heterozygous (hereinafter, H or H-type), and the distribution of peduncle inclination angles was analyzed. These results are shown in FIG. 8.

FIG. 8 is a graph showing the distribution of peduncle inclination angles for each genotype of the upright trait SNP markers. In FIG. 8, the vertical axis indicates the peduncle inclination angle, and the horizontal axis indicates the line of the sunflower plant. As shown in FIG. 8, in a case where both SNP(c) and SNP(d) were A (F2-AA), the peduncle inclination angle (D) showed a mean of about 16.92°, and all individuals showed the upright trait. Further, in a case where SNP(c) and SNP(d) were H and A, or A and H, respectively, the peduncle inclination angle (D) showed a mean of about 41.92°, and the upright trait was shown as a plant population of sunflower plants. From these results, it was found that the region between 181,000,331 bp and 200,021,302 bp of chromosome 4 is the located region of the upright locus of the peduncle. Further, SNP(c) and SNP(d) are each sufficiently linked to the resistance locus, and it was found that the resistance locus located in the located region can be detected using one of the SNPs. Note that in a plant population comprising each sunflower plant, the ratio of individuals in which the peduncle angle is 0 to 45° was 100% for F2-AA, 50% for F2-HA/AH, 12% for F2-HH, and 0% for F2-BB.

### (5) Analysis of SNPs of commercial varieties

It was confirmed that other sunflower plants do not have the upright locus of the peduncle possessed by the sunflower plant showing the novel upright trait.

For each of the sunflower plants in Table 7 below, DNA was extracted, and SNP analysis was performed using SNP markers comprising Ha_chr04_181000331 and Ha_chr04_200021302, which are strongly linked to the upright gene of the peduncle. These results are shown in Table 7 below. In Table 7 below, A indicates having each SNP in a homozygous form for the upright trait, and B indicates having each SNP in a homozygous form for the horizontal trait. Further, as shown in Table 7 below, Takii34 had the homozygous form for the upright trait for all SNP markers of Ha_chr04_172545117, Ha_chr04_175698294, Ha_chr04_181000331, Ha_chr04_200021302, Ha_chr04_204904126, and Ha_chr04_208654314. In contrast, sunflower plants other than Takii34 had the homozygous form for the horizontal trait for all SNP markers of Ha_chr04_172545117, Ha_chr04_175698294, Ha_chr04_181000331, Ha_chr04_200021302, Ha_chr04_204904126, and Ha_chr04_208654314. From these results, it was found that the SNPs are specific to Takii34.

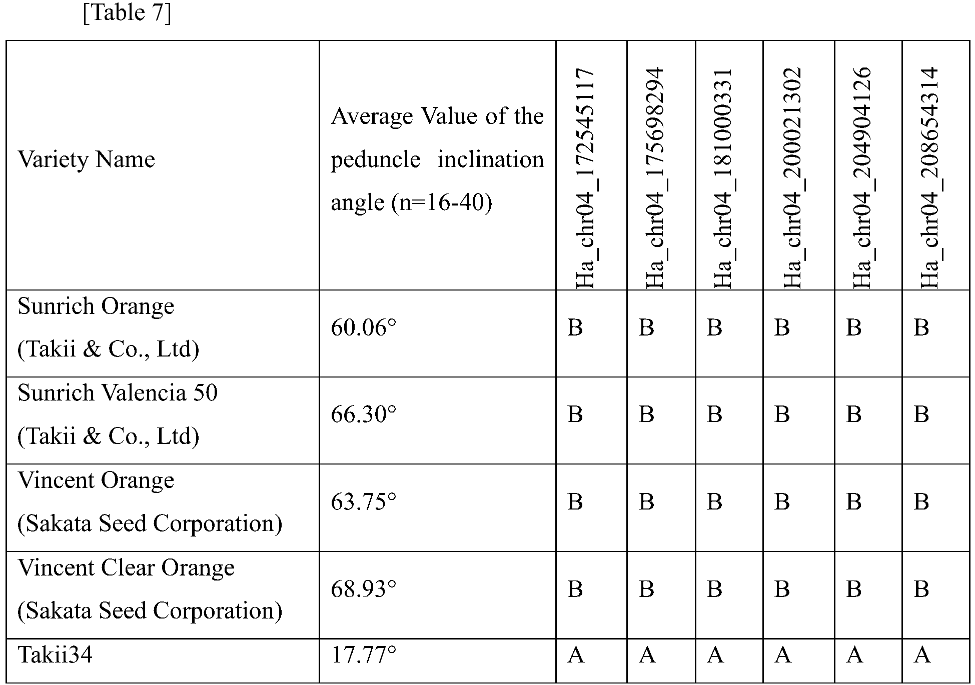

### [Example 2]

Further, the identification of the novel upright locus of the peduncle in the sunflower plant having the upright trait was performed.

### (1) Identification of upright locus of the peduncle

A polymorphism on the upright locus of the peduncle was further identified. Specifically, new leaves were collected from 40 individuals per line of Takii34 and a sunflower plant "VL" showing the horizontal trait of the peduncle. 10 g of the collected new leaves were prepared for each, and DNA was extracted according to a common method. After the extraction, PacBio Revio HiFi read sequencing was implemented. As a result, in Takii34, an insertion sequence specific to uprightness (hereinafter, also referred to as an Indel marker) was found in the *Ha412HOChr04g0188691* gene of the reference sequence HA412-HOv2.0 assembly.

### (2) Analysis of distribution of peduncle inclination angle

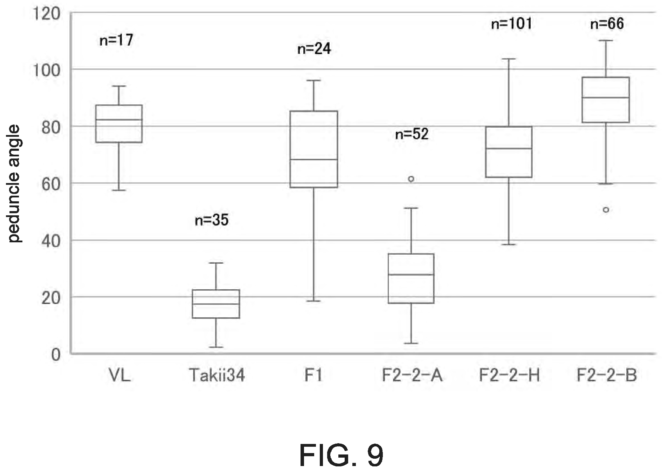

Next, Takii34 and "VL" were crossed to obtain an F1 generation. Next, 219 individuals of an F2 generation were obtained by selfing the F1 generation. For the 219 individuals of the F2 generation, genotypes and phenotypes of each individual were obtained using a primer set (SEQ ID NOs: 24 to 25) for detecting the Indel marker. In the F2 generation, the classification was performed into a group having the Indel marker in a homozygous form (group F2-2-A), a group having the Indel marker in a heterozygous form (group F2-2-H), and a group not having the Indel marker (group F2-2-B), and the distribution of the peduncle inclination angle was analyzed. These results are shown in FIG. 9.

### Primer set for detecting Indel marker

Forward primer (SEQ ID NO: 24)
   5'-AGTATGATCATCCACACAGTCTTC-3'
Reverse primer (SEQ ID NO: 25)
   5'-CACGCGTAAAGAATTATCTATTG-3'

FIG. 9 is a graph showing the distribution of the peduncle inclination angle for each genotype of the upright trait Indel marker of the peduncle. In FIG. 9, the vertical axis indicates the peduncle inclination angle, and the horizontal axis indicates the line of the sunflower plant. As shown in FIG. 9, individuals of the group F2-2-A having the Indel marker in a homozygous form showed a peduncle inclination angle equivalent to that of Takii34. On the other hand, the group F2-2-B not having the Indel marker showed a peduncle inclination angle equivalent to that of VL. From these results, it was found that a sunflower plant having the Indel marker in a homozygous form shows the upright trait.

### (3) Presence or absence of Indel marker in commercial varieties

It was examined whether or not other sunflower plants have the Indel marker possessed by the sunflower plant showing the novel upright trait. Specifically, for each sunflower plant in Table 8 below, DNA was extracted and analysis was performed using molecular markers including Ha_chr04_181000331 and Ha_chr04_200021302, which were strongly linked to the upright gene of the peduncle in Example 1, and the Indel marker. These results are shown in Table 8 below. In Table 8 below, A indicates having each molecular marker in a homozygous form for the upright trait, H indicates having each molecular marker in a heterozygous form for the upright trait, and B indicates having each molecular marker in a homozygous form for the horizontal trait. As shown in Table 8 below, Takii34 had the homozygous form for the upright trait for all molecular markers of Ha_chr04_181000331, Ha_chr04_200021302, and the Indel marker. In contrast, sunflower plants other than Takii34 held the heterozygous form for the upright trait or the homozygous form for the horizontal trait for all molecular markers of Ha_chr04_181000331, Ha_chr04_200021302, and the Indel marker. From these results, it was found that the molecular markers are specific to Takii34.

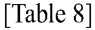

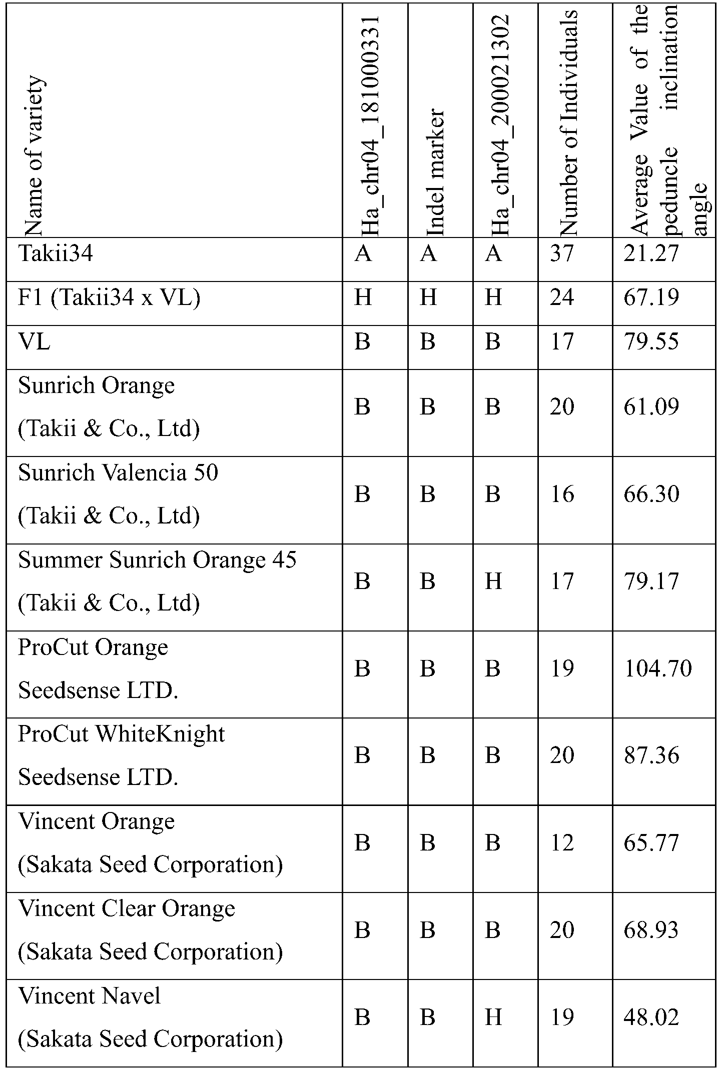

### (4) Identification of gene controlling upright trait

A genomic sequence comprising the *Ha412HOChr04g0188691* gene was examined. Specifically, for Takii34 and some varieties described in Table 8, PCR was performed using a primer set (SEQ ID NOs: 26 to 27) to obtain an amplified fragment of the full length of the *Ha412HOChr04g0188691* gene. The amplified fragment was sequenced using Flongle from Oxford Nanopore Technologies (hereinafter, "ONT"), and the base sequence was determined. The base sequence was compared with a region corresponding to HanXRQChr04:187089837-187088778 of the *HanXRQr2Chr04g0181821* gene corresponding to the *Ha412HOChr04g0188691* gene, and a base sequence of a region 198407926-198409321 of Ha412-HOv2.0. As a result, in Takii34, it became clear that there is an insertion sequence of 855 bases at position 198408790 on chromosome 4 of Ha412-HOv2.0. Further, the insertion position was position 187089296 of chromosome 4 of HanXRQr2, and corresponded to the third exon of the *HanXRQr2Chr04g0181821* gene. Further, as a result of performing a homology search using the amino acid sequence of the *HanXRQr2Chr04g0181821* gene as a query, it was found that there is homology with QIG55756.1 (Tiller Angle Control 1: *Chrysanthemum_x_morifolium*), XP_023746513.1 (TILLER ANGLE CONTROL *1: Lactuca sativa),* XP_043621825.1 (TILLER ANGLE CONTROL *1: Erigeron canadensis),* and AGW17262.1 (TAC1: *Prunus persica*)*.* Note that the *TILLER ANGLE CONTROL 1* (*TAC1*) gene is one of the genes controlling inclined gravitropism. It is known that in plants such as rice, when the function of *TAC1* is lost, lateral branches grow more upward, and when *TAC1* is allowed to function excessively, lateral branches grow horizontally.

### Primer set for full-length amplification of Ha412HOChr04g0188691 gene

Forward primer (SEQ ID NO: 26)
   5'-TCGTTCAACAACACATACATAAAA-3'
Reverse primer (SEQ ID NO: 27)
   5'-TACGGATACATGTAATTTTTGAGG-3'

### (5) Examination of presence or absence of Ha412HOChr04g0188691 gene expression

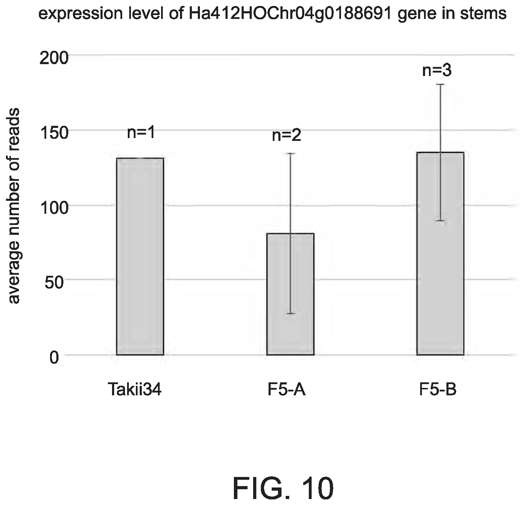

Next, it was examined whether or not the *Ha412HOChr04g0188691* gene is expressed in a sunflower plant showing the upright trait. Specifically, for Takii34 (1 individual), F5-A (2 individuals) which is a homozygous form (A) for the upright trait from a selfing progeny of F4 in which the Indel marker was a heterozygous form, and F5-B (3 individuals), which is a homozygous form (B) for the horizontal trait from a selfing progeny of the F4, RNA-seq was performed, and expression levels of the *Ha412HOChr04g0188691* gene were compared. Stems of each individual were used for the RNA-seq. These results are shown in FIG. 10.

FIG. 10 is a graph showing the expression level of the *Ha412HOChr04g0188691* gene in stems. In FIG. 10, the vertical axis indicates the average number of reads, and the horizontal axis indicates the line of the sunflower plant. As shown in FIG. 10, no significant difference was observed in the expression level of the *Ha412HOChr04g0188691* gene among Takii34, F5-A, and F5-B. From these results, it was found that the *Ha412HOChr04g0188691* gene is expressed regardless of the genotype.

### (6) Examination of presence or absence of loss of function of Ha412HOChr04g0188691 gene

Next, it was examined whether or not a normal Ha412HOChr04g0188691 protein is synthesized in a sunflower plant showing the upright trait. Specifically, using RNA extracted from a stem of Takii34 as a template, cDNA of the *Ha412HOChr04g0188691* gene was synthesized, and the base sequence was determined (SEQ ID NO: 22). As a result, it was found that there is an insertion sequence of 855 bases after the 352nd position of the *Ha412HOChr04g0188691* gene. While the translated protein ccomprises of 258 amino acids in the wild-type, it ccomprises of 138 amino acids in Takii34, and it was found that a normal Ha412HOChr04g0188691 protein is not synthesized and an incomplete *Ha412HOChr04g0188691* protein is synthesized (SEQ ID NO: 23). From these results, it was suggested that in Takii34, the normal function of Ha412HOChr04g0188691 is lost due to the mutation of the *Ha412HOChr04g0188691* gene, and the upright trait is not controlled, showing the upright trait.

Base sequence of *Ha412HOChr04g0188691* gene showing the upright trait (SEQ ID NO: 22)

5’-ATGAAGATCTTCAACTGGGTTCATCGTCGATTTCATCACAAATATGAAGTTAGCCAA
GATGTGAAGAAAGCCGAATTTACTGCGAATGGGAAGGACAATCTATCACTTCTTGAA
AATGAAGGAATGGATGGTGTGTTTGATGGTTGGAAAGAAGGCATACTTGCTATTGGG
ACATTTGGGTTTGATCCTTCGTTATTGAAAGATTTCGATCATGAAGACGACACAT
ACTTATGTGAATACACGCGTAAAGAATTATCTATTGATCATGATGAGGATAATGGTGGA
GACCAAGAAGAAGAAGAAGAAGAAGTTGAGTTTCCATTGGTGTTCAAAGCTTG
TAAGTATGCAGTGGCGGACCCAAGAATTTTTCCATGGGGGTGCGAAACATTTTTAAAA
ATTTTAGGCCCCTAGGTATATAAGTAAAAAAATCGGTTCGTATCGGGTCGGTTCGGGTC
GGGTCATGTAAAACAAAAGAAATTGCGCCATAACGTCCCTACTCATGGTTTCTATCAC
AAACAAATTGATGCATAAGTTTATCGCTCCATAAAATAATGTTTCAATTTTAATTTTCAA
CCCTAGAAATCGTGTGTAATCAGCCTAAAAATTATCTAAACAACATAATCACCCTAAA
AATCATCTAAACAACCATAAACATCGTCAAAACACATGAAATTTCAAACCTATTTAAG
AATTTTGTTACCCTTTGGTGTCGGACGGCGGTGGTTCGCGGCTGCTGATGACGTGCTG
TTGTGGTCGATGATCGCTTGTAGGAGACGACCCAAGAGAGGGAGGGCGGGATGAATT
CTAAGGGACTATTGGGCTTATTTTAATTATTATACTTTGAACTTAGGTTGAGTTTGGTTA
ATGGGCTAGTAATTAGTGGGCTAATTATGTTTAATGAAATAGTGGGTTAAGGTATTGGG
TATTTGGGTTAAGTTGGGTAGTATAAGTTTATATAATTTAGATAGTTTTTTTAATTAGAG
TTTACTAAAAAATTTAAAATATAAATCGAAAACATTTTTTACCTAAGGTGTGTGGACGA
AAAATTCCAGGGGTGCAGATGAAAAAATTCAAGGGGTGCGGACGAAAAAATCCAAA
GGGTGCAGACGAGATTTTCAACGGAAAATAGCACTAAATTTTTTTTTCCCGGGGGTGC
GCCCGCCCACCTTGGGCTATATGTATGTCCGCCCTTGTAAGTATGGGTTCTTTCATGAT

AAAAAAGATGATCACACGCAATGTGATGACGTTTGTAAACCTAATGATCATGAAGACT
GTGTGGATGATCATACTGACCAGATGAAAAAGGGAAAGAAGGCTGGAGAAAGAACC
ACTCTTGCTGATCTGTTTTTGGCTGATTCTGATAAGAACTTGTTGAACAAGAAGTTGA
CTGATGAAGATGATCAAGAAGTCAAAGTCCACTACATACCTGAATCCAACATAAAAC
ATACTTCATGTGATGAAAGGGTTGCTTTGATCTCTAAAAAGAAGATAACGAAAGATGT
TGCTGCTCGCCCGATCAAGAAAATTAACCGAATGGTGAGGAAAATGTTGAAGAAGAA
GATCCATCCCGACGTCGAAAATCAAAAGGATAAATGCAGGTTTGAAGCCATGCGCGT
ACATGGATGA-3’

Amino acid sequence of incomplete Ha412HOChr04g0188691 protein (SEQ ID NO: 23)

MKIFNWVHRRFHHKYEVSQDVKKAEFTANGKDNLSLLENEGMDGVFDGWKEGILAIG
TFGFDPSLLKDFDHEDDDTYLCEYTRKELSIDHDEDNGGDQEEEEEEEVEFPLVFKACKY
AVADPRIFPWGCETFLKILGP

From the above, it was found that a sunflower plant showing the upright trait can be obtained from a progeny line by crossing using the deposited line Takii34 as a first parent and a sunflower plant not showing the upright trait as a second parent. Further, it was found that by using the SNP markers, a sunflower plant showing the upright trait of the present disclosure can be selected.

While the present disclosure has been described above with reference to exemplary embodiments and examples, the present disclosure is by no means limited thereto. Various changes and modifications that may become apparent to those skilled in the art may be made in the configuration and specifics of the present disclosure without departing from the scope of the present disclosure.

This application claims priority from Japanese Patent Application 2023-173251 filed on September 16, 2023, and incorporates all of its disclosure herein.

### <Supplementary Notes>

The whole or part of the exemplary embodiments and examples disclosed above can be described as, but not limited to, the following Supplementary Notes.

### <Sunflower plant>

### (Supplementary Note 1)

A sunflower plant comprising a mutant of *Ha412HOChr04g0188691* gene, wherein the peduncle is upright.

### (Supplementary Note 2)

The sunflower plant according to Supplementary Note 1,
wherein the mutant of the *Ha412HOChr04g0188691* gene comprises a base sequence in which one or several bases are deleted, inserted, substituted, and/or added in a base sequence of a wild-type *Ha412HOChr04g0188691* gene, and expresses an upright trait.

### (Supplementary Note 3)

The sunflower plant according to Supplementary Note 1 or 2,
wherein the mutant of the *Ha412HOChr04g0188691* gene comprises of an amino acid sequence in which one or several amino acids are inserted, deleted, substituted, and/or added in an amino acid sequence encoded by a wild-type *Ha412HOChr04g0188691* gene, and comprises a base sequence encoding a protein that expresses an upright trait.

### (Supplementary Note 4)

The sunflower plant according to any one of Supplementary Notes 1 to 3,
wherein the mutant is a mutant gene comprising a missense mutation relative to a base sequence of a wild-type *Ha412HOChr04g0188691* gene, and expresses an upright trait.

### (Supplementary Note 5)

The sunflower plant according to any one of Supplementary Notes 1 to 4,
wherein the mutant is a mutant gene comprising a frameshift mutation relative to a base sequence of a wild-type *Ha412HOChr04g0188691* gene, and expresses an upright trait.

### (Supplementary Note 6)

The sunflower plant according to any one of Supplementary Notes 1 to 5,
wherein the mutant is a mutant gene comprising a splicing mutation relative to a base sequence of a wild-type *Ha412HOChr04g0188691* gene, and expresses an upright trait.

### (Supplementary Note 7)

The sunflower plant according to any one of Supplementary Notes 1 to 6,
wherein the wild-type *Ha412HOChr04g0188691* gene is a gene comprising a polynucleotide of the following (P):
(P) any of polynucleotides (P1) to (P7) below:
(P1) a polynucleotide comprising of any of base sequences of SEQ ID NOs: 16 to 18;
(P2) a polynucleotide comprising of a base sequence in which one or several bases are deleted, substituted, inserted and/or added in the base sequence of the (P1), and encoding a protein that controls an upright trait;
(P3) a polynucleotide comprising of a base sequence having an identity of at least 80% to the base sequence of the (P1), and encoding a protein that controls an upright trait;
(P4) a polynucleotide comprising of a base sequence complementary to a polynucleotide that hybridizes under stringent conditions to the polynucleotide comprising of the base sequence of the (P1), and encoding a protein that controls an upright trait;
(P5) a polynucleotide encoding a protein comprising of any of amino acid sequences of SEQ ID NOs: 19 to 21;
(P6) a polynucleotide encoding a protein comprising of an amino acid sequence in which one or several amino acids are deleted, substituted, inserted and/or added in the amino acid sequence of the (P5), and that controls the upright trait of the peduncle;
(P7) a polynucleotide encoding a protein comprising of an amino acid sequence having at least 80 % identity to the amino acid sequence of the (P5), and that controls the upright trait of the peduncle.

### (Supplementary Note 8)

A sunflower plant comprising an upright locus of the peduncle on chromosome 4, wherein the peduncle is upright.

### (Supplementary Note 9)

The sunflower plant according to Supplementary Note 8,
wherein the upright locus of the peduncle is identified by a molecular marker selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314.

### (Supplementary Note 10)

The sunflower plant according to Supplementary Note 8 or 9,
wherein the upright locus of the peduncle is identified by a molecular marker selected from the group comprising of Ha_chr04_181000331, an Indel marker of *Ha412HOChr04g0188691* gene, and Ha_chr04_200021302.

### (Supplementary Note 11)

The sunflower plant according to any one of Supplementary Notes 8 to 10, wherein the upright locus of the peduncle is identified by an Indel marker of *Ha412HOChr04g0188691* gene.

### (Supplementary Note 12)

The sunflower plant according to any one of Supplementary Notes 8 to 11, wherein the upright locus of the peduncle is identified by a polynucleotide selected from the group comprising of (a) to (g) below:
(a) a polynucleotide of (a1), (a2), or (a3) below:
   (a1) a polynucleotide comprising of SEQ ID NO: 1;
   (a2) a polynucleotide that comprises of a base sequence in which one or several bases are deleted, substituted, inserted and/or added in the base sequence of the (a1) with the 101st base (A) of the (a1) being conserved;
   (a3) a polynucleotide that comprises of a base sequence having an identity of at least 80% to the base sequence of the (a1) with the 101st base (A) of the (a1) being conserved.
(b) a polynucleotide of (b1), (b2), or (b3) below:
   (b1) a polynucleotide comprising of the base sequence of SEQ ID NO: 2;
   (b2) a polynucleotide that comprises of a base sequence in which one or several bases are deleted, substituted, inserted and/or added in the base sequence of the (b1) with the 202nd to 213th bases (TAAAAAAAAAAA) of the (b1) being conserved;
   (b3) a polynucleotide that comprises of a base sequence having an identity of at least 80% to the base sequence of the (b1) with the 202nd to 213th bases (A) of the (b1) being conserved;
(c) a polynucleotide of (c1), (c2), or (c3) below:
   (c1) a polynucleotide comprising of the base sequence of SEQ ID NO: 3;
   (c2) a polynucleotide that comprises of a base sequence in which one or several bases are deleted, substituted, inserted and/or added in the base sequence of the (c1) with the 101st base (T) of the (c1) being conserved;
   (c3) a polynucleotide that comprises of a base sequence having an identity of at least 80% to the base sequence of the (c1) with the 101st base (T) of the (c1) being conserved;
(d) a polynucleotide of (d1), (d2), or (d3) below:
   (d1) a polynucleotide comprising of the base sequence of SEQ ID NO: 4;
   (d2) a polynucleotide that comprises of a base sequence in which one or several bases are deleted, substituted, inserted and/or added in the base sequence of the (d1) with the 101st base (C) of the (d1) being conserved;
   (d3) a polynucleotide that comprises of a base sequence having an identity of at least 80% to the base sequence of the (d1) with the 101st base (C) of the (d1) being conserved;
(e) a polynucleotide of (e1), (e2), or (e3) below:
   (e1) a polynucleotide comprising of the base sequence of SEQ ID NO: 5;
   (e2) a polynucleotide that comprises of a base sequence in which one or several bases are deleted, substituted, inserted and/or added in the base sequence of the (e1) with the 101st base (G) of the (e1) being conserved;
   (e3) a polynucleotide that comprises of a base sequence having an identity of at least 80% to the base sequence of the (e1) with the 101st base (G) of the (e1) being conserved;
(f) a polynucleotide of (f1), (f2), or (f3) below:
   (f1) a polynucleotide comprising of the base sequence of SEQ ID NO: 6;
   (f2) a polynucleotide that comprises of a base sequence in which one or several bases are deleted, substituted, inserted and/or added in the base sequence of the (f1) with the 101st base (A) of the (fl) being conserved;
   (f3) a polynucleotide that comprises of a base sequence having an identity of at least 80% to the base sequence of the (f1) with the 101st base (A) of the (f1) being conserved;
(g) a polynucleotide of (g1), (g2), or (g3) below:
   (g1) a polynucleotide having a polynucleotide of 1 to 855 bases between a polynucleotide comprising of the base sequence of SEQ ID NO: 14 and a polynucleotide comprising of the base sequence of SEQ ID NO: 15;
   (g2) a polynucleotide that comprises of a base sequence in which one or several bases are deleted, substituted, inserted and/or added in the base sequence of the (g1) with the base sequence of the polynucleotide of 1 to 855 bases in the (g1) being conserved;
   (g3) a polynucleotide that comprises of a base sequence having an identity of 80% or more to the base sequence of the (g1) with the base sequence of the polynucleotide of 1 to 855 bases in the (g1) being conserved.

### (Supplementary Note 13)

The sunflower plant according to any one of Supplementary Notes 8 to 12, wherein the upright locus of the peduncle is identified by a polynucleotide of the following (ga):
(ga) a polynucleotide of (gal), (ga2), or (ga3) below:
(gal) a polynucleotide comprising of the base sequence of SEQ ID NO: 13;
(ga2) a polynucleotide comprising of a base sequence in which one or several bases are deleted, substituted, inserted and/or added in the base sequence of the (gal);
(ga3) a polynucleotide comprising of a base sequence having an identity of at least 80% to the base sequence of the (gal).

### (Supplementary Note 14)

The sunflower plant according to Supplementary Note 12, wherein the upright locus of the peduncle is identified by a polynucleotide selected from the group comprising of the (c), (d), and (g).

### (Supplementary Note 15)

The sunflower plant according to Supplementary Note 14,
wherein the upright locus of the peduncle is identified by the polynucleotide of the (g).

### (Supplementary Note 16)

The sunflower plant according to any one of Supplementary Notes 8 to 15,
wherein the upright locus of the peduncle is located in a region between the sites of two molecular markers selected from the group comprising of Ha_chr04_172545117, Ha_chr04_175698294, Ha_chr04_181000331, an Indel marker of *Ha412HOChr04g0188691* gene, Ha_chr04_200021302, Ha_chr04_204904126, and Ha_chr04_208654314 in the chromosome.

### (Supplementary Note 17)

The sunflower plant according to Supplementary Note 16,
wherein the upright locus of the peduncle is located in a region
between the sites of molecular markers of the Ha_chr04_175698294 and the Ha_chr04_204904126, or
between the sites of molecular markers of the Ha_chr04_181000331 and the Ha_chr04_200021302
in the chromosome.

### (Supplementary Note 18)

The sunflower plant according to Supplementary Note 17,
wherein the upright locus of the peduncle is located in a region
between sites of molecular markers of the Ha_chr04_181000331 and the Ha_chr04_200021302 in the chromosome.

### (Supplementary Note 19)

The sunflower plant according to any one of Supplementary Notes 1 to 18,
wherein the sunflower plant is a sunflower plant identified by Accession No. FERM BP-22482 or a progeny line thereof.

### (Supplementary Note 20)

The sunflower plant according to any one of Supplementary Notes 1 to 19, wherein a peduncle angle is 0 to 45°.

### <Plant population>

### (Supplementary Note 21)

A plant population of sunflower plants,
wherein the plant population comprises sunflower plants in which the peduncle is upright, and in the plant population, a ratio of individuals in which a peduncle angle is 0 to 45° is at least 50%.

### (Supplementary Note 22)

The plant population of sunflower plants according to Supplementary Note 21, comprising the sunflower plant according to any one of Supplementary Notes 1 to 20.

### <Part>

### (Supplementary Note 23)

A part of the sunflower plant according to any one of Supplementary Notes 1 to 20.

### (Supplementary Note 24)

The part of a plant according to Supplementary Note 23, wherein the part of a plant is a flower, a stem, a leaf, a root, pollen, and/or a seed.

### <Method for producing>

### (Supplementary Note 25)

A method for producing a sunflower plant or a plant population of sunflower plants showing an upright trait, characterized by comprising following steps (a) and (b):
(a) a step of crossing the sunflower plant according to any one of Supplementary Notes 1 to 20 with another sunflower plant;
(b) a step of selecting a sunflower plant showing the upright trait from the sunflower plant or a progeny line thereof obtained by the step (a).

### (Supplementary Note 26)

The method for producing a sunflower plant or a plant population of sunflower plants showing an upright trait according to Supplementary Note 25, comprising following step (x) prior to the step (a):
(x) a step of selecting the sunflower plant according to any one of Supplementary Notes 1 to 20 from a test sunflower plant.

### (Supplementary Note 27)

The method for producing a sunflower plant or a plant population of sunflower plants according to Supplementary Note 26,
wherein the selection in the step (x) is the selection of a sunflower plant comprising a mutant of the *Ha412HOChr04g0188691* gene.

### (Supplementary Note 28)

The method for producing a sunflower plant or a plant population of sunflower plants showing an upright trait according to Supplementary Note 26,
wherein the selection in the step (x) is the selection of a sunflower plant showing the upright trait comprising an upright locus of the peduncle on chromosome 4.

### (Supplementary Note 29)

The method for producing a sunflower plant or a plant population of sunflower plants showing an upright trait according to Supplementary Note 28,
wherein the selection in the step (x) is the selection of a sunflower plant showing the upright trait having an upright locus of the peduncle identified by a molecular marker selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314.

### (Supplementary Note 30)

The method for producing a sunflower plant or a plant population of sunflower plants showing an upright trait according to Supplementary Note 29,
wherein the selection in the step (x) is the selection of a sunflower plant showing the upright trait having an upright locus of the peduncle identified by a molecular marker selected from the group comprising of Ha_chr04_181000331, an Indel marker of the *Ha412HOChr04g0188691* gene, and Ha_chr04_200021302.

### (Supplementary Note 31)

The method for producing a sunflower plant or a plant population of sunflower plants showing an upright trait according to Supplementary Note 30,
wherein the selection in the step (x) is the selection of a sunflower plant showing the upright trait having an upright locus of the peduncle identified by an Indel marker of the *Ha412HOChr04g0188691* gene.

### (Supplementary Note 32)

The method for producing a sunflower plant or a plant population of sunflower plants showing an upright trait according to any one of Supplementary Notes 28 to 31,
wherein the selection in the step (x) is a method for selecting a sunflower plant showing the upright trait having an upright locus of the peduncle identified by a polynucleotide selected from the group comprising of (a) to (g) below:
(a) a polynucleotide of (a1), (a2), or (a3) below:
   (a1) a polynucleotide comprising of SEQ ID NO: 1;
   (a2) a polynucleotide that comprises of a base sequence in which one or several bases are deleted, substituted, inserted and/or added in the base sequence of the (a1) with the 101st base (A) of the (a1) being conserved;
   (a3) a polynucleotide that comprises of a base sequence having an identity of at least 80% to the base sequence of the (a1) with the 101st base (A) of the (a1) being conserved.
(b) a polynucleotide of (b1), (b2), or (b3) below:
   (b1) a polynucleotide comprising of the base sequence of SEQ ID NO: 2;
   (b2) a polynucleotide that comprises of a base sequence in which one or several bases are deleted, substituted, inserted and/or added in the base sequence of the (b1) with the 202nd to 213th bases (TAAAAAAAAAAA) of the (b1) being conserved;
   (b3) a polynucleotide that comprises of a base sequence having an identity of at least 80% to the base sequence of the (bl) with the 202nd to 213th bases (A) of the (b1) being conserved;
(c) a polynucleotide of (c1), (c2), or (c3) below:
   (c1) a polynucleotide comprising of the base sequence of SEQ ID NO: 3;
   (c2) a polynucleotide that comprises of a base sequence in which one or several bases are deleted, substituted, inserted and/or added in the base sequence of the (c1) with the 101st base (T) of the (c1) being conserved;
   (c3) a polynucleotide that comprises of a base sequence having an identity of at least 80% to the base sequence of the (c1) with the 101st base (T) of the (c1) being conserved;
(d) a polynucleotide of (d1), (d2), or (d3) below:
   (d1) a polynucleotide comprising of the base sequence of SEQ ID NO: 4;
   (d2) a polynucleotide that comprises of a base sequence in which one or several bases are deleted, substituted, inserted and/or added in the base sequence of the (d1) with the 101st base (C) of the (d1) being conserved;
   (d3) a polynucleotide that comprises of a base sequence having an identity of at least 80% to the base sequence of the (d1) with the 101st base (C) of the (d1) being conserved;
(e) a polynucleotide of (e1), (e2), or (e3) below:
   (e1) a polynucleotide comprising of the base sequence of SEQ ID NO: 5;
   (e2) a polynucleotide that comprises of a base sequence in which one or several bases are deleted, substituted, inserted and/or added in the base sequence of the (e1) with the 101st base (G) of the (e1) being conserved;
   (e3) a polynucleotide that comprises of a base sequence having an identity of at least 80% to the base sequence of the (e1) with the 101st base (G) of the (e1) being conserved;
(f) a polynucleotide of (f1), (f2), or (f3) below:
   (f1) a polynucleotide comprising of the base sequence of SEQ ID NO: 6;
   (f2) a polynucleotide that comprises of a base sequence in which one or several bases are deleted, substituted, inserted and/or added in the base sequence of the (f1) with the 101st base (A) of the (f1) being conserved;
   (f3) a polynucleotide that comprises of a base sequence having an identity of at least 80% to the base sequence of the (f1) with the 101st base (A) of the (f1) being conserved;
(g) a polynucleotide of (g1), (g2), or (g3) below:
   (g1) a polynucleotide having a polynucleotide of 1 to 855 bases between a polynucleotide comprising of the base sequence of SEQ ID NO: 14 and a polynucleotide comprising of the base sequence of SEQ ID NO: 15;
   (g2) a polynucleotide that comprises of a base sequence in which one or several bases are deleted, substituted, inserted and/or added in the base sequence of the (g1) with the base sequence of the polynucleotide of 1 to 855 bases in the (g1) being conserved;
   (g3) a polynucleotide that comprises of a base sequence having an identity of 80% or more to the base sequence of the (g1) with the base sequence of the polynucleotide of 1 to 855 bases in the (g1) being conserved.

### (Supplementary Note 33)

The method for producing a sunflower plant or a plant population of sunflower plants showing an upright trait according to any one of Supplementary Notes 28 to 32,
wherein the selection in the step (x) is a method for selecting a sunflower plant showing the upright trait having an upright locus of the peduncle identified by a polynucleotide of the following (ga):
(ga) a polynucleotide of (gal), (ga2), or (ga3) below:
(gal) a polynucleotide comprising of the base sequence of SEQ ID NO: 13;
(ga2) a polynucleotide comprising of a base sequence in which one or several bases are deleted, substituted, inserted and/or added in the base sequence of the (gal);
(ga3) a polynucleotide comprising of a base sequence having an identity of at least 80% to the base sequence of the (gal).

### (Supplementary Note 34)

The method for producing a sunflower plant or a plant population of sunflower plants showing an upright trait according to Supplementary Note 32,
wherein the selection in the step (x) is the selection of a sunflower plant showing the upright trait having an upright locus of the peduncle identified by a polynucleotide selected from the group comprising of the (c), (d), and (g).

### (Supplementary Note 35)

The method for producing a sunflower plant or a plant population of sunflower plants showing an upright trait according to Supplementary Note 34,
wherein the selection in the step (x) is the selection of a sunflower plant showing the upright trait having an upright locus of the peduncle identified by the polynucleotide of the (g).

### (Supplementary Note 36)

The method for producing a sunflower plant or a plant population of sunflower plants showing an upright trait according to any one of Supplementary Notes 28 to 35,
wherein the selection in the step (x) is the selection of a sunflower plant showing the upright trait having an upright locus of the peduncle located in a region between sites of two molecular markers selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 in the chromosome.

### (Supplementary Note 37)

The method for producing a sunflower plant or a plant population of sunflower plants showing an upright trait according to Supplementary Note 36,
wherein the selection in the step (x) is the selection of a sunflower plant showing the upright trait having an upright locus of the peduncle located in a region
between the sites of molecular markers of the Ha_chr04_175698294 and the Ha_chr04_204904126, or
between the sites of molecular markers of the Ha_chr04_181000331 and the Ha_chr04_200021302
in the chromosome.

### (Supplementary Note 38)

The method for producing a sunflower plant or a plant population of sunflower plants showing an upright trait according to Supplementary Note 37,
wherein the selection in the step (x) is the selection of a sunflower plant showing the upright trait having an upright locus of the peduncle located in a region
between the sites of molecular markers of the Ha_chr04_181000331 and the Ha_chr04_200021302 in the chromosome.

### (Supplementary Note 39)

The method for producing a sunflower plant or a plant population of sunflower plants showing an upright trait according to any one of Supplementary Notes 28 to 38,
wherein the selection in the step (x) is the selection of a sunflower plant in which a peduncle angle is 0 to 45°.

### (Supplementary Note 40)

The method for producing a sunflower plant or a plant population of sunflower plants according to any one of Supplementary Notes 25 to 39,
wherein the selection in the step (b) is the selection of a sunflower plant comprising a mutant of the *Ha412HOChr04g0188691* gene.

### (Supplementary Note 41)

The method for producing a sunflower plant or a plant population of sunflower plants showing an upright trait according to any one of Supplementary Notes 25 to 40,
wherein the selection in the step (b) is the selection of a sunflower plant showing the upright trait comprising an upright locus of the peduncle on chromosome 4.

### (Supplementary Note 42)

The method for producing a sunflower plant or a plant population of sunflower plants showing an upright trait according to any one of Supplementary Notes 25 to 40,
wherein the selection in the step (b) is the selection of a sunflower plant showing the upright trait having an upright locus of the peduncle identified by a molecular marker selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314.

### (Supplementary Note 43)

The method for producing a sunflower plant or a plant population of sunflower plants showing an upright trait according to Supplementary Note 42,
wherein the selection in the step (b) is the selection of a sunflower plant showing the upright trait having an upright locus of the peduncle identified by a molecular marker selected from the group comprising of Ha_chr04_181000331, an Indel marker of the *Ha412HOChr04g0188691* gene, and Ha_chr04_200021302.

### (Supplementary Note 44)

The method for producing a sunflower plant or a plant population of sunflower plants showing an upright trait according to Supplementary Note 43,
wherein the selection in the step (b) is the selection of a sunflower plant showing the upright trait having an upright locus of the peduncle identified by an Indel marker of the *Ha412HOChr04g0188691* gene.

### (Supplementary Note 45)

The method for producing a sunflower plant or a plant population of sunflower plants showing an upright trait according to any one of Supplementary Notes 25 to 44,
wherein the selection in the step (b) is a method for selecting a sunflower plant showing the upright trait having an upright locus of the peduncle identified by a polynucleotide selected from the group comprising of (a) to (g) below:
(a) a polynucleotide of (a1), (a2), or (a3) below:
   (a1) a polynucleotide comprising of SEQ ID NO: 1;
   (a2) a polynucleotide that comprises of a base sequence in which one or several bases are deleted, substituted, inserted and/or added in the base sequence of the (a1) with the 101st base (A) of the (a1) being conserved;
   (a3) a polynucleotide that comprises of a base sequence having an identity of at least 80% to the base sequence of the (a1) with the 101st base (A) of the (a1) being conserved.
(b) a polynucleotide of (b1), (b2), or (b3) below:
   (b1) a polynucleotide comprising of the base sequence of SEQ ID NO: 2;
   (b2) a polynucleotide that comprises of a base sequence in which one or several bases are deleted, substituted, inserted and/or added in the base sequence of the (b1) with the 202nd to 213th bases (TAAAAAAAAAAA) of the (b1) being conserved;
   (b3) a polynucleotide that comprises of a base sequence having an identity of at least 80% to the base sequence of the (bl) with the 202nd to 213th bases (A) of the (b1) being conserved;
(c) a polynucleotide of (c1), (c2), or (c3) below:
   (c1) a polynucleotide comprising of the base sequence of SEQ ID NO: 3;
   (c2) a polynucleotide that comprises of a base sequence in which one or several bases are deleted, substituted, inserted and/or added in the base sequence of the (c1) with the 101st base (T) of the (c1) being conserved;
   (c3) a polynucleotide that comprises of a base sequence having an identity of at least 80% to the base sequence of the (c1) with the 101st base (T) of the (c1) being conserved;
(d) a polynucleotide of (d1), (d2), or (d3) below:
   (d1) a polynucleotide comprising of the base sequence of SEQ ID NO: 4;
   (d2) a polynucleotide that comprises of a base sequence in which one or several bases are deleted, substituted, inserted and/or added in the base sequence of the(d1) with the 101st base (C) of the (d1) being conserved;
   (d3) a polynucleotide that comprises of a base sequence having an identity of at least 80% to the base sequence of the (d1) with the 101st base (C) of the (d1) being conserved;
(e) a polynucleotide of (e1), (e2), or (e3) below:
   (e1) a polynucleotide comprising of the base sequence of SEQ ID NO: 5;
   (e2) a polynucleotide that comprises of a base sequence in which one or several bases are deleted, substituted, inserted and/or added in the base sequence of the (e1) with the 101st base (G) of the (e1) being conserved;
   (e3) a polynucleotide that comprises of a base sequence having an identity of at least 80% to the base sequence of the (e1) with the 101st base (G) of the (e1) being conserved;
(f) a polynucleotide of (f1), (f2), or (f3) below:
   (f1) a polynucleotide comprising of the base sequence of SEQ ID NO: 6;
   (f2) a polynucleotide that comprises of a base sequence in which one or several bases are deleted, substituted, inserted and/or added in the base sequence of the (f1) with the 101st base (A) of the (f1) being conserved;
   (f3) a polynucleotide that comprises of a base sequence having an identity of at least 80% to the base sequence of the (f1) with the 101st base (A) of the (f1) being conserved;
(g) a polynucleotide of (g1), (g2), or (g3) below:
   (g1) a polynucleotide having a polynucleotide of 1 to 855 bases between a polynucleotide comprising of the base sequence of SEQ ID NO: 14 and a polynucleotide comprising of the base sequence of SEQ ID NO: 15;
   (g2) a polynucleotide that comprises of a base sequence in which one or several bases are deleted, substituted, inserted and/or added in the base sequence of the (g1) with the base sequence of the polynucleotide of 1 to 855 bases in the (g1) being conserved;
   (g3) a polynucleotide that comprises of a base sequence having an identity of 80% or more to the base sequence of the (g1) with the base sequence of the polynucleotide of 1 to 855 bases in the (g1) being conserved.

### (Supplementary Note 46)

The method for producing a sunflower plant or a plant population of sunflower plants showing an upright trait according to any one of Supplementary Notes 25 to 45,
wherein the selection in the step (b) is a method for selecting a sunflower plant showing the upright trait having an upright locus of the peduncle identified by a polynucleotide of the following (ga):
(ga) a polynucleotide of (gal), (ga2), or (ga3) below:
(gal) a polynucleotide comprising of the base sequence of SEQ ID NO: 13;
(ga2) a polynucleotide comprising of a base sequence in which one or several bases are deleted, substituted, inserted and/or added in the base sequence of the (gal);
(ga3) a polynucleotide comprising of a base sequence having an identity of at least 80% to the base sequence of the (gal).

### (Supplementary Note 47)

The method for producing a sunflower plant or a plant population of sunflower plants showing an upright trait according to Supplementary Note 45,
wherein the selection in the step (b) is the selection of a sunflower plant showing the upright trait having an upright locus of the peduncle identified by a polynucleotide selected from the group comprising of the (c), (d), and (g).

### (Supplementary Note 48)

The method for producing a sunflower plant or a plant population of sunflower plants showing an upright trait according to Supplementary Note 47,
wherein the selection in the step (b) is the selection of a sunflower plant showing the upright trait having an upright locus of the peduncle identified by the polynucleotide of the (g).

### (Supplementary Note 49)

The method for producing a sunflower plant or a plant population of sunflower plants showing an upright trait according to any one of Supplementary Notes 25 to 48,
wherein the selection in the step (b) is the selection of a sunflower plant showing the upright trait having an upright locus of the peduncle located in a region between sites of two molecular markers selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 in the chromosome.

### (Supplementary Note 50)

The method for producing a sunflower plant or a plant population of sunflower plants showing an upright trait according to Supplementary Note 49,
wherein the selection in the step (b) is the selection of a sunflower plant showing the upright trait having an upright locus of the peduncle located in a region
between the sites of molecular markers of the Ha_chr04_175698294 and the Ha_chr04_204904126, or
between the sites of molecular markers of the Ha_chr04_181000331 and the Ha_chr04_200021302
in the chromosome.

### (Supplementary Note 51)

The method for producing a sunflower plant or a plant population of sunflower plants showing an upright trait according to Supplementary Note 50,
wherein the selection in the step (b) is the selection of a sunflower plant showing the upright trait having an upright locus of the peduncle located in a region
between the sites of molecular markers of the Ha_chr04_181000331 and the Ha_chr04_200021302 in the chromosome.

### <Method for imparting>

### (Supplementary Note 52)

A method for imparting an upright trait to a sunflower plant, the method comprising an upright step of making a peduncle of a sunflower plant upright by changing a function of the *Ha412HOChr04g0188691* gene of the sunflower plant.

### (Supplementary Note 53)

The method for imparting according to Supplementary Note 52
wherein the upright step comprises a suppression step of suppressing the gene expression of the *Ha412HOChr04g0188691* gene of the sunflower plant.

### (Supplementary Note 54)

The method for imparting according to Supplementary Note 52 or 53,
wherein the upright step comprises a mutation step of mutating the *Ha412HOChr04g0188691* gene of the sunflower plant.

### (Supplementary Note 55)

The method for imparting according to Supplementary Note 54,
wherein in the mutation step, a mutation is introduced into a wild-type *Ha412HOChr04g0188691* gene of the sunflower plant to produce an upright sunflower plant comprising a mutant of the *Ha412HOChr04g0188691* gene and in which the peduncle is upright.

### (Supplementary Note 56)

The method for imparting according to Supplementary Note 55,
wherein the mutant comprises a base sequence in which one or several bases are deleted, substituted, inserted and/or added in a base sequence of a wild-type *Ha412HOChr04g0188691* gene, and expresses an upright trait.

### (Supplementary Note 57)

The method for imparting according to Supplementary Note 55 or 56,
wherein the mutant is a mutant gene comprising a missense mutation relative to a base sequence of a wild-type *Ha412HOChr04g0188691* gene, and expresses an upright trait.

### (Supplementary Note 58)

The method for imparting according to Supplementary Note 55 or 56,
wherein the mutant is a mutant gene comprising a frameshift mutation relative to a base sequence of a wild-type *Ha412HOChr04g0188691* gene, and expresses an upright trait.

### (Supplementary Note 59)

The method for imparting according to any one of Supplementary Notes 55 to 58,
wherein the mutant is a mutant gene comprising a splicing mutation relative to a base sequence of a wild-type *Ha412HOChr04g0188691* gene, and expresses an upright trait.

### (Supplementary Note 60)

The method for imparting according to any one of Supplementary Notes 55 to 59,
wherein the wild-type *Ha412HOChr04g0188691* gene is a gene comprising a polynucleotide of the following (P):
(P) any of polynucleotides (P1) to (P7) below:
(P1) a polynucleotide comprising of any of base sequences of SEQ ID NOs: 16 to 18;
(P2) a polynucleotide comprising of a base sequence in which one or several bases are deleted, substituted, inserted and/or added in the base sequence of the (P1), and encoding a protein that controls an upright trait;
(P3) a polynucleotide comprising of a base sequence having an identity of at least 80% to the base sequence of the (P1), and encoding a protein that controls an upright trait;
(P4) a polynucleotide comprising of a base sequence complementary to a polynucleotide that hybridizes under stringent conditions to the polynucleotide comprising of the base sequence of the (P1), and encoding a protein that controls an upright trait;
(P5) a polynucleotide encoding a protein comprising of any of the amino acid sequences of SEQ ID NOs: 19 to 21;
(P6) a polynucleotide encoding a protein comprising of an amino acid sequence in which one or several amino acids are deleted, substituted, inserted and/or added in the amino acid sequence of the (P5), and that controls the upright trait of the peduncle;
(P7) a polynucleotide encoding a protein comprising of an amino acid sequence having at least 80% identity to the amino acid sequence of the (P5), and that controls the upright trait of the peduncle.

### (Supplementary Note 61)

A method for imparting an upright trait to a sunflower plant, comprising an introduction step of introducing an upright locus of the peduncle on chromosome 4 into a sunflower plant.

### (Supplementary Note 62)

The method for imparting according to Supplementary Note 61,
wherein the upright locus of the peduncle is identified by a molecular marker selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314.

### (Supplementary Note 63)

The method for imparting according to Supplementary Note 62,
wherein it is identified by a molecular marker selected from the group comprising of Ha_chr04_181000331, an Indel marker of the *Ha412HOChr04g0188691* gene, and Ha_chr04_200021302.

### (Supplementary Note 64)

The method for imparting according to Supplementary Note 63,
wherein it is identified by an Indel marker of the *Ha412HOChr04g0188691* gene.

### (Supplementary Note 65)

The method for imparting according to any one of Supplementary Notes 61 to 64,
wherein the upright locus of the peduncle is identified by a polynucleotide selected from the group comprising of (a) to (g) below:
(a) a polynucleotide of (a1), (a2), or (a3) below:
   (a1) a polynucleotide comprising of SEQ ID NO: 1;
   (a2) a polynucleotide that comprises of a base sequence in which one or several bases are deleted, substituted, inserted and/or added in the base sequence of the (a1) with the 101st base (A) of the (a1) being conserved;
   (a3) a polynucleotide that comprises of a base sequence having an identity of at least 80% to the base sequence of the (a1) with the 101st base (A) of the (a1) being conserved.
(b) a polynucleotide of (b1), (b2), or (b3) below:
   (b1) a polynucleotide comprising of the base sequence of SEQ ID NO: 2;
   (b2) a polynucleotide that comprises of a base sequence in which one or several bases are deleted, substituted, inserted and/or added in the base sequence of the (b1) with the 202nd to 213th bases (TAAAAAAAAAAA) of the (b1) being conserved;
   (b3) a polynucleotide that comprises of a base sequence having an identity of at least 80% to the base sequence of the (bl) with the 202nd to 213th bases (A) of the (b1) being conserved;
(c) a polynucleotide of (c1), (c2), or (c3) below:
   (c1) a polynucleotide comprising of the base sequence of SEQ ID NO: 3;
   (c2) a polynucleotide that comprises of a base sequence in which one or several bases are deleted, substituted, inserted and/or added in the base sequence of the (c1) with the 101st base (T) of the (c1) being conserved;
   (c3) a polynucleotide that comprises of a base sequence having an identity of at least 80% to the base sequence of the (c1) with the 101st base (T) of the (c1) being conserved;
(d) a polynucleotide of (d1), (d2), or (d3) below:
   (d1) a polynucleotide comprising of the base sequence of SEQ ID NO: 4;
   (d2) a polynucleotide that comprises of a base sequence in which one or several bases are deleted, substituted, inserted and/or added in the base sequence of the (d1) with the 101st base (C) of the (d1) being conserved;
   (d3) a polynucleotide that comprises of a base sequence having an identity of at least80 % to the base sequence of the (d1) with the 101st base (C) of the (d1) being conserved;
(e) a polynucleotide of (e1), (e2), or (e3) below:
   (e1) a polynucleotide comprising of the base sequence of SEQ ID NO: 5;
   (e2) a polynucleotide that comprises of a base sequence in which one or several bases are deleted, substituted, inserted and/or added in the base sequence of the (e1) with the 101st base (G) of the (e1) being conserved;
   (e3) a polynucleotide that comprises of a base sequence having an identity of at least 80% to the base sequence of the (e1) with the 101st base (G) of the (e1) being conserved;
(f) a polynucleotide of (f1), (f2), or (f3) below:
   (f1) a polynucleotide comprising of the base sequence of SEQ ID NO: 6;
   (f2) a polynucleotide that comprises of a base sequence in which one or several bases are deleted, substituted, inserted and/or added in the base sequence of the (f1) with the 101st base (A) of the (f1) being conserved;
   (f3) a polynucleotide that comprises of a base sequence having an identity of at least 80% to the base sequence of the (f1) with the 101st base (A) of the (f1) being conserved;
(g) a polynucleotide of (g1), (g2), or (g3) below:
   (g1) a polynucleotide having a polynucleotide of 1 to 855 bases between a polynucleotide comprising of the base sequence of SEQ ID NO: 14 and a polynucleotide comprising of the base sequence of SEQ ID NO: 15;
   (g2) a polynucleotide that comprises of a base sequence in which one or several bases are deleted, substituted, inserted and/or added in the base sequence of the (g1) with the base sequence of the polynucleotide of 1 to 855 bases in the (g1) being conserved;
   (g3) a polynucleotide that comprises of a base sequence having an identity of 80% or more to the base sequence of the (g1) with the base sequence of the polynucleotide of 1 to 855 bases in the (g1) being conserved.

### (Supplementary Note 66)

The method for imparting according to any one of Supplementary Notes 61 to 65,
wherein the upright locus of the peduncle is identified by a polynucleotide of the following (ga):
(ga) a polynucleotide of (gal), (ga2), or (ga3) below:
(gal) a polynucleotide comprising of the base sequence of SEQ ID NO: 13;
(ga2) a polynucleotide comprising of a base sequence in which one or several bases are deleted, substituted, inserted and/or added in the base sequence of the (gal);
(ga3) a polynucleotide comprising of a base sequence having an identity of at least 80% to the base sequence of the (gal).

### (Supplementary Note 67)

The method for imparting according to Supplementary Note 65,
wherein the upright locus of the peduncle is identified by a polynucleotide selected from the group comprising of the (c), (d), and (g).

### (Supplementary Note 68)

The method for imparting according to Supplementary Note 67,
wherein the upright locus of the peduncle is identified by the polynucleotide of the (g).

### (Supplementary Note 69)

The method for imparting according to any one of Supplementary Notes 61 to 68,
wherein the upright locus of the peduncle is located in a region between the sites of the two molecular markers selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 on the chromosome.

### (Supplementary Note 70)

The method for imparting according to Supplementary Note 69,
wherein the upright locus of the peduncle is located in a region
between the sites of molecular markers of the Ha__chr04_175698294 and the Ha_chr04_204904126, or
between the sites of molecular markers of the Ha_chr04_181000331 and the Ha_chr04_200021302
on the chromosome.

### (Supplementary Note 71)

The method for imparting according to Supplementary Note 70,
wherein the upright locus of the peduncle is located in a region
between the sites of molecular markers of the Ha_chr04_181000331 and the Ha_chr04_200021302 on the chromosome.

### (Supplementary Note 72)

The method for imparting according to any one of Supplementary Notes 61 to 71,
wherein the upright locus of the peduncle is derived from an upright locus of the peduncle of a sunflower plant identified by Accession No. FERM BP-22482.

### (Supplementary Note 73)

The method for imparting according to any one of Supplementary Notes 61 to 72,
wherein the upright locus of the peduncle on the chromosome 4 is introduced by crossing with the sunflower plant according to any one of Supplementary Notes 8 to 18.

### (Supplementary Note 74)

The method for imparting according to Supplementary Note 73,
wherein the sunflower plant is a sunflower plant identified by Accession No. FERM BP-22482 or a progeny line thereof.

### <Nucleic acid>

### (Supplementary Note 75)

An isolated nucleic acid having a function capable of determining an upright trait of the peduncle of a sunflower plant, comprising a contiguous base sequence of at least 15 bases in a base sequence selected from the group comprising of (a) to (g) below in a chromosome of a sunflower plant:
(a) a base sequence of the following (a1), (a2), or (a3):
   (a1) the base sequence of SEQ ID NO: 1;
   (a2) a base sequence in which one or several bases are deleted, substituted, inserted and/or added in the base sequence of the (a1) with the 101st base (A) of the (a1) being conserved;
   (a3) a base sequence having an identity of at least 80% to the base sequence of the (a1) with the 101st base (A) of the (a1) being conserved;
(b) a base sequence of the following (b1), (b2), or (b3):
   (b1) the base sequence of SEQ ID NO: 2;
   (b2) a base sequence in which one or several bases are deleted, substituted, inserted and/or added in the base sequence of the (b1) with the 202nd to 213th bases (TAAAAAAAAAAA) of the (b1) being conserved;
   (b3) a base sequence having an identity of at least 80% to the base sequence of the (b1) with the 202nd to 213th bases (A) of the (b1) being conserved;
(c) a base sequence of the following (c1), (c2), or (c3):
   (c1) the base sequence of SEQ ID NO: 3;
   (c2) a base sequence in which one or several bases are deleted, substituted, inserted and/or added in the base sequence of the (c1) with the 101st base (T) of the (c1) being conserved;
   (c3) a base sequence having an identity of at least 80% to the base sequence of the (cl) with the 101st base (T) of the (c1) being conserved;
(d) a base sequence of the following (d1), (d2), or (d3):
   (d1) the base sequence of SEQ ID NO: 4;
   (d2) a base sequence in which one or several bases are deleted, substituted, inserted and/or added in the base sequence of the (d1) with the 101st base (C) of the (d1) being conserved;
   (d3) a base sequence having an identity of at least 80% to the base sequence of the (d1) with the 101st base (C) of the (d1) being conserved;
(e) a base sequence of the following (e1), (e2), or (e3):
   (e1) the base sequence of SEQ ID NO: 5;
   (e2) a base sequence in which one or several bases are deleted, substituted, inserted and/or added in the base sequence of the (e1) with the 101st base (G) of the (e1) being conserved;
   (e3) a base sequence having an identity of at least 80% to the base sequence of the (el) with the 101st base (G) of the (e1) being conserved;
(f) a base sequence of the following (f1), (f2), or (f3):
   (f1) the base sequence of SEQ ID NO: 6;
   (f2) a base sequence in which one or several bases are deleted, substituted, inserted and/or added in the base sequence of the (f1) with the 101st base (A) of the (f1) being conserved;
   (f3) a base sequence having an identity of at least 80% to the base sequence of the (f1) with the 101st base (A) of the (f1) being conserved;
(g) a base sequence of the following (g1), (g2), or (g3):
   (g1) a base sequence having a base sequence of 1 to 855 bases between the base sequence of SEQ ID NO: 14 and the base sequence of SEQ ID NO: 15;
   (g2) a base sequence in which one or several bases are deleted, substituted, inserted and/or added in the base sequence of the (g1) with the base sequence of the polynucleotide of 1 to 855 bases in the (g1) being conserved;
   (g3) a base sequence having an identity of 80% or more to the base sequence of the (g1) with the base sequence of the polynucleotide of 1 to 855 bases in the (g1) being conserved.

### <Detection method>

### (Supplementary Note 77)

A method for detecting an upright trait of a peduncle of a sunflower plant, the method comprising a detection step of detecting whether the *Ha412HOChr04g0188691* gene is mutated in a test sunflower plant.

### (Supplementary Note 78)

The detection method according to Supplementary Note 77,
wherein the detection step comprises a detection step of detecting whether a mutant of the *Ha412HOChr04g0188691* gene is present in the test sunflower plant.

### (Supplementary Note 79)

The detection method according to Supplementary Note 78,
wherein the mutant comprises a base sequence in which one or several bases are deleted, substituted, inserted and/or added in a base sequence of a wild-type *Ha412HOChr04g0188691* gene, and expresses an upright trait.

### (Supplementary Note 80)

The detection method according to Supplementary Note 78 or 79,
wherein the mutant is a mutant gene comprising a missense mutation relative to a base sequence of a wild-type *Ha412HOChr04g0188691* gene, and expresses an upright trait.

### (Supplementary Note 81)

The detection method according to any one of Supplementary Notes 78 to 80, wherein the mutant is a mutant gene comprising a frameshift mutation relative to a base sequence of a wild-type *Ha412HOChr04g0188691* gene, and expresses an upright trait.

### (Supplementary Note 82)

The detection method according to any one of Supplementary Notes 78 to 81,
wherein the mutant is a mutant gene comprising a splicing mutation relative to a base sequence of a wild-type *Ha412HOChr04g0188691* gene, and expresses an upright trait.

### (Supplementary Note 83)

The detection method according to any one of Supplementary Notes 79 to 82,
wherein the wild-type *Ha412HOChr04g0188691* gene is a gene comprising a polynucleotide of the following (P):
(P) any of polynucleotides (P1) to (P7) below:
(P1) a polynucleotide comprising of any of the base sequences of SEQ ID NOs: 16 to 18;
(P2) a polynucleotide comprising of a base sequence in which one or several bases are deleted, substituted, inserted and/or added in the base sequence of the (P1), and encoding a protein that controls an upright trait;
(P3) a polynucleotide comprising of a base sequence having an identity of at least 80% to the base sequence of the (P1), and encoding a protein that controls an upright trait;
(P4) a polynucleotide comprising of a base sequence complementary to a polynucleotide that hybridizes under stringent conditions to the polynucleotide comprising of the base sequence of the (P1), and encoding a protein that controls an upright trait;
(P5) a polynucleotide encoding a protein comprising of any of the amino acid sequences of SEQ ID NOs: 19 to 21;
(P6) a polynucleotide encoding a protein comprising of an amino acid sequence in which one or several amino acids are deleted, substituted, inserted and/or added in the amino acid sequence of the (P5), and that controls the upright trait of the peduncle;
(P7) a polynucleotide encoding a protein comprising of an amino acid sequence having at least 80% identity to the amino acid sequence of the (P5), and that controls the upright trait of the peduncle.

### (Supplementary Note 84)

A method for detecting an upright trait of a peduncle of a sunflower plant, the method comprising a detection step of detecting an upright locus of the peduncle on chromosome 4 for a test sunflower plant.

### (Supplementary Note 85)

The detection method according to Supplementary Note 84,
wherein the upright locus of the peduncle is detected by detecting a molecular marker selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 in the detection step.

### (Supplementary Note 86)

The detection method according to Supplementary Note 85,
wherein the upright locus of the peduncle is detected by detecting a molecular marker selected from the group comprising of Ha_chr04_181000331, an Indel marker of the *Ha412HOChr04g0188691* gene, and Ha_chr04_200021302 in the detection step.

### (Supplementary Note 87)

The detection method according to Supplementary Note 86,
wherein the upright locus of the peduncle is detected by detecting an Indel marker of the *Ha412HOChr04g0188691* gene in the detection step.

### (Supplementary Note 88)

The detection method according to any one of Supplementary Notes 84 to 87,
wherein the upright locus of the peduncle is detected by detecting a polynucleotide selected from the group comprising of (a) to (g) below in the detection step:
(a) a polynucleotide of (a1), (a2), or (a3) below:
   (a1) a polynucleotide comprising of SEQ ID NO: 1;
   (a2) a polynucleotide that comprises of a base sequence in which one or several bases are deleted, substituted, inserted and/or added in the base sequence of the (a1) with the 101st base (A) of the (a1) being conserved;
   (a3) a polynucleotide that comprises of a base sequence having an identity of at least 80% to the base sequence of the (a1) with the 101st base (A) of the (a1) being conserved.
(b) a polynucleotide of (b1), (b2), or (b3) below:
   (b1) a polynucleotide comprising of the base sequence of SEQ ID NO: 2;
   (b2) a polynucleotide that comprises of a base sequence in which one or several bases are deleted, substituted, inserted and/or added in the base sequence of the (b1) with the 202nd to 213th bases (TAAAAAAAAAAA) of the (b1) being conserved;
   (b3) a polynucleotide that comprises of a base sequence having an identity of at least 80% to the base sequence of the (bl) with the 202nd to 213th bases (A) of the (b1) being conserved;
(c) a polynucleotide of (c1), (c2), or (c3) below:
   (c1) a polynucleotide comprising of the base sequence of SEQ ID NO: 3;
   (c2) a polynucleotide that comprises of a base sequence in which one or several bases are deleted, substituted, inserted and/or added in the base sequence of the (c1) with the 101st base (T) of the (c1) being conserved;
   (c3) a polynucleotide that comprises of a base sequence having an identity of at least 80% to the base sequence of the (c1) with the 101st base (T) of the (c1) being conserved;
(d) a polynucleotide of (d1), (d2), or (d3) below:
   (d1) a polynucleotide comprising of the base sequence of SEQ ID NO: 4;
   (d2) a polynucleotide that comprises of a base sequence in which one or several bases are deleted, substituted, inserted and/or added in the base sequence of the (d1) with the 101st base (C) of the (d1) being conserved;
   (d3) a polynucleotide that comprises of a base sequence having an identity of at least 80% to the base sequence of the (d1) with the 101st base (C) of the (d1) being conserved;
(e) a polynucleotide of (e1), (e2), or (e3) below:
   (e1) a polynucleotide comprising of the base sequence of SEQ ID NO: 5;
   (e2) a polynucleotide that comprises of a base sequence in which one or several bases are deleted, substituted, inserted and/or added in the base sequence of the (e1) with the 101st base (G) of the (e1) being conserved;
   (e3) a polynucleotide that comprises of a base sequence having an identity of at least 80% to the base sequence of the (e1) with the 101st base (G) of the (e1) being conserved;
(f) a polynucleotide of (f1), (f2), or (f3) below:
   (f1) a polynucleotide comprising of the base sequence of SEQ ID NO: 6;
   (f2) a polynucleotide that comprises of a base sequence in which one or several bases are deleted, substituted, inserted and/or added in the base sequence of the (f1) with the 101st base (A) of the (f1) being conserved;
   (f3) a polynucleotide that comprises of a base sequence having an identity of at least 80% to the base sequence of the (f1) with the 101st base (A) of the (f1) being conserved;
(g) a polynucleotide of (g1), (g2), or (g3) below:
   (g1) a polynucleotide having a polynucleotide of 1 to 855 bases between a polynucleotide comprising of the base sequence of SEQ ID NO: 14 and a polynucleotide comprising of the base sequence of SEQ ID NO: 15;
   (g2) a polynucleotide that comprises of a base sequence in which one or several bases are deleted, substituted, inserted and/or added in the base sequence of the (g1) with the base sequence of the polynucleotide of 1 to 855 bases in the (g1) being conserved;
   (g3) a polynucleotide that comprises of a base sequence having an identity of 80% or more to the base sequence of the (g1) with the base sequence of the polynucleotide of 1 to 855 bases in the (g1) being conserved.

### (Supplementary Note 89)

The detection method according to any one of Supplementary Notes 84 to 88, wherein the upright locus of the peduncle is detected by detecting a polynucleotide of the following (ga) in the detection step:
(ga) a polynucleotide of (gal), (ga2), or (ga3) below:
(gal) a polynucleotide comprising of the base sequence of SEQ ID NO: 13;
(ga2) a polynucleotide comprising of a base sequence in which one or several bases are deleted, substituted, inserted and/or added in the base sequence of the (gal);
(ga3) a polynucleotide comprising of a base sequence having an identity of at least 80% to the base sequence of the (gal).

### (Supplementary Note 90)

The detection method according to Supplementary Note 89,
wherein the upright locus of the peduncle is detected by detecting a polynucleotide selected from the group comprising of the (c), (d), and (g) in the detection step.

### (Supplementary Note 91)

The detection method according to Supplementary Note 90,
wherein the upright locus of the peduncle is detected by detecting the polynucleotide of the (g) in the detection step.

### (Supplementary Note 92)

The detection method according to any one of Supplementary Notes 84 to 91,
wherein an upright locus of the peduncle located in a region between sites of two molecular markers selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 in the chromosome is detected in the detection step.

### (Supplementary Note 93)

The detection method according to Supplementary Note 92,
wherein an upright locus of the peduncle located in a region
between the sites of molecular markers of the Ha_chr04_175698294 and the Ha_chr04_204904126, or
between sites of molecular markers of the Ha_chr04_181000331 and the Ha_chr04_200021302
on the chromosome is detected in the detection step.

### (Supplementary Note 94)

The detection method according to Supplementary Note 93, wherein an upright locus of the peduncle located in a region
between the sites of molecular markers of the Ha_chr04_181000331 and the Ha_chr04_200021302 on the chromosome is detected in the detection step.

### <Screening method>

### (Supplementary Note 95)

A screening method for a sunflower plant, comprising a selection step of selecting, from a test sunflower plant, a test sunflower in which the *Ha412HOChr04g0188691* gene is mutated, as a sunflower plant showing an upright trait.

### (Supplementary Note 96)

The screening method according to Supplementary Note 95,
wherein in the selection step, a test sunflower plant comprising a mutant of the *Ha412HOChr04g0188691* gene is selected as a sunflower plant showing an upright trait.

### (Supplementary Note 97)

The screening method according to Supplementary Note 96,
wherein the mutant comprises a base sequence in which one or several bases are deleted, substituted, inserted and/or added in a base sequence of a wild-type *Ha412HOChr04g0188691* gene, and expresses an upright trait.

### (Supplementary Note 98)

The screening method according to Supplementary Note 96 or 97,
wherein the mutant is a mutant gene comprising a missense mutation relative to a base sequence of a wild-type *Ha412HOChr04g0188691* gene, and expresses an upright trait.

### (Supplementary Note 99)

The screening method according to any one of Supplementary Notes 96 to 98,
wherein the mutant is a mutant gene comprising a frameshift mutation relative to a base sequence of a wild-type *Ha412HOChr04g0188691* gene, and expresses an upright trait.

### (Supplementary Note 100)

The screening method according to any one of Supplementary Notes 96 to 99,
wherein the mutant is a mutant gene comprising a splicing mutation relative to a base sequence of a wild-type *Ha412HOChr04g0188691* gene, and expresses an upright trait.

### (Supplementary Note 101)

The screening method according to any one of Supplementary Notes 97 to 100,
wherein the wild-type *Ha412HOChr04g0188691* gene is a gene comprising a polynucleotide of the following (P):
(P) any of polynucleotides (P1) to (P7) below:
(P1) a polynucleotide comprising of any of base sequences of SEQ ID NOs: 16 to 18;
(P2) a polynucleotide comprising of a base sequence in which one or several bases are deleted, substituted, inserted and/or added in the base sequence of the (P1), and encoding a protein that controls an upright trait;
(P3) a polynucleotide comprising of a base sequence having an identity of at least 80% to the base sequence of the (P1), and encoding a protein that controls an upright trait;
(P4) a polynucleotide comprising of a base sequence complementary to a polynucleotide that hybridizes under stringent conditions to the polynucleotide comprising of the base sequence of the (P1), and encoding a protein that controls an upright trait;
(P5) a polynucleotide encoding a protein comprising of any of the amino acid sequences of SEQ ID NOs: 19 to 21;
(P6) a polynucleotide encoding a protein comprising of an amino acid sequence in which one or several amino acids are deleted, substituted, inserted and/or added in the amino acid sequence of the (P5), and that controls the upright trait of the peduncle;
(P7) a polynucleotide encoding a protein comprising of an amino acid sequence having at least 80% identity to the amino acid sequence of the (P5), and that controls the upright trait of the peduncle.

### (Supplementary Note 102)

A screening method for a sunflower plant, the method comprising a selection step of selecting, from a test sunflower plant, a sunflower plant comprising an upright locus of the peduncle on chromosome 4, as a sunflower plant showing an upright trait.

### (Supplementary Note 103)

The screening method according to Supplementary Note 102,
wherein in the selection step, a sunflower plant having an upright locus of the peduncle identified by a molecular marker selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 is selected as a sunflower plant showing an upright trait.

### (Supplementary Note 104)

The screening method according to Supplementary Note 103,
wherein in the selection step, a sunflower plant having an upright locus of the peduncle identified by a molecular marker selected from the group comprising of Ha_chr04_181000331, an Indel marker of the *Ha412HOChr04g0188691* gene, and Ha_chr04_200021302 is selected as a sunflower plant showing an upright trait.

### (Supplementary Note 105)

The screening method according to Supplementary Note 104,
wherein in the selection step, a sunflower plant having an upright locus of the peduncle identified by an Indel marker of the *Ha412HOChr04g0188691* gene is selected as a sunflower plant showing an upright trait.

### (Supplementary Note 106)

The screening method according to any one of Supplementary Notes 102 to 105,
wherein in the selection, a sunflower plant having an upright locus of the peduncle identified by a polynucleotide selected from the group comprising of (a) to (g) below is selected as a sunflower plant showing an upright trait:
(a) a polynucleotide of (a1), (a2), or (a3) below:
   (a1) a polynucleotide comprising of SEQ ID NO: 1;
   (a2) a polynucleotide that comprises of a base sequence in which one or several bases are deleted, substituted, inserted and/or added in the base sequence of the (a1) with the 101st base (A) of the (a1) being conserved;
   (a3) a polynucleotide that comprises of a base sequence having an identity of at least 80% to the base sequence of the (a1) with the 101st base (A) of the (a1) being conserved.
(b) a polynucleotide of (b1), (b2), or (b3) below:
   (b1) a polynucleotide comprising of the base sequence of SEQ ID NO: 2;
   (b2) a polynucleotide that comprises of a base sequence in which one or several bases are deleted, substituted, inserted and/or added in the base sequence of the (b1) with the 202nd to 213th bases (TAAAAAAAAAAA) of the (b1) being conserved;
   (b3) a polynucleotide that comprises of a base sequence having an identity of at least 80% to the base sequence of the (b1) with the 202nd to 213th bases (A) of the (b1) being conserved;
(c) a polynucleotide of (c1), (c2), or (c3) below:
   (c1) a polynucleotide comprising of the base sequence of SEQ ID NO: 3;
   (c2) a polynucleotide that comprises of a base sequence in which one or several bases are deleted, substituted, inserted and/or added in the base sequence of the (c1) with the 101st base (T) of the (c1) being conserved;
   (c3) a polynucleotide that comprises of a base sequence having an identity of at least 80% to the base sequence of the (c1) with the 101st base (T) of the (c1) being conserved;
(d) a polynucleotide of (d1), (d2), or (d3) below:
   (d1) a polynucleotide comprising of the base sequence of SEQ ID NO: 4;
   (d2) a polynucleotide that comprises of a base sequence in which one or several bases are deleted, substituted, inserted and/or added in the base sequence of the (d1) with the 101st base (C) of the (d1) being conserved;
   (d3) a polynucleotide that comprises of a base sequence having an identity of at least 80% to the base sequence of the (d1) with the 101st base (C) of the (d1) being conserved;
(e) a polynucleotide of (e1), (e2), or (e3) below:
   (e1) a polynucleotide comprising of the base sequence of SEQ ID NO: 5;
   (e2) a polynucleotide that comprises of a base sequence in which one or several bases are deleted, substituted, inserted and/or added in the base sequence of the (e1) with the 101st base (G) of the (e1) being conserved;
   (e3) a polynucleotide that comprises of a base sequence having an identity of at least 80% to the base sequence of the (e1) with the 101st base (G) of the (e1) being conserved;
(f) a polynucleotide of (f1), (f2), or (f3) below:
   (f1) a polynucleotide comprising of the base sequence of SEQ ID NO: 6;
   (f2) a polynucleotide that comprises of a base sequence in which one or several bases are deleted, substituted, inserted and/or added in the base sequence of the (f1) with the 101st base (A) of the (f1) being conserved;
   (f3) a polynucleotide that comprises of a base sequence having an identity of at least 80% to the base sequence of the (f1) with the 101st base (A) of the (f1) being conserved;
(g) a polynucleotide of (g1), (g2), or (g3) below:
   (g1) a polynucleotide having a polynucleotide of 1 to 855 bases between a polynucleotide comprising of the base sequence of SEQ ID NO: 14 and a polynucleotide comprising of the base sequence of SEQ ID NO: 15;
   (g2) a polynucleotide that comprises of a base sequence in which one or several bases are deleted, substituted, inserted and/or added in the base sequence of the (g1) with the base sequence of the polynucleotide of 1 to 855 bases in the (g1) being conserved;
   (g3) a polynucleotide that comprises of a base sequence having an identity of 80% or more to the base sequence of the (g1) with the base sequence of the polynucleotide of 1 to 855 bases in the (g1) being conserved.

### (Supplementary Note 107)

The screening method according to any one of Supplementary Notes 102 to 106,
wherein in the selection, a sunflower plant having an upright locus of the peduncle identified by a polynucleotide of (ga) below is selected as a sunflower plant showing an upright trait.
(ga) a polynucleotide of (ga1), (ga2), or (ga3) below:
(ga1) a polynucleotide comprising of the base sequence of SEQ ID NO: 13;
(ga2) a polynucleotide comprising of a base sequence in which one or several bases are deleted, substituted, inserted and/or added in the base sequence of the (ga1);
(ga3) a polynucleotide comprising of a base sequence having an identity of at least 80% to the base sequence of the (ga1).

### (Supplementary Note 108)

The screening method according to Supplementary Note 106,
wherein in the selection step, a sunflower plant having an upright locus of the peduncle identified by a polynucleotide selected from the group comprising of the (c), (d), and (g) is selected as a sunflower plant showing an upright trait.

### (Supplementary Note 109)

The screening method according to Supplementary Note 108,
wherein in the selection step, a sunflower plant having an upright locus of the peduncle identified by the polynucleotide of the (g) is selected as a sunflower plant showing an upright trait.

### (Supplementary Note 110)

The screening method according to any one of Supplementary Notes 102 to 109,
wherein in the selection step, a sunflower plant having an upright locus of the peduncle located in a region between the sites of the two molecular markers selected from the group comprising of Ha_chr04_172545117, Ha_chr04_175698294, Ha_chr04_181000331, an Indel marker of the *Ha412HOChr04g0188691* gene, Ha_chr04_200021302, Ha_chr04_204904126, and Ha_chr04_208654314 on the chromosome is selected as a sunflower plant showing an upright trait.

### (Supplementary Note 111)

The screening method according to Supplementary Note 110,
wherein in the selection step, a sunflower plant having an upright locus of the peduncle located in a region
between the sites of molecular markers of the Ha_chr04_175698294 and the Ha_chr04_204904126, or
between the sites of molecular markers of the Ha_chr04_181000331 and the Ha_chr04_200021302
on the chromosome is selected as a sunflower plant showing an upright trait.

### (Supplementary Note 112)

The screening method according to Supplementary Note 111,
wherein in the selection step, a sunflower plant having an upright locus of the peduncle located in a region
between the sites of molecular markers of the Ha_chr04_181000331 and the Ha_chr04_200021302 on the chromosome is selected as a sunflower plant showing an upright trait.

### [Industrial Applicability]

As described above, according to the present disclosure, a new sunflower plant can be provided. Therefore, the present disclosure is extremely useful in agricultural fields such as breeding, for example.

## Claims

1. A sunflower plant comprising a mutant of the *Ha412HOChr04g0188691* gene, wherein the peduncle is upright.

2. The sunflower plant according to claim 1,
wherein the mutant of the *Ha412HOChr04g0188691* gene comprises a base sequence in which one or several nucleotides are deleted, inserted, substituted, and/or added in a base sequence of a wild-type *Ha412HOChr04g0188691* gene, and confers an upright trait of the peduncle.

3. The sunflower plant according to claim 1 or 2,
wherein the mutant of the *Ha412HOChr04g0188691* gene comprises of an amino acid sequence in which one or several amino acids are inserted, deleted, substituted, and/or added in an amino acid sequence encoded by a wild-type *Ha412HOChr04g0188691* gene, and comprises a base sequence encoding a protein that confers the upright trait of the peduncle.

4. A sunflower plant comprising an upright locus of the peduncle on chromosome 4,
wherein the peduncle is upright.

5. The sunflower plant according to claim 4,
wherein the upright locus of the peduncle is specified by a polynucleotide selected from the group comprising of the following (a) to (g):
(a) a polynucleotide of the following (a1), (a2), or (a3):
(a1) a polynucleotide comprising of the base sequence of SEQ ID NO: 1;
(a2) a polynucleotide that comprises of a base sequence in which 1 to 20 bases are deleted, substituted, inserted and/or added in the base sequence of the (a1) with the 101st base (A) of the (a1) being conserved;
(a3) a polynucleotide that comprises of a base sequence having an identity of at least 90% to the base sequence of the (a1) with the 101st base (A) of the (a1) being conserved;
(b) a polynucleotide of the following (b1), (b2), or (b3):
(b1) a polynucleotide comprising of the base sequence of SEQ ID NO: 2;
(b2) a polynucleotide that comprises of a base sequence in which 1 to 40 bases are deleted, substituted, inserted and/or added in the base sequence of the (b1) with the 202nd to 213th bases (TAAAAAAAAAAA) of the (b1) being conserved;
(b3) a polynucleotide that comprises of a base sequence having an identity of at least 90% to the base sequence of the (b1) with the 202nd to 213th bases (A) of the (b1) being conserved;
(c) a polynucleotide of the following (c1), (c2), or (c3):
(c1) a polynucleotide comprising of the base sequence of SEQ ID NO: 3;
(c2) a polynucleotide that comprises of a base sequence in which 1 to 20 bases are deleted, substituted, inserted and/or added in the base sequence of the (c1) with the 101st base (T) of the (c1) being conserved;
(c3) a polynucleotide that comprises of a base sequence having an identity of at least 90% to the base sequence of the (c1) with the 101st base (T) of the (c1) being conserved;
(d) a polynucleotide of the following (d1), (d2), or (d3):
(d1) a polynucleotide comprising of the base sequence of SEQ ID NO: 4;
(d2) a polynucleotide that comprises of a base sequence in which 1 to 20 bases are deleted, substituted, inserted and/or added in the base sequence of the(d1) with the 101st base (C) of the (d1) being conserved;
(d3) a polynucleotide that comprises of a base sequence having an identity of at least 90% to the base sequence of the (d1) with the 101st base (C) of the (d1) being conserved;
(e) a polynucleotide of the following (e1), (e2), or (e3):
(e1) a polynucleotide comprising of the base sequence of SEQ ID NO: 5;
(e2) a polynucleotide that comprises of a base sequence in which 1 to 20 bases are deleted, substituted, inserted and/or added in the base sequence of the (e1) with the 101st base (G) of the (e1) being conserved;
(e3) a polynucleotide that comprises of a base sequence having an identity of at least 90% to the base sequence of the (e1) with the 101st base (G) of the (e1) being conserved;
(f) a polynucleotide of the following (f1), (f2), or (f3):
(f1) a polynucleotide comprising of the base sequence of SEQ ID NO: 6;
(f2) a polynucleotide that comprises of a base sequence in which 1 to 20 bases are deleted, substituted, inserted and/or added in the base sequence of the (f1) with the 101st base (A) of the (f1) being conserved;
(f3) a polynucleotide that comprises of a base sequence having an identity of at least 90% to the base sequence of the (f1) with the 101st base (A) of the (f1) being conserved;
(g) a polynucleotide of the following (g1), (g2), or (g3):
(g1) a polynucleotide having a polynucleotide of 1 to 855 nucleotides between a polynucleotide comprising of the base sequence of SEQ ID NO: 14 and a polynucleotide comprising of the base sequence of SEQ ID NO: 15;
(g2) a polynucleotide that comprises of a base sequence in which 1 to 3 bases are deleted, substituted, inserted and/or added in the base sequence of the (g1) with the base sequence of the polynucleotide of 1 to 855 bases in the (g1) being conserved;
(g3) a polynucleotide that comprises of a base sequence having an identity of 90% or more to the base sequence of the (g1) with the base sequence of the polynucleotide of 1 to 855 bases in the (g1) being conserved.

6. The sunflower plant according to claim 4 or 5,
wherein the upright locus of the peduncle is specified by a polynucleotide of the following (ga):
(ga) a polynucleotide of the following (ga1), (ga2), or (ga3):
(ga1) a polynucleotide comprising of the base sequence of SEQ ID NO: 13;
(ga2) a polynucleotide comprising of a base sequence in which 1 to 85 bases are deleted, substituted, inserted and/or added in the base sequence of the (ga1);
(ga3) a polynucleotide comprising of a base sequence having an identity of 90% or more to the base sequence of the (ga1).

7. The sunflower plant according to any one of claims 4 to 6,
wherein the upright locus of the peduncle is located in a region
between the sites of molecular markers of the Ha_chr04_175698294 and the Ha_chr04_204904126, or
between the sites of molecular markers of the Ha_chr04_181000331 and the Ha_chr04_200021302 on the chromosome.

8. The sunflower plant according to any one of claims 1 to 7,
wherein the sunflower plant is a sunflower plant specified by Accession No. FERM BP-22482 or a progeny line thereof.

9. The sunflower plant according to any one of claims 1 to 8,
wherein the peduncle angle is 0 to 45°.

10. A plant population of sunflower plants,
wherein the plant population comprises sunflower plants in which the peduncle is upright, and in the plant population, a ratio of individuals in which the peduncle angle is 0 to 45° is 50% or more.

11. The plant population according to claim 10,
wherein the population of the sunflower plants comprises the sunflower plant according to any one of claims 1 to 9.

12. A plant part of the sunflower plant according to any one of claims 1 to 9.

13. The plant part according to claim 12,
wherein the plant part is a flower, a stem, a leaf, a root, pollen, and/or a seed.

14. A method for producing a sunflower plant or a plant population of sunflower plants showing an upright trait of the peduncle, the method comprising the steps of:
(a) crossing the sunflower plant according to any one of claims 1 to 9 with another sunflower plant;
(b) selecting a sunflower plant showing the upright trait of the peduncle from the sunflower plant or a progeny line thereof obtained by the step (a).

15. The method for producing a sunflower plant or a plant population of sunflower plants showing an upright trait of the peduncle according to claim 14, the method comprising the following step (x) prior to the step (a):
(x) selecting the sunflower plant according to any one of claims 1 to 9 from a test sunflower plant.

16. The method for producing a sunflower plant or a plant population of sunflower plants according to claim 15,
wherein the selection in the step (x) is the selection of a sunflower plant comprising a mutant of the *Ha412HOChr04g0188691* gene.

17. The method for producing a sunflower plant or a plant population of sunflower plants showing an upright trait of the peduncle according to claim 15,
wherein the selection in the step (x) is the selection of a sunflower plant showing the upright trait of the peduncle comprising an upright locus of the peduncle on chromosome 4.

18. The method for producing a sunflower plant or a plant population of sunflower plants showing an upright trait of the peduncle according to claim 17,
wherein the selection in the step (x) is a method for selecting a sunflower plant showing the upright trait of the peduncle having an upright locus of the peduncle specified by a polynucleotide selected from the group comprising of the following (a) to (g):
(a) a polynucleotide of the following (a1), (a2), or (a3):
(a1) a polynucleotide comprising of the base sequence of SEQ ID NO: 1;
(a2) a polynucleotide that comprises of a base sequence in which 1 to 20 bases are deleted, substituted, inserted and/or added in the base sequence of the (a1) with the 101st base (A) of the (a1) being conserved;
(a3) a polynucleotide that comprises of a base sequence having an identity of at least 90% to the base sequence of the (a1) with the 101st base (A) of the (a1) being conserved;
(b) a polynucleotide of the following (b1), (b2), or (b3):
(b1) a polynucleotide comprising of the base sequence of SEQ ID NO: 2;
(b2) a polynucleotide that comprises of a base sequence in which 1 to 40 bases are deleted, substituted, inserted and/or added in the base sequence of the (b1) with the 202nd to 213th bases (TAAAAAAAAAAA) of the (b1) being conserved;
(b3) a polynucleotide that comprises of a base sequence having an identity of at least 90% to the base sequence of the (b1) with the 202nd to 213th bases (A) of the (b1) being conserved;
(c) a polynucleotide of the following (c1), (c2), or (c3):
(c1) a polynucleotide comprising of the base sequence of SEQ ID NO: 3;
(c2) a polynucleotide that comprises of a base sequence in which 1 to 20 bases are deleted, substituted, inserted and/or added in the base sequence of the (c1) with the 101st base (T) of the (c1) being conserved;
(c3) a polynucleotide that comprises of a base sequence having an identity of at least 90% to the base sequence of the (c1) with the 101st base (T) of the (c1) being conserved;
(d) a polynucleotide of the following (d1), (d2), or (d3):
(d1) a polynucleotide comprising of the base sequence of SEQ ID NO: 4;
(d2) a polynucleotide that comprises of a base sequence in which 1 to 20 bases are deleted, substituted, inserted and/or added in the base sequence of the (d1) with the 101st base (C) of the (d1) being conserved;
(d3) a polynucleotide that comprises of a base sequence having an identity of at least 90% to the base sequence of the (d1) with the 101st base (C) of the (d1) being conserved;
(e) a polynucleotide of the following (e1), (e2), or (e3):
(e1) a polynucleotide comprising of the base sequence of SEQ ID NO: 5;
(e2) a polynucleotide that comprises of a base sequence in which 1 to 20 bases are deleted, substituted, inserted and/or added in the base sequence of the (e1) with the 101st base (G) of the (e1) being conserved;
(e3) a polynucleotide that comprises of a base sequence having an identity of at least 90% to the base sequence of the (e1) with the 101st base (G) of the (e1) being conserved;
(f) a polynucleotide of the following (f1), (f2), or (f3):
(f1) a polynucleotide comprising of the base sequence of SEQ ID NO: 6;
(f2) a polynucleotide that comprises of a base sequence in which 1 to 20 bases are deleted, substituted, inserted and/or added in the base sequence of the (f1) with the 101st base (A) of the (f1) being conserved;
(f3) a polynucleotide that comprises of a base sequence having an identity of at least 90% to the base sequence of the (f1) with the 101st base (A) of the (f1) being conserved;
(g) a polynucleotide of the following (g1), (g2), or (g3):
(g1) a polynucleotide having a polynucleotide of 1 to 855 nucleotides between a polynucleotide comprising of the base sequence of SEQ ID NO: 14 and a polynucleotide comprising of the base sequence of SEQ ID NO: 15;
(g2) a polynucleotide that comprises of a base sequence in which 1 to 3 bases are deleted, substituted, inserted and/or added in the base sequence of the (g1) with the base sequence of the polynucleotide of 1 to 855 bases in the (g1) being conserved;
(g3) a polynucleotide that comprises of a base sequence having an identity of 90% or more to the base sequence of the (g1) with the base sequence of the polynucleotide of 1 to 855 bases in the (g1) being conserved.

19. The method for producing a sunflower plant or a plant population of sunflower plants showing an upright trait of the peduncle according to claim 17 or 18,
wherein the selection in the step (x) is a method for selecting a sunflower plant showing the upright trait of the peduncle having an upright locus of the peduncle specified by a polynucleotide of the following (ga):
(ga) a polynucleotide of the following (ga1), (ga2), or (ga3):
(ga1) a polynucleotide comprising of the base sequence of SEQ ID NO: 13;
(ga2) a polynucleotide comprising of a base sequence in which 1 to 85 bases are deleted, substituted, inserted and/or added in the base sequence of the (ga1);
(ga3) a polynucleotide comprising of a base sequence having an identity of 90% or more to the base sequence of the (ga1).

20. The method for producing a sunflower plant or a plant population of sunflower plants showing an upright trait of the peduncle according to any one of claims 17 to 19,
wherein the selection in the step (x) is the selection of a sunflower plant showing the upright trait of the peduncle having an upright locus of the peduncle located in a region between the sites of molecular markers of the Ha_chr04_175698294 and the Ha_chr04_204904126, or between the sites of molecular markers of the Ha_chr04_181000331 and the Ha_chr04_200021302 on the chromosome.

21. The method for producing a sunflower plant or a plant population of sunflower plants showing an upright trait of the peduncle according to any one of claims 17 to 20,
wherein the selection in the step (x) is the selection of a sunflower plant in which the peduncle angle is 0 to 45°.

22. A method for imparting an upright trait of the peduncle to a sunflower plant, the method comprising an upright step of making the peduncle of the sunflower plant upright by changing the function of the *Ha412HOChr04g0188691* gene of the sunflower plant.

23. The method for imparting according to claim 22,
wherein in the upright step, the method comprises a suppression step of suppressing gene expression of the *Ha412HOChr04g0188691* gene of the sunflower plant.

24. The method for imparting according to claim 22 or 23,
wherein in the upright step, the method comprises a mutation step of mutating the *Ha412HOChr04g0188691* gene of the sunflower plant.

25. The method for imparting according to claim 24,
wherein in the mutation step, a mutation is introduced into the *Ha412HOChr04g0188691* gene of the sunflower plant to produce an upright sunflower plant comprising a mutant of the *Ha412HOChr04g0188691* gene and in which the peduncle is upright.

26. The method for imparting according to claim 25,
wherein the mutant comprises a base sequence in which one or several nucleotides are deleted, substituted, inserted and/or added in a base sequence of a wild-type *Ha412HOChr04g0188691* gene, and confers the upright trait of the peduncle.

27. An isolated nucleic acid having a function capable of determining an upright trait of the peduncle of a sunflower plant, comprising a contiguous base sequence of 15 nucleotides or more in a base sequence selected from the group comprising of the following (a) to (g) in a chromosome of a sunflower plant:
(a) a base sequence of the following (a1), (a2), or (a3):
(a1) the base sequence of SEQ ID NO: 1;
(a2) a base sequence in which 1 to 20 bases are deleted, substituted, inserted and/or added in the base sequence of the (a1) with the 101st base (A) of the (a1) being conserved;
(a3) a base sequence having an identity of at least 90% to the base sequence of the (a1) with the 101st base (A) of the (a1) being conserved;
(b) a base sequence of the following (b1), (b2), or (b3):
(b1) the base sequence of SEQ ID NO: 2;
(b2) a base sequence in which 1 to 40 bases are deleted, substituted, inserted and/or added in the base sequence of the (b1) with the 202nd to 213th bases (TAAAAAAAAAAA) of the (b1) being conserved;
(b3) a base sequence having an identity of at least 90% to the base sequence of the (b1) with the 202nd to 213th bases (A) of the (b1) being conserved;
(c) a base sequence of the following (c1), (c2), or (c3):
(c1) the base sequence of SEQ ID NO: 3;
(c2) a base sequence in which 1 to 20 bases are deleted, substituted, inserted and/or added in the base sequence of the (c1) with the 101st base (T) of the (c1) being conserved;
(c3) a base sequence having an identity of at least 90% to the base sequence of the (c1) with the 101st base (T) of the (c1) being conserved;
(d) a base sequence of the following (d1), (d2), or (d3):
(d1) the base sequence of SEQ ID NO: 4;
(d2) a base sequence in which 1 to 20 bases are deleted, substituted, inserted and/or added in the base sequence of the(d1) with the 101st base (C) of the (d1) being conserved;
(d3) a base sequence having an identity of at least 90% to the base sequence of the (d1) with the 101st base (C) of the (d1) being conserved;
(e) a base sequence of the following (e1), (e2), or (e3):
(e1) the base sequence of SEQ ID NO: 5;
(e2) a base sequence in which 1 to 20 bases are deleted, substituted, inserted and/or added in the base sequence of the (e1) with the 101st base (G) of the (e1) being conserved;
(e3) a base sequence having an identity of at least 90% to the base sequence of the (e1) with the 101st base (G) of the (e1) being conserved;
(f) a base sequence of the following (f1), (f2), or (f3):
(f1) the base sequence of SEQ ID NO: 6;
(f2) a base sequence in which 1 to 20 bases are deleted, substituted, inserted and/or added in the base sequence of the (f1) with the 101st base (A) of the (f1) being conserved;
(f3) a base sequence having an identity of at least 90% to the base sequence of the (f1) with the 101st base (A) of the (f1) being conserved;
(g) a base sequence of the following (g1), (g2), or (g3):
(g1) a base sequence having a base sequence of 1 to 855 bases between the base sequence of SEQ ID NO: 14 and the base sequence of SEQ ID NO: 15;
(g2) a base sequence in which 1 to 3 bases are deleted, substituted, inserted and/or added in the base sequence of the (g1) with the base sequence of the polynucleotide of 1 to 855 bases in the (g1) being conserved;
(g3) a base sequence having an identity of 90% or more to the base sequence of the (g1) with the base sequence of the polynucleotide of 1 to 855 bases in the (g1) being conserved.

28. The nucleic acid according to claim 27, comprising a contiguous base sequence of 15 nucleotides or more in a base sequence selected from the group comprising of the following (ga) in a chromosome of a sunflower plant:
(ga) a base sequence of the following (ga1), (ga2), or (ga3):
(ga1) the base sequence of SEQ ID NO: 13;
(ga2) a base sequence in which 1 to 85 bases are deleted, substituted, inserted and/or added in the base sequence of the (ga1);
(ga3) a base sequence having an identity of 90% or more to the base sequence of the (ga1).

29. A method for detecting an upright trait of the peduncle of a sunflower plant, the method comprising a detection step of detecting whether the *Ha412HOChr04g0188691* gene is mutated in a test sunflower plant.

30. The method for detecting according to claim 29,
wherein the detection step comprises a detection step of detecting whether a mutant of the *Ha412HOChr04g0188691* gene is present in the test sunflower plant.

31. The method for detecting according to claim 30,
wherein the mutant comprises a base sequence in which one or several nucleotides are deleted, substituted, inserted and/or added in a base sequence of a wild-type *Ha412HOChr04g0188691* gene, and confers the upright trait of the peduncle.

32. A method for detecting an upright trait of the peduncle of a sunflower plant, the method comprising a detection step of detecting an upright locus of the peduncle on chromosome 4 for a test sunflower plant.

33. A method for screening a sunflower plant, the method comprising a selection step of selecting, from a test sunflower plant, a test sunflower in which the *Ha412HOChr04g0188691* gene is mutated, as a sunflower plant showing an upright trait of the peduncle.

34. The method for screening according to claim 33, wherein in the selection step, a test sunflower plant comprising a mutant of the *Ha412HOChr04g0188691* gene is selected as a sunflower plant showing the upright trait of the peduncle.

35. A method for screening a sunflower plant, the method comprising a selection step of selecting, for a test sunflower plant, a sunflower plant comprising an upright locus of the peduncle on chromosome 4, as a sunflower plant showing an upright trait of the peduncle.
